# EUROPEAN PATENT SPECIFICATION

(11) **EP 3 617 314 B1**
(45) Date of publication and mention of the grant of the patent: **15.02.2023**
(21) Application number: 18792160.6
(22) Date of filing: 01.05.2018
(51) Int. Cl.: C12N 15/113, A61K 31/713, A61K 48/00, A61P 35/00

(54) **OLIGONUCLEOTIDE DERIVATIVE OR SALT THEREOF**
OLIGONUKLEOTIDDERIVAT ODER SALZ DARAUS
DÉRIVÉ D'OLIGONUCLÉOTIDE OU SEL DE CELUI-CI

(30) Priority: 28.04.2017 JP 2017090613
(43) Date of publication of application: 04.03.2020
(73) Proprietor: Kyowa Kirin Co., Ltd., Chiyoda-ku Tokyo 1000004 (JP)
(72) Inventor: YAMAMOTO, Junichiro, Tokyo 100-0004 (JP); SHINOHARA, Fumikazu, Tokyo 100-0004 (JP); HARUMOTO, Toshimasa, Tokyo 100-0004 (JP); HOMMA, Masakazu, Tokyo 100-0004 (JP); HAGIWARA, Kenji, Tokyo 100-004 (JP)
(74) Representative: D Young & Co LLP
(86) International application number: PCT/JP2018/017462
(87) International publication number: WO 2018/199340

(56) References cited:
- WO-A1-2013/129663
- WO-A1-2014/134029
- WO-A2-03/072705
- WO-A2-2008/137758
- WO-A2-2010/045512
- WO-A2-2010/120803
- JP-A- 2014 143 923
- XIAOOU JIANG ET AL: "Advanced Design of Dumbbell-shaped Genetic Minimal Vectors Improves Non-coding and Coding RNA Expression", MOLECULAR THERAPY, vol. 24, no. 9, 1 September 2016 (2016-09-01), pages 1581-1591, XP055554168, US ISSN: 1525-0016, DOI: 10.1038/mt.2016.138
- ABE, NAHOKO: 'RNA interference effect of circular RNA and its characteristics as translation template' DOCTORAL THESIS, [Online] 25 March 2015, HOKKAIDO UNIVERSITY, XP055528884 Retrieved from the Internet: <URL:https://eprints.lib.hokudai.ac.jp/dspa ce/bitstream/2115/59261/1/Naoko_Abe.pdf> [retrieved on 2018-07-23]
- SIERANT, M. ET AL.: 'Longer 19-Base Pair Short Interfering RNA Duplexes Rather Than Shorter Duplexes Trigger RNA Interference' OLIGONUCLEOTIDES vol. 20, no. 4, August 2010, pages 199 - 206, XP055528875

## Description

### Technical Field

The present invention relates to an oligonucleotide derivative or a salt thereof. Specifically, the present invention relates to an oligonucleotide derivative or a salt thereof which exhibits improved resistance to degradation by an enzyme *in vivo.*

### Background Art

Small interfering RNA (hereinafter, referred to as siRNA) is RNA that is involved in RNA interference (hereinafter, referred to as RNAi) and has a function as a guide for silencing a target gene expression (Non Patent Literature 1). siRNA is capable of selectively suppressing (knocking down) the expression of a protein, of which messenger RNA (mRNA) is in charge, via cleavage of the mRNA, and is therefore expected to be applied to medicines (Non Patent Literature 2).

A challenge to the application of siRNA to pharmaceutical products includes *in vivo* instability, i.e., susceptibility to degradation by nuclease.

In order to improve resistance to degradation by nuclease, Patent Literature 1 discloses a nucleic acid prepared by circularizing a simplex, or a dumbbell-shaped nucleic acid. A feature of this nucleic acid is the absence of an RNA end, which renders the nucleic acid less degradable by nuclease.

Also, Patent Literature 2 discloses that siRNA is constructed in a cell by co-administering two circular nucleic acids into the cell, wherein the two circular nucleic acids are designed so as to cause ring opening in the cell, and have sequences complementary to each other.

WO 03/072705 A2 relates to small nucleic acid molecules, such as short interfering nucleic acid (siNA), short interfering RNA (siRNA), double-stranded RNA (dsRNA), micro-RNA (miRNA), and short hairpin RNA (shRNA) molecules capable of mediating RNA interference (RNAi) against cyclin D1 gene expression.

WO 2008/137758 A2 relates to amino acid lipid compounds and compositions useful for drug delivery, such as nucleic acid therapeutics, for example circular siRNA molecules.

WO 2010/120803 A2 relates to the circularization of small RNA targets or their conjugates with oligonucleotide adapters.

### Citation List

### Patent Literature

Patent Literature 1: Japanese Patent Laid-Open No. 2008-278784
Patent Literature 2: Japanese Patent Laid-Open No. 2014-143923

### Non Patent Literature

Non Patent Literature 1: Nature, Vol. 411, No. 6836, p. 494-498 (2001)
Non Patent Literature 2: Nature Reviews Cancer, Vol. 11, p. 59-67 (2011)

### Summary of Invention

### Technical Problem

However, there are a few pieces of information on gene silencing as to the circular nucleic acid disclosed in Patent Literature 1 or 2, though its improved stability against nuclease has been reported. Thus, a circularized nucleic acid having strong knock-down activity has been strongly desired.

An object of the present invention is to provide a novel oligonucleotide derivative having resistance to nuclease and having strong knock-down activity.

### Solution to Problem

The invention is set out in the appended set of claims.

In one aspect, the invention provides an oligonucleotide derivative or a salt thereof comprising a circular oligonucleotide and a linear oligonucleotide, wherein
the circular oligonucleotide and the linear oligonucleotide have base sequences complementary to each other, and form a complex via a hydrogen bond between the complementary base sequences, and
wherein the circular oligonucleotide has a base length of 10 to 40, and
wherein the circular oligonucleotide is represented by formula 1: 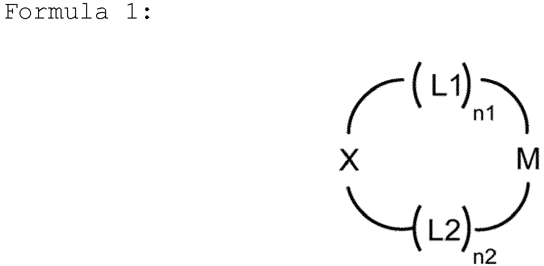 wherein
   L1 and L2 each represents a linker;
   n1 and n2 each independently represents an integer of 0 to 10;
   M represents a moiety comprising a chemical structure that is cleaved by an intracellular environment; and
   X represents an oligonucleotide.

In a further aspect, the invention provides a pharmaceutical composition comprising an oligonucleotide derivative or a salt thereof according to the invention.

In a further aspect, the invention provides an agent for suppressing an expression of a target gene utilizing RNA interference (RNAi), comprising an oligonucleotide derivative or a salt thereof according to the invention.

In a further aspect, the invention provides a circular oligonucleotide comprising at least one phosphorothioate bond, the circular oligonucleotide being represented by formula 4: 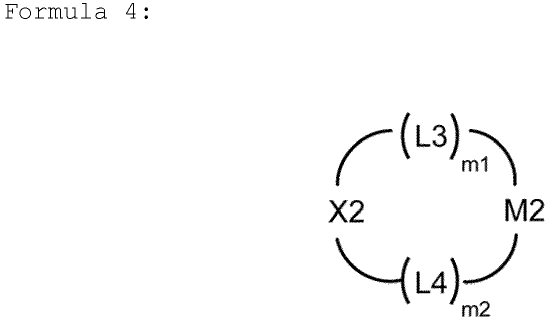 wherein
L3 and L4 each represents a linker;
m1 and m2 each independently represents an integer of 0 to 10;
M2 represents a moiety comprising a chemical structure that is cleaved by an intracellular environment,
wherein the chemical structure that is cleaved by an intracellular environment is -S-S-, -S-C(O)- or -C(O)-S-,
wherein M2 is selected from the group consisting of 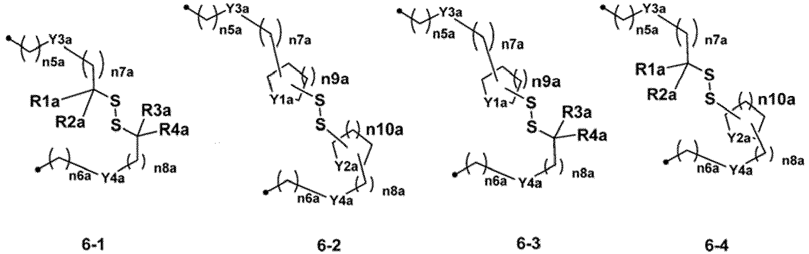 formula 6-1 to formula 6-6:
   wherein
   R1a and R2a each independently represents a hydrogen atom or C1-C3 alkyl, or R1a and R2a, together with the carbon atom to which they are bonded, form a ring having 3 to 6 carbon atoms;
   R3a and R4a each independently represents a hydrogen atom or C1-C3 alkyl, or R3a and R4a, together with the carbon atom to which they are bonded, form a ring having 3 to 6 carbon atoms;
   n5a to n8a each independently represents an integer of 0 to 10;
   n9a and n10a each independently represents an integer of 1 to 4;
   Y1a to Y4a each independently represents a bond, - NR5a-, -O- or -S-; and
   R5a represents a hydrogen atom, C1-C3 alkyl or C2-C4 alkanoyl, and 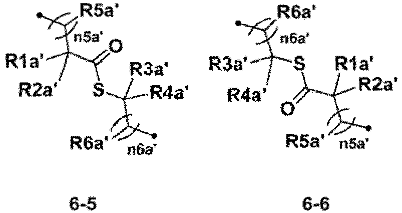 wherein
      R1a' and R2a' each independently represents a hydrogen atom or C1-C3 alkyl, or R1a' and R2a', together with the carbon atom to which they are bonded, form a ring having 3 to 6 carbon atoms;
      R3a' and R4a' each independently represents a hydrogen atom or C1-C3 alkyl, or R3a' and R4a', together with the carbon atom to which they are bonded, form a ring having 3 to 6 carbon atoms;
      R5a' and R6a' represent a hydrogen atom or C1-C3 alkyl independently on the basis of each carbon atom to which they are bonded; and
      n5a' and n6a' each independently represents an integer of 1 to 10; and
      X2 represents an oligonucleotide.

In a further aspect, the invention provides a linear oligonucleotide comprising at least one phosphorothioate bond, the linear oligonucleotide being represented by formula 7: 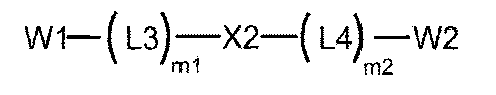 wherein
X2, L3, L4, m1 and m2 are as defined in claim 11; and
W1 and W2 are moieties comprising or forming functional groups reacting with each other to form a chemical structure that is cleaved by an intracellular environment,
wherein the chemical structure that is cleaved by an intracellular environment is -S-S-, -S-C(O)- or -C(O)-S-, and
wherein W1 and W2 are each independently -A1-S-S-A2 or -B1-COO-B2 (wherein the case is excluded where W1 and W2 are -B1-COO-B2 at the same time);
A1 and B1 are each independently C2-C10 alkylene optionally having a substituent;
A2 is C1-C10 alkyl optionally having a substituent; and
B2 is a hydrogen atom or C1-C6 alkyl optionally having a substituent.

In a further aspect, the invention provides a method for producing a circular oligonucleotide according to the invention, comprising circularizing a linear oligonucleotide according to the invention.

In a further aspect, the invention provides a method for producing an oligonucleotide derivative or a salt thereof according to the invention, comprising complexing a circular oligonucleotide according to the invention with a linear oligonucleotide having a base sequence complementary to the circular oligonucleotide via a hydrogen bond.

### Advantageous Effects of Invention

The present invention can provide a novel oligonucleotide derivative having resistance to degradation by nuclease and having strong knock-down activity.

### Brief Description of Drawings

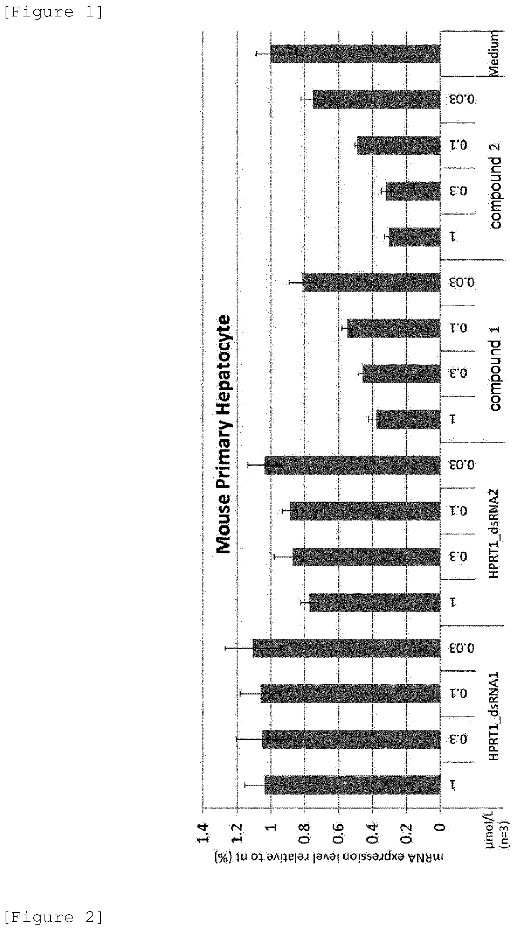

[Figure 1] Figure 1 shows the knock-down activity of compound 1 and compound 2 targeting hypoxanthine-guanine phosphoribosyltransferase 1 (HPRT1) in mouse primary hepatocytes. This figure shows test results obtained when HPRT1_dsRNA1, HPRT1_dsRNA2 and compounds 1 and 2 were each added at concentrations of 1 µmoL/L, 0.3 µmoL/L, 0.1 µmoL/L and 0.03 µmol/L to the mouse primary hepatocytes. Medium depicts test results about an siRNA-unintroduced group (control group). The ordinate depicts mean ± standard deviation (n = 3) of a relative ratio of the mRNA level of HPRT1 in a specimen in which each siRNA described above was introduced, when the mRNA level of HPRT1 in Medium (control group) was defined as 1. HPRT1_dsRNA1 and HPRT1_dsRNA2 are negative control groups for compound 1 and compound 2, respectively.
[Figure 2] Figure 2 shows the knock-down activity of compound 3 targeting B2M in mouse primary hepatocytes. This figure shows test results obtained when B2M_dsRNA and compound 3 were each added at concentrations of 1 µmoL/L, 0.3 µmoL/L, 0.1 µmoL/L and 0.03 µmol/L to the mouse primary hepatocytes. Medium depicts test results about an siRNA-unintroduced group (control group). The ordinate depicts mean ± standard deviation (n = 3) of a relative ratio of the mRNA level of B2M in a specimen in which each siRNA described above was introduced, when the mRNA level of B2M in Medium (control group) was defined as 1. B2M_dsRNA is a negative control group for compound 3.

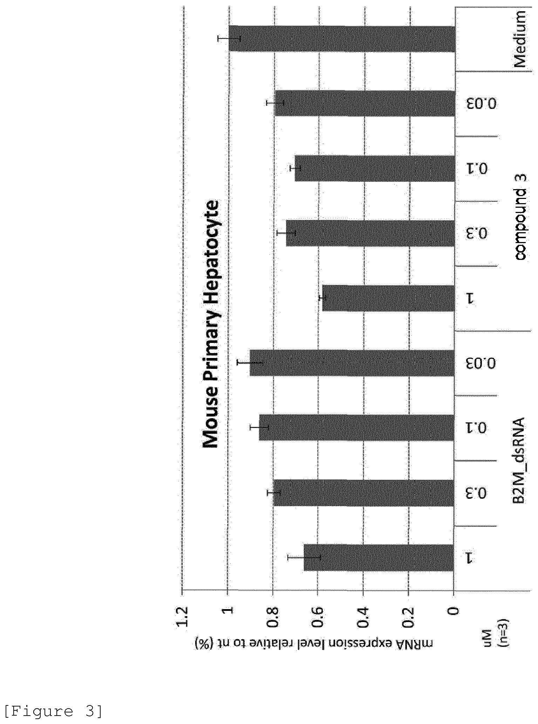

[Figure 3] Figure 3 shows the knock-down activity of compound 4 and compound 5 targeting hypoxanthine-guanine phosphoribosyltransferase 1 (HPRT1) in mouse primary hepatocytes. This figure shows test results obtained when HPRT1_dsRNA3, HPRT1_dsRNA4 and compounds 4 and 5 were each added at concentrations of 1 µmoL/L, 0.3 µmoL/L, 0.1 µmoL/L and 0.03 µmol/L to the mouse primary hepatocytes. Medium depicts test results about an siRNA-unintroduced group (control group). The ordinate depicts mean ± standard deviation (n = 3) of a relative ratio of the mRNA level of HPRT1 in a specimen in which each siRNA described above was introduced, when the mRNA level of HPRT1 in Medium (control group) was defined as 1. HPRT1_dsRNA3 and HPRT1_dsRNA4 are negative control groups for compound 4 and compound 5, respectively.

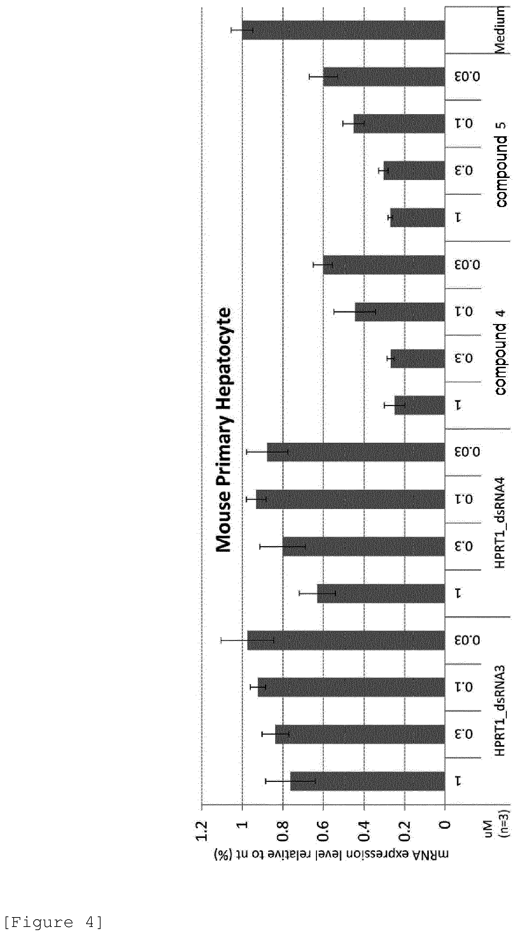

[Figure 4] Figure 4 shows the knock-down activity of compound 6 targeting hypoxanthine-guanine phosphoribosyltransferase 1 (HPRT1) in mouse primary hepatocytes. This figure shows test results obtained when HPRT1_dsRNA5 and compound 6 were each added at concentrations of 0.3 µmoL/L, 0.1 µmoL/L, 0.03 µmol/L and 0.01 µmol/L to the mouse primary hepatocytes. Medium depicts test results about an siRNA-unintroduced group (control group). The ordinate depicts mean ± standard deviation (n = 3) of a relative ratio of the mRNA level of HPRT1 in a specimen in which each siRNA described above was introduced, when the mRNA level of HPRT1 in Medium (control group) was defined as 1. HPRT1_dsRNA5 is a negative control group for compound 6.

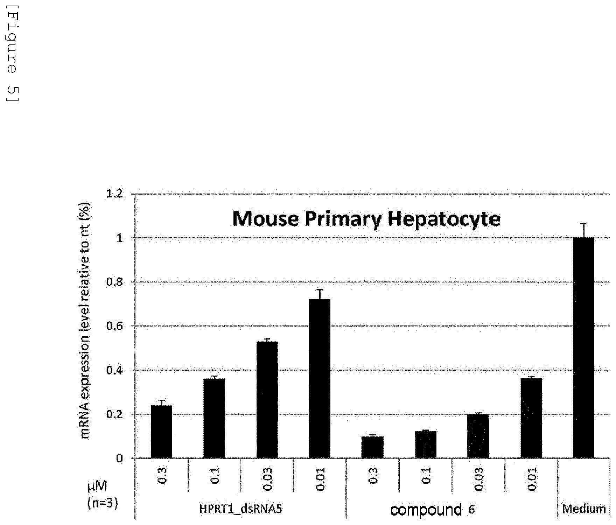

[Figure 5] Figure 5 shows the knock-down activity of compound 6 targeting hypoxanthine-guanine phosphoribosyltransferase 1 (HPRT1) in HeLa cells. This figure shows test results obtained when HPRT1_dsRNA5 and compound 6 were each added at concentrations of 3 µmoL/L, 1 µmoL/L, 0.3 µmol/L, and 0.1 µmol/L to the HeLa cells. Medium depicts test results about an siRNA-unintroduced group (negative control group). The ordinate depicts mean ± standard deviation (n = 3) of a relative ratio of the mRNA level of HPRT1 in a specimen in which each siRNA described above was introduced, when the mRNA level of HPRT1 in the Medium group (negative control group) was defined as 1. HPRT1_dsRNA5 was used as a negative control group for compound 6.

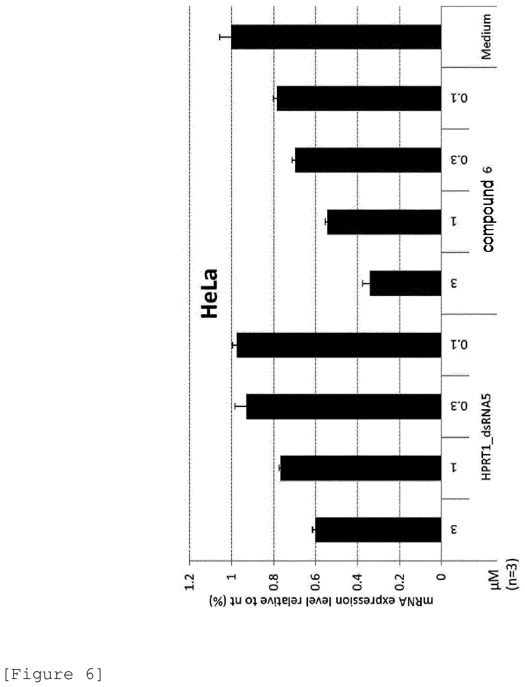

[Figure 6] Figure 6 shows the knock-down activity of compound 6 targeting hypoxanthine-guanine phosphoribosyltransferase 1 (HPRT1) in HepG2 cells. This figure shows test results obtained when HPRT1_dsRNA5 and compound 6 were each added at concentrations of 3 µmoL/L, 1 µmoL/L, 0.3 µmol/L, and 0.1 µmol/L to the HepG2 cells. Medium depicts test results about an siRNA-unintroduced group (control group). The ordinate depicts mean ± standard deviation (n = 3) of a relative ratio of the mRNA level of HPRT1 in a specimen in which each siRNA described above was introduced, when the mRNA level of HPRT1 in Medium (control group) was defined as 1. HPRT1_dsRNA5 is a negative control group for compound 6.

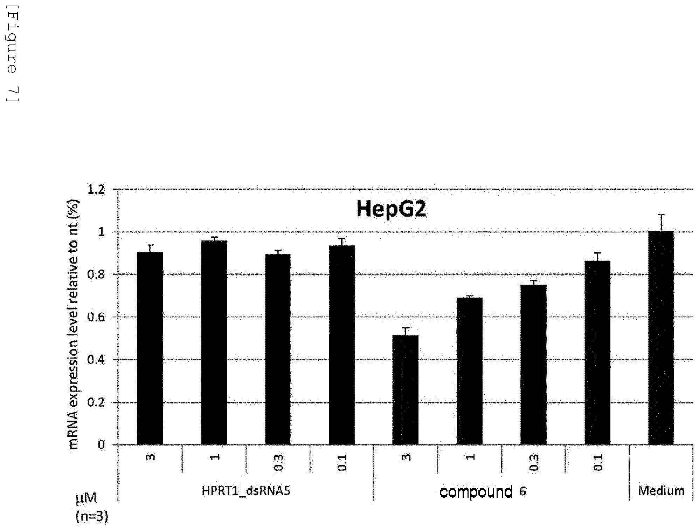

[Figure 7] Figure 7 shows the knock-down activity of compound 6 targeting hypoxanthine-guanine phosphoribosyltransferase 1 (HPRT1) in HuH-7 cells. This figure shows test results obtained when HPRT1_dsRNA5 and compound 6 were each added at concentrations of 3 µmoL/L, 1 µmoL/L, 0.3 µmol/L, and 0.1 µmol/L to the HuH-7 cells. Medium depicts test results about an siRNA-unintroduced group (control group). The ordinate depicts mean ± standard deviation (n = 3) of a relative ratio of the mRNA level of HPRT1 in a specimen in which each siRNA described above was introduced, when the mRNA level of HPRT1 in Medium (control group) was defined as 1. HPRT1_dsRNA5 is a negative control group for compound 6.

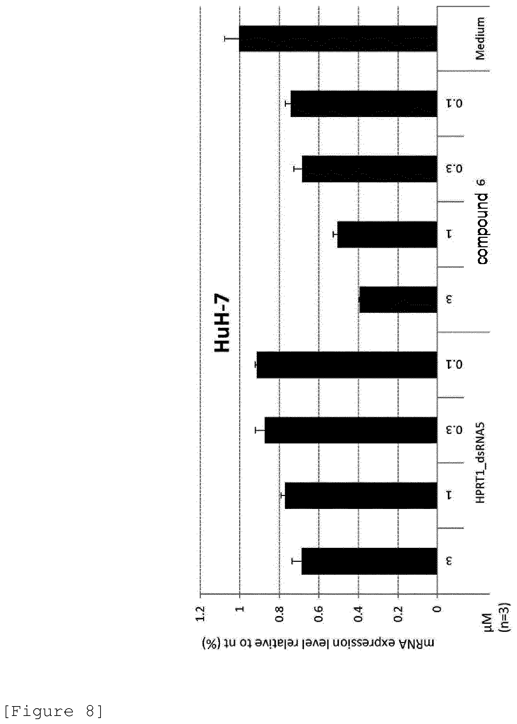

[Figure 8] Figure 8 shows the knock-down activity of compound 6 targeting hypoxanthine-guanine phosphoribosyltransferase 1 (HPRT1) in RAW264.7 cells. This figure shows test results obtained when HPRT1_dsRNA5 and compound 6 were each added at concentrations of 3 µmoL/L, 1 µmoL/L, 0.3 µmol/L, and 0.1 µmol/L to the RAW264.7 cells. Medium depicts test results about an siRNA-unintroduced group (control group). The ordinate depicts mean ± standard deviation (n = 3) of a relative ratio of the mRNA level of HPRT1 in a specimen in which each siRNA described above was introduced, when the mRNA level of HPRT1 in Medium (control group) was defined as 1. HPRT1_dsRNA5 is a negative control group for compound 6.
[Figure 9] Figure 9 shows the residual rate of an antisense strand of compound 6 targeting hypoxanthine-guanine phosphoribosyltransferase 1 (HPRT1) in rat serum. The abscissa depicts a reaction time, which was a time from the start of addition of HPRT1_dsRNA5 and compound 6 to the rat serum. The ordinate depicts mean ± standard deviation (n = 3) of a relative residual rate at each point in time when the antisense strand level at the start of reaction was defined as 100%. HPRT1_dsRNA5 was used as a negative control group for compound 6.
[Figure 10] Figure 10 shows the residual rate of an antisense strand of compound 6 targeting hypoxanthine-guanine phosphoribosyltransferase 1 (HPRT1) in an exonuclease-containing solution. The abscissa depicts a reaction time, which was a time from the start of addition of HPRT1_dsRNA5 and compound 6 to the exonuclease-containing solution. The ordinate depicts mean ± standard deviation (n = 3) of a relative residual rate at each point in time when the antisense strand level at the start of reaction was defined as 100%. HPRT1_dsRNA5 is a negative control group for compound 6.
[Figure 11] Figure 11 shows the knock-down activity of compound 7 targeting phosphatase and tensin homolog deleted from chromosome 10 (PTEN) and compound 8 targeting Factor 9 in mouse primary hepatocytes.
[Figure 12] Figure 12 shows the knock-down activity of each compound targeting HPRT1 in HeLa cells.
[Figure 13] Figure 13 shows the knock-down activity of each compound targeting HPRT1 in HeLa cells.
[Figure 14] Figure 14 shows the knock-down activity of each compound targeting HPRT1 in HeLa cells.
[Figure 15] Figure 15 shows the knock-down activity of each compound targeting HPRT1 in HeLa cells.
[Figure 16] Figure 16 shows the knock-down activity of each compound targeting HPRT1 in HeLa cells.
[Figure 17] Figure 17 shows the knock-down activity of each compound targeting HPRT1 in HeLa cells.
[Figure 18] Figure 18 shows the knock-down activity of compound 47 targeting HPRT1 in HeLa cells.
[Figure 19] Figure 19 shows the knock-down activity of compounds 48 and 49 targeting HPRT1 in mouse primary hepatocytes.
[Figure 20] Figure 20 shows the knock-down activity of compound 50 targeting B2M in mouse primary hepatocytes.
[Figure 21] Figure 21 shows the knock-down activity of compound 51 targeting HPRT1 in HeLa cells.
[Figure 22] Figure 22 shows the knock-down activity of compound 52 targeting PTEN and compound 53 targeting Factor 9 in mouse primary hepatocytes.

### Description of Embodiments

### <Oligonucleotide derivative>

The present invention relates to an oligonucleotide derivative comprising a circular oligonucleotide and a linear oligonucleotide, wherein the circular oligonucleotide and the linear oligonucleotide have base sequences complementary to each other, and form a complex via a hydrogen bond between the complementary base sequences. In the present specification, the oligonucleotide derivative according to the present invention is also referred to as a nucleic acid complex.

In the present invention, a circular oligonucleotide and a linear oligonucleotide are used. Each oligonucleotide may be any molecule, regardless of whether to be circular or linear, as long as the molecule is a nucleotide polymer. Examples thereof include DNA which is a deoxyribonucleotide polymer, RNA which is a ribonucleotide polymer, and a chimeric nucleic acid which is a polymer of DNA and RNA.

The oligonucleotide may be a nucleotide polymer comprising a molecule functionally equivalent to a nucleotide instead of the whole or a portion of the nucleotides, and may be a nucleotide polymer derived from DNA, RNA or a chimeric nucleic acid by the substitution of at least one nucleotide (deoxyribonucleotide, ribonucleotide, etc.) with a molecule functionally equivalent to the nucleotide. Uracil (U) in RNA and thymine (T) in DNA can be used interchangeably with each other.

Among the nucleotides constituting the oligonucleotide, all the nucleotides may be molecules functionally equivalent to the nucleotides, or one or some nucleotides may be molecules functionally equivalent to the nucleotides.

Examples of the molecules functionally equivalent to nucleotides include nucleotide derivatives prepared by modifying nucleotides.

Use of such a nucleotide derivative has the advantage that, for example, nuclease resistance can be improved or stabilized, affinity for a complementary strand nucleic acid can be enhanced, and/or cell permeability can be enhanced, as compared with DNA or RNA, though the advantage is not limited thereto.

Examples of the nucleotide derivative include a nucleotide modified at its sugar moiety, a nucleotide modified at its phosphodiester bond, a nucleotide modified at its base, and a nucleotide co-modified at two or more of its sugar moiety, phosphodiester bond and base.

The nucleotide modified at its sugar moiety can be any nucleotide in which the chemical structure of the sugar of the nucleotide is partially or wholly modified or substituted with an arbitrary substituent or substituted with an arbitrary atom. A 2'-modified nucleotide is preferably used.

Examples of the 2'-modified nucleotide include a 2'-modified nucleotide in which the 2'-OH group of ribose is substituted with a substituent selected from the group consisting of -OR, -R, -R'OR, -SH, -SR, -NH₂, -NHR, -NR₂, -N₃ (azide), -CN (cyano), -F, -Cl, -Br and -I (wherein R is alkyl or aryl, preferably alkyl having 1 to 6 carbon atoms; R' is alkylene, preferably alkylene having 1 to 6 carbon atoms; and two R moieties of -NR₂ are the same or different).

A 2'-modified nucleotide in which the 2'-OH group of ribose is substituted with -F, a methoxy group or an ethoxy group is preferably used as the 2'-modified nucleotide.

Examples of the 2'-modified nucleotide also include a 2'-modified nucleotide in which the 2'-OH group of ribose is substituted with a substituent selected from the group consisting of a 2-(methoxy)ethoxy group, a 3-aminopropoxy group, a 2-[(N,N-dimethylamino)oxy]ethoxy group, a 3-(N,N-dimethylamino)propoxy group, a 2-[2-(N,N-dimethylamino)ethoxy]ethoxy group, a 2-(methylamino)-2-oxoethoxy group, a 2-(N-methylcarbamoyl)ethoxy group and a 2-cyanoethoxy group.

Bridged nucleic acid (BNA) having two circular structures by the introduction of a bridged structure to the sugar moiety is also suitably used as another form of the nucleotide modified at its sugar moiety.

Examples of the nucleotide modified at its sugar moiety include locked nucleic acid (LNA) having an oxygen atom at position 2' and a carbon atom at position 4' bridged via methylene [Tetrahedron Letters, 38, 873 (1997); and Tetrahedron, 54, 3607 (1998)], ethylene bridged nucleic acid (ENA) [Nucleic Acid Research, 32, e175 (2004)], constrained ethyl (cEt) [The Journal of Organic Chemistry 75, 1569 (2010)], amido-bridged nucleic acid (AmNA) [Chem Bio Chem 13, 2513 (2012)] and 2'-O,4'-c-spirocyclopropylene bridged nucleic acid (scpBNA) [Chem. Commun., 51, 9737 (2015)].

Examples of the nucleotide modified at its sugar moiety also include peptide nucleic acid (PNA) [Acc. Chem. Res., 32, 624 (1999)], oxy-peptide nucleic acid (OPNA) [J. Am. Chem. Soc., 123, 4653 (2001)] and peptide ribonucleic acid (PRNA) [J. Am. Chem. Soc., 122, 6900 (2000)].

The nucleotide modified at its phosphodiester bond can be any nucleotide in which the chemical structure of the phosphodiester bond of the nucleotide is partially or wholly modified or substituted with an arbitrary substituent or substituted with an arbitrary atom.

Examples of the nucleotide modified at its phosphodiester bond include a nucleotide in which the phosphodiester bond is substituted with a phosphorothioate bond, a nucleotide in which the phosphodiester bond is substituted with a phosphorodithioate bond, a nucleotide in which the phosphodiester bond is substituted with an alkyl phosphonate bond, and a nucleotide in which the phosphodiester bond is substituted with a phosphoramidate bond, and preferably include a nucleotide in which the phosphodiester bond is substituted with a phosphorothioate bond.

The nucleotide modified at its base can be any nucleotide in which the chemical structure of the base of the nucleotide is partially or wholly modified or substituted with an arbitrary substituent or substituted with an arbitrary atom.

Examples of the nucleotide modified at its base include a nucleotide in which an oxygen atom in the base is substituted with a sulfur atom, a nucleotide in which a hydrogen atom in the base is substituted with an alkyl group having 1 to 6 carbon atoms or a halogen group, a nucleotide in which a methyl group in the base is substituted with a hydrogen atom, a hydroxymethyl group or an alkyl group having 2 to 6 carbon atoms, and a nucleotide in which an amino group in the base is substituted with an alkyl group having 1 to 6 carbon atoms, an alkanoyl group having 1 to 6 carbon atoms, an oxo group, a hydroxy group, or the like.

Examples of the nucleotide modified at its base also include a nucleotide in which cytosine (C) is substituted with 5-methylcytosine (5-mC).

The circular oligonucleotide or the linear oligonucleotide in the nucleotide derivative may have two or more of the substitutions described as the nucleotide modified at its sugar moiety, the nucleotide modified at its phosphodiester bond and the nucleotide modified at its base, at the same time in the oligonucleotide moiety.

An oligonucleotide comprising a base sequence complementary to a partial base sequence of mRNA of a target gene, preferably mRNA of a human target gene is referred to as an antisense strand, and an oligonucleotide comprising a base sequence complementary to the base sequence of the antisense strand is referred to as a sense strand. The sense strand and the antisense strand can pair with each other to form a duplex.

An oligonucleotide itself consisting of the partial base sequence of mRNA of a target gene, preferably mRNA of a human target gene may be used as the sense strand.

The circular oligonucleotide according to the present invention may comprise either an antisense strand or a sense strand. The circular oligonucleotide preferably comprises a sense strand. When the circular oligonucleotide comprises an antisense strand, the linear nucleotide preferably comprises a sense strand. When the circular oligonucleotide comprises a sense strand, the linear nucleotide more preferably comprises an antisense strand.

In the oligonucleotide derivative of the present invention, the circular oligonucleotide and the linear oligonucleotide have base sequences complementary to each other, and form a complex via a hydrogen bond between the complementary base sequences.

The oligonucleotide derivative according to the present invention has resistance to nuclease by the formation of a complex between the circular oligonucleotide and the linear oligonucleotide. In the nucleic acid complex, the complementary base sequence moieties respectively carried by the circular oligonucleotide and the linear oligonucleotide may wholly or partially form a duplex.

In the present invention, the circular oligonucleotide has a structure that is cleaved in a cell, and is thereby cleaved and converted into a linear double-stranded oligonucleotide after being delivered to a predetermined cell. The linear double-stranded oligonucleotide usually forms a complex with an intracellular protein called RNA induced silencing complex (RISC) and then initiates the cleavage of target mRNA by RISC, probably exhibiting strong knock-down activity.

In the present invention, when an antisense strand and a sense strand are mentioned, the antisense strand is complementary to a partial base sequence of mRNA of a target gene, and the sense strand is complementary to the antisense strand.

In the present invention, the circular oligonucleotide and the linear oligonucleotide have base sequences complementary to each other.

In the present specification, the term "complementary" not only means that one of the oligonucleotides and the other oligonucleotide have base sequences completely complementary to each other, but means that these oligonucleotides can have 30% or less, 20% or less or 10% or less mismatch bases between the base sequences.

One of the oligonucleotides and the other oligonucleotide may have 1 to 8, preferably 1 to 6, 1 to 4 or 1 to 3, in particular, 2 or 1 mismatch base(s) between the base sequences complementary to each other.

In the present specification, for example, one of the oligonucleotides having a base sequence complementary to the other oligonucleotide may have a base sequence derived from the completely complementary base sequence by the substation, addition and/or deletion of one or more bases.

In the oligonucleotide derivative of the present invention, the base sequences complementary to each other carried by the circular oligonucleotide and the linear oligonucleotide usually have 15 to 27 base pairs, preferably 15 to 25 base pairs, more preferably 19 to 23 base pairs.

The complementary base sequences of the circular oligonucleotide and the linear oligonucleotide are not particularly limited as long as a hydrogen bond is formed therebetween. The bases in the oligonucleotide of the circular oligonucleotide and the bases in the oligonucleotide of the linear oligonucleotide can be opposed to each other so as to form a hydrogen bond therebetween, and may form base pairs or may have a mismatch.

When one of the circular oligonucleotide and the linear oligonucleotide has an antisense strand, the oligonucleotide having the antisense strand may have, in addition to one of the 15- to 27-base pair base sequences complementary to each other, a base sequence having a base length of 1 to 7, preferably a base length of 2 to 4, more preferably a base length of 2, at the 3' end of the base sequence.

The circular oligonucleotide used in the present invention may have only an oligonucleotide and a chemical structure that is cleaved *in vivo* for circularization, or the oligonucleotide and the chemical structure that is cleaved *in vivo* may be linked via a linker such that the oligonucleotide is circularized.

The oligonucleotide of the circular oligonucleotide may be an oligonucleotide consisting only of nucleotides, may be an oligonucleotide comprising a nucleotide derivative, or may be an oligonucleotide having a modification other than nucleotides or nucleotide derivatives.

The oligonucleotide of the circular oligonucleotide has a base sequence having a base length of 10 to 40, more preferably has a base sequence having a base length of 15 to 40, and still more preferably has a base sequence having a base length of 15 to 30.

The linear oligonucleotide used in the present invention consists of an oligonucleotide. The term "linear" means that the whole structure of the linear oligonucleotide is a single-stranded structure in a linear form.

The linear oligonucleotide may be an oligonucleotide consisting only of nucleotides, may be an oligonucleotide comprising a nucleotide derivative, or may be an oligonucleotide having a modification other than nucleotides or nucleotide derivatives.

The linear oligonucleotide preferably has a base sequence having a base length of 15 to 80, more preferably has a base sequence having a base length of 15 to 40, still more preferably has a base sequence having a base length of 19 to 30, and further preferably has a base sequence having a base length of 19 to 25.

In the oligonucleotide derivative of the present invention, preferably, the base length of the circular oligonucleotide is the same as that of the linear oligonucleotide, or is longer than that of the linear oligonucleotide.

As for the lengths of the respective suitable base sequences having a base length of 15 to 80, of the circular oligonucleotide and the linear oligonucleotide, the base length of the oligonucleotide moiety of the circular oligonucleotide is preferably longer by 1 to 10 bases, more preferably by 2 to 8 bases, still more preferably by 4 to 6 bases, than that of the linear oligonucleotide.

The circular oligonucleotide according to the present invention is an oligonucleotide represented by formula 1: 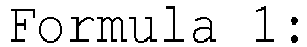 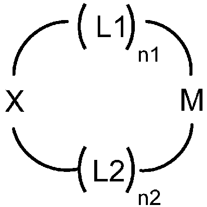 wherein L1 and L2 each represents a linker; n1 and n2 each independently represents an integer of 0 to 10; M represents a moiety comprising a chemical structure that is cleaved by an intracellular environment; and X represents an oligonucleotide.

Examples of the intracellular environment include an intracellular enzyme and intracellular pH.

In formula 1, n1 and n2 may each independently be an integer of 0 to 8, an integer of 0 to 6, an integer of 0 to 4, an integer of 1 to 8, an integer of 2 to 8, an integer of 3 to 8 or an integer of 4 to 8.

When both n1 and n2 in formula 1 are 0, a circular oligonucleotide represented by formula 1-1 wherein L1 and L2 are absent is constituted. 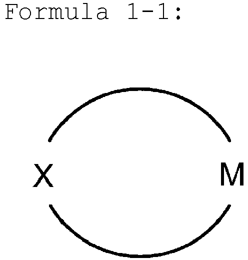 wherein M and X are as defined in formula 1.

When L1 and L2 are absent, the oligonucleotide represented by X is preferably bonded at both of its 5' end and 3' end to M.

L1 is not particularly limited as long as L1 is a structure that links the 5' end of the oligonucleotide represented by X to M. A structure known in the art for use in the modification of a 5' end or a 3' end in oligonucleotide synthesis may be adopted.

When both L1 and L2 are present, L1 and L2 are preferably linked to X or M through a phosphodiester bond, a phosphorothioate bond or a phosphorodithioate bond.

L1 and L2 may have the same structures or different structures.

When each of n1 and n2 is an integer of 2 or larger, the repeat structures of L1 and L2 may each consist of the same structures or may each be a structure where different structures are linked.

M in formula 1 represents a moiety comprising a chemical structure that is cleaved by an intracellular environment.

For example, structures shown in Table 1 are known as the chemical structure that is cleaved by an intracellular environment.

**[Table 1]**

| Intracellular environment | Chemical structure to be cleaved | General name of chemical structure to be cleaved | Reference |
|---|---|---|---|
| Glutathione | -S-S- | Disulfide | J. Med. Chem., 2006, 49, 4392-4408 |
| | -S-S(O)- | Thiosulfinate | |
| | -S-SO₂- | Thiosulfonate | |
| | -S-CO- | Thioester | |
| Thioesterase | -S-CO- | Thioester | |
| Carboxy esterase | -CO-O- | Ester | |
| | -O-CO-NH- | Carbamate | |
| Cathepsin B | -Val-Cit- | Valine-citrulline | Bioconjugate Chem., 2008, 19, 1960-3 |
| | -Phe-Lys- | Phenylalanine-lysine | Bioconjugate Chem., 2002 13, 855-69 |
| | | (Many peptides other than those described above) | INT. J. ONCOL., 2013, 42. 373-383 |
| Serine protease | -His-Ser-Ser-Lys-Leu-Gln- | Histidine-serine-serinelysine-leucine-glutamine | Cancer Res., 1997, 57. 4924-30 |
| β-Glucuronidase | | Glucuronic acid | Biochem . Pharmcol., 1999, 58, 325-328 |
| Low pH | >C=N-O- | Oxime | Proc. NatI. Acad. Sci. USA. |
| | | | 2012, 109, 16101-09 |
| | >C=N- | Imine | |
| | >C=N-NH- | Hydrazone | Science 1994, 261, 212-15 |
| | | | Bioconjugate Chem. , 2002, 13. 40-46 |
| | | Acetal | WO2016063959 A1 |
| | -O-CO-O- | Carbonate | Clin Cancer Res.. 2011, 17, 3157-3169 |
| | -O-Si-O- | Silyl ether | Med. Chem. Common., 2014, 5, 1355-1358 |

The chemical structure that is cleaved by an intracellular environment, represented by M is preferably -S-S-, -C(O)-S, -S-C(O)-.

M is preferably selected from the group consisting of formula 3-1 to formula 3-6: 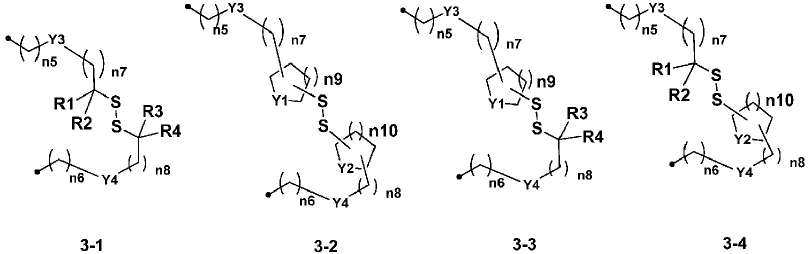 wherein
R1 and R2 each independently represents a hydrogen atom or C1-C3 alkyl, or R1 and R2, together with the carbon atom to which they are bonded, form a ring having 3 to 6 carbon atoms;
R3 and R4 each independently represents a hydrogen atom or C1-C3 alkyl, or R3 and R4, together with the carbon atom to which they are bonded, form a ring having 3 to 6 carbon atoms;
n5 to n8 each independently represents an integer of 0 to 10;
n9 and n10 each independently represents an integer of 1 to 4;
Y1 to Y4 each independently represents a bond, -NR5-, -O- or -S-; and
R5 represents a hydrogen atom, C1-C3 alkyl or C2-C4 alkanoyl, and 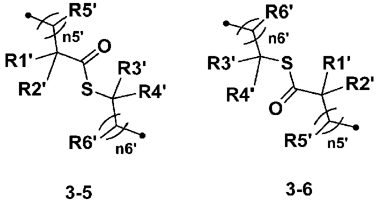 wherein
   R1' and R2' each independently represents a hydrogen atom or C1-C3 alkyl, or R1' and R2', together with the carbon atom to which they are bonded, form a ring having 3 to 6 carbon atoms;
   R3' and R4' each independently represents a hydrogen atom or C1-C3 alkyl, or R3' and R4', together with the carbon atom to which they are bonded, form a ring having 3 to 6 carbon atoms;
   R5' and R6' represent a hydrogen atom or C1-C3 alkyl independently on the basis of each carbon atom to which they are bonded; and
   n5' and n6' each independently represents an integer of 1 to 10.

In formula 3-1, R1 to R4 are preferably each independently a hydrogen atom or C1-C3 alkyl.

In formula 3-1, each of Y3 and Y4 is preferably a bond or -O-.

In formula 3-1, the total of n5 and n7 is preferably an integer of 0 to 5.

In formula 3-1, the total of n6 and n8 is preferably an integer of 0 to 5.

In formula 3-2, Y1 and Y2 are preferably each independently a bond or -O-.

In formula 3-2, each of Y3 and Y4 is preferably a bond.

In formula 3-2, the total of n5 and n7 and the total of n6 and n8 are preferably 0.

In formula 3-2, n9 is preferably 2 when Y1 is -O-, and is preferably 3 when Y1 is a bond.

In formula 3-2, n10 is preferably 2 when Y2 is -O-, and is preferably 3 when Y2 is a bond.

In formula 3-3, each of R3 and R4 is preferably hydrogen.

In formula 3-3, Y1 is preferably a bond or -O-.

In formula 3-3, each of Y3 and Y4 is preferably a bond.

In formula 3-3, the total of n5 and n7 is preferably 0.

In formula 3-3, the total of n6 and n8 is preferably 5.

In formula 3-3, n9 is preferably 2 when Y1 is -O-, and is preferably 3 when Y1 is a bond.

In formula 3-4, each of R1 and R2 is preferably a hydrogen atom.

In formula 3-4, Y2 is preferably a bond or -O-.

In formula 3-4, each of Y3 and Y4 is preferably a bond.

In formula 3-4, the total of n5 and n7 is preferably 5.

In formula 3-4, the total of n6 and n8 is preferably 0.

In formula 3-4, n10 is preferably 2 when Y2 is -O-, and is preferably 3 when Y2 is a bond.

In formula 3-5, each of R1' and R2' is preferably a hydrogen atom.

In formula 3-5, each of R3' and R4' is preferably a hydrogen atom.

In formula 3-5, R5' is preferably a hydrogen atom or methyl independently on the basis of each carbon atom to which it is bonded.

In formula 3-5, R6' is preferably a hydrogen atom.

In formula 3-5, n5' is preferably 2.

In formula 3-5, n6' is preferably 2.

In formula 3-6, each of R1' and R2' is preferably a hydrogen atom.

In formula 3-6, each of R3' and R4' is preferably a hydrogen atom.

In formula 3-6, R5' is preferably a hydrogen atom or methyl independently on the basis of each carbon atom to which it is bonded.

In formula 3-6, R6' is preferably a hydrogen atom.

In formula 3-6, n5' is preferably 2.

In formula 3-6, n6' is preferably 2.

Examples of the C1-C3 alkyl include methyl, ethyl, propyl, isopropyl, and cyclopropyl.

The C2-C4 alkanoyl has a structure where C1-C3 alkyl is bonded to a carbonyl group. The C1-C3 alkyl moiety of the C2-C4 alkanoyl is as defined above.

Examples of the ring having 3 to 6 carbon atoms include cyclopropyl, cyclobutyl, cyclopentyl, and cyclohexyl.

M may be a moiety consisting of 2 to 6 amino acid residues.

The structures of L1 and L2 are not particularly limited. Examples of L1 and L2 can include structures of formula 2-1 to formula 2-4: 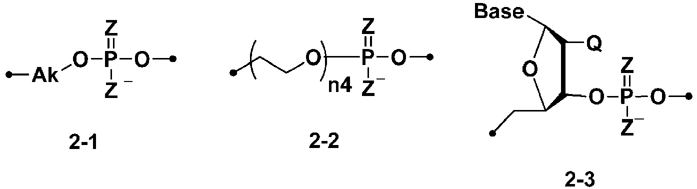 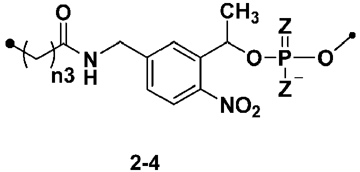 wherein
n3 and n4 each independently represents an integer of 1 to 15;
Ak represents C2-C22 alkylene optionally having a substituent;
Base represents a hydrogen atom, adeninyl optionally having a substituent, guaninyl optionally having a substituent, cytosinyl optionally having a substituent, thyminyl optionally having a substituent or uracinyl optionally having a substituent;
Q represents a hydrogen atom, a hydroxy group, halogen, or a C1-C4 alkyloxy group optionally having a substituent; and
Z represents an oxygen atom or a sulfur atom.

In formula 2-1 to formula 2-4, preferably, one of the Z moieties is an oxygen atom, and the other Z moiety is a sulfur atom.

In formula 2-1, examples of the substituent for the C2-C22 alkylene represented by Ak include an aryl group optionally having a substituent, an amino group optionally having a substituent, and an alkoxy group optionally having a substituent.

Examples of the aryl group optionally having a substituent can include the following structure:

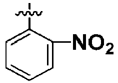

Examples of the amino group optionally having a substituent can include the following structures:

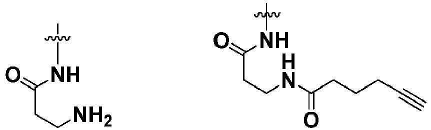

Examples of the alkoxy group optionally having a substituent can include the following structure:

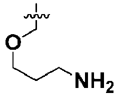

In formula 2-2, n4 may be an integer of 1 to 12, an integer of 1 to 9, an integer of 1 to 6 or an integer of 1 to 3.

In formula 2-3, Base is preferably uracinyl optionally having a substituent. Examples of the uracinyl optionally having a substituent can include the following structures:

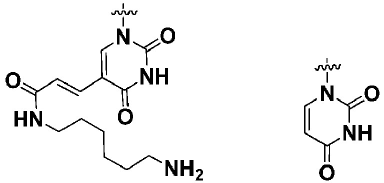

In formula 2-3, Q is preferably a hydrogen atom or a C1-C4 alkyloxy group optionally having a substituent, more preferably a hydrogen atom or a C1-C4 alkyloxy group.

In formula 2-4, n3 may be an integer of 1 to 12, an integer of 1 to 9, an integer of 1 to 6, an integer of 1 to 3 or 3.

In formulas 2-1 to 2-4, examples of the counterion of Z⁻ include, but are not particularly limited to, a proton (H⁺), a metal ion, and an ammonium ion.

Examples of the metal ion include: alkali metal ions such as a sodium ion and a potassium ion; alkaline earth metal ions such as a magnesium ion and a calcium ion; an aluminum ion; and a zinc ion.

Examples of the ammonium ion include an ammonium ion and a tetramethylammonium ion.

When L1 and L2 are present, the black circles in each of formula 3-1 to formula 3-6 are preferably respectively linked to L1 and L2 through a phosphodiester bond or a phosphorothioate bond contained in L1 and L2. Specifically, the black circles in each of formula 3-1 to formula 3-6 are bonded to the black circle from O (oxygen atom) bonded to P (phosphorus atom) in each of the structures represented by formulas 2-1 to 2-4, which are shown as suitable structures of L1 and L2.

In the present specification, when L1 and L2 are present, the upper and lower black circles in each of the structures represented by formula 3-1 to formula 3-6 represent a bond to L1 and a bond to L2, respectively.

The bond represented by the black circle from the carbon atom in each of the structures represented by formula 2-1 to formula 2-4 preferably means a bond to a phosphodiester bond or a phosphorothioate bond located at the 5' end or 3' end of the oligonucleotide.

In the present invention, it should be understood that when the linkage of X to L1 and the linkage of X to L2 are made (also including the case where the circular oligonucleotide does not comprise L1 or L2 so that M and X are directly linked to each other) through a phosphodiester bond or a phosphorothioate bond, the structure derived from the phosphodiester bond or the phosphorothioate bond involved in this bond is a structure carried by the oligonucleotide.

In the present invention, the 5' end and/or 3' end of the linear oligonucleotide may be modified with a phosphoric acid group or a thiophosphoric acid group.

In the present invention, examples of the salt of the oligonucleotide derivative include, but are not particularly limited to, an acid-addition salt, a metal salt, an ammonium salt, an organic amine-addition salt, and an amino acid-addition salt.

Examples of the acid-addition salt include: inorganic acid salts such as hydrochloride, sulfate, and phosphate; and organic acid salts such as acetate, maleate, fumarate, citrate, and methanesulfonate.

Examples of the metal salt include: alkali metal salts such as sodium salt and potassium salt; alkaline earth metal salts such as magnesium salt and calcium salt; and aluminum salt; and zinc salt.

Examples of the ammonium salt include ammonium salt and tetramethylammonium salt.

Examples of the organic amine-addition salt include salts with organic amines such as morpholine and piperidine. Examples of the amino acid-addition salt include salts with amino acids such as lysine, glycine, and phenylalanine.

In the present invention, a targeting compound may be added to an appropriate site of the circular oligonucleotide and/or the linear oligonucleotide. The targeting compound means a group derived from a compound capable of binding to a receptor expressed on a target cell. In the present invention, a targeting compound for the target cell of the oligonucleotide can be selected. The targeting compound is preferably bonded to at least one of L1 and L2. Examples of the targeting compound include cholesterol, tocopherol, docosahexaenoic acid, myristic acid, palmitic acid, and N-acetyl-D-galactosamine. A plurality of ligand-nucleic acid complexes utilizing N-acetyl-D-galactosamine (GalNAc) as a sugar ligand capable of binding to asialoglycoprotein receptor (ASGPR) very highly expressed on hepatocytes have been reported as the targeting compound (e.g., Journal of American Chemical Society, 2014, Vol. 136, p. 16958-16961; and International Publication No. WO 2017/131236).

Examples of the structures of L1 and L2 bonded to the targeting compound include the following formulas 8 and 9: 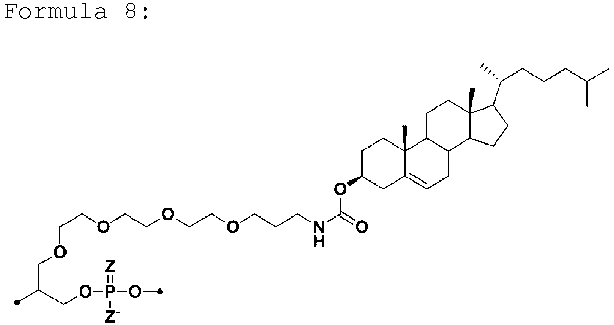 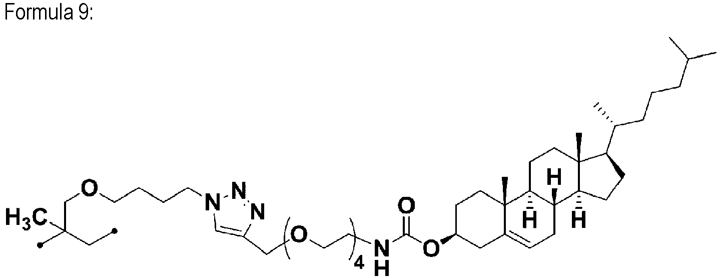 wherein Z is as defined above.

### <Circular oligonucleotide>

The present invention is also directed to the circular oligonucleotide itself contained in the oligonucleotide derivative described above. The circular oligonucleotide comprises at least one phosphorothioate bond and is represented by formula 4: 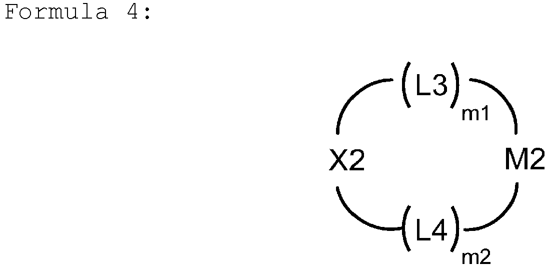 wherein
L3 and L4 each represents a linker;
m1 and m2 each represents an integer of 0 to 10;
M2 represents a moiety comprising a chemical structure that is cleaved by an intracellular environment; and
X2 represents an oligonucleotide.

When both m1 and m2 in formula 4 are 0, a circular oligonucleotide represented by formula 4-1 wherein L3 and L4 are absent is constituted. 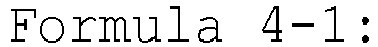 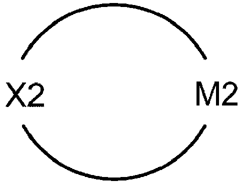 wherein M2 and X2 are as defined in formula 4.

When L3 and L4 are absent, the oligonucleotide represented by X2 is preferably bonded at both of its 5' end and 3' end to M2.

A preferred form of the circular oligonucleotide represented by formula 4 is as described about formula 1, formula 2-1 to formula 2-4, and formula 3-1 to formula 3-6, and L3, L4, m1, m2, M2 and X2 in formula 4 correspond to L1, L2, n1, n2, M and X, respectively, in formula 1.

### <Linear oligonucleotide>

The present invention is also directed to a linear oligonucleotide comprising at least one phosphorothioate bond, the linear oligonucleotide being a precursor of the circular oligonucleotide described above and being represented by formula 7. The linear oligonucleotide itself also has knock-down activity. 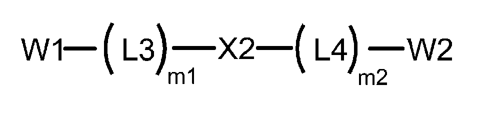 wherein
X2, L3, L4, m1 and m2 are as defined in formula 4; and
W1 and W2 are moieties comprising or forming functional groups reacting with each other to form a chemical structure that is cleaved by an intracellular environment.

The chemical structure that is cleaved by an intracellular environment is as described about M in formula 1.

W1 and W2 are each independently -A1-S-S-A2 or -B1-COO-B2 (wherein the case is excluded where W1 and W2 are -B1-COO-B2 at the same time).

A1 and B1 are each independently C2-C10 alkylene optionally having a substituent.

A2 is C1-C10 alkyl optionally having a substituent. The substituent is preferably a hydroxyl group.

B2 is a hydrogen atom or C1-C6 alkyl optionally having a substituent.

### <Method for producing oligonucleotide derivative>

The oligonucleotide derivative described above can be produced by, for example, a method comprising the steps of: circularizing a linear oligonucleotide represented by formula 7 to form a circular oligonucleotide represented by formula 4; and complexing the circular oligonucleotide represented by formula 4 with a linear oligonucleotide having a base sequence complementary to the circular oligonucleotide via a hydrogen bond.

One example of the method for producing the oligonucleotide derivative of the present invention will be shown.

The linear oligonucleotide can be produced by a chemical synthesis method known in the art. Examples of such a chemical synthesis method include phosphoramidite method, phosphorothioate method, phosphotriester method, and CEM method (see Nucleic Acids Research, 35, 3287 (2007)).

Specifically, the linear oligonucleotide can be synthesized using ABI3900 high-throughput nucleic acid synthesizer (manufactured by Applied Biosystems, Inc.).

Specifically, the circular oligonucleotide can be synthesized by appropriately adopting reagents appropriate for structures corresponding to L1, L2 and M, with reference to a method described in Examples. The oligonucleotide moiety of the circular oligonucleotide can be produced in the same way as in the linear oligonucleotide.

Alternatively, structures corresponding to L1, L2 and M are constructed on a solid phase by use of a solid-phase method. Then, the structure that is cleaved in a cell, represented by M, is chemically constructed while the oligonucleotide can be circularized.

The solid-phase reagent or the amidite for use in the solid-phase method can be obtained as a commercially available product or by a method known in the art (Bioconjugate Chem., Vol. 20, No. 6, p. 1065-1094, 2009) or a method equivalent thereto.

In addition, the solid-phase reagent or the amidite can also be produced by methods given below. In the production methods given below, the compound of interest can be produced by use of methods for introducing and removing protective groups commonly used in synthetic organic chemistry [e.g., methods described in Protective Groups in Organic Synthesis, third edition, T.W. Greene, John Wiley & Sons Inc. (1999)] when a defined group is altered under conditions of the production methods or is inappropriate for carrying out the production methods. If necessary, the order of reaction steps such as substituent introduction may be changed.

### <Method A for producing solid-phase reagent>

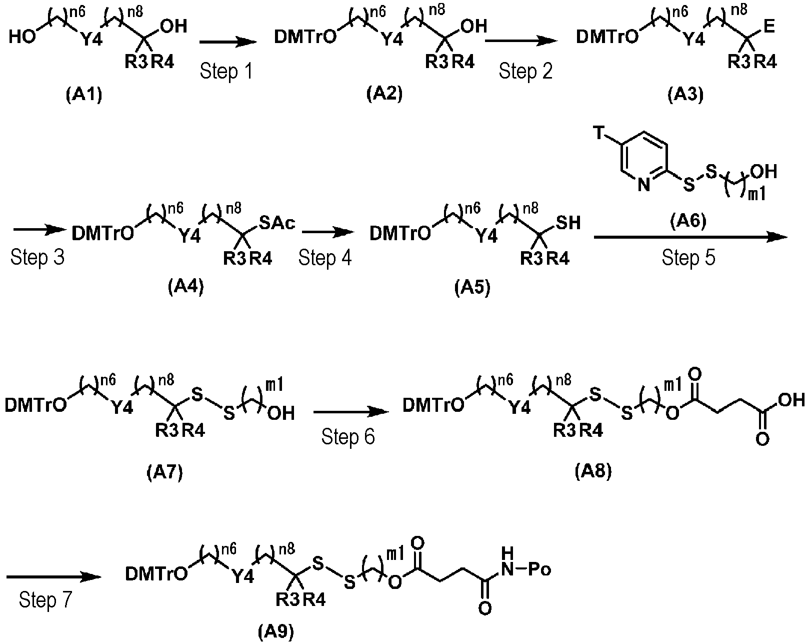

wherein R3, R4, Y4, n6, and n8 are each as defined above; m1 represents an integer of 2 to 20; E represents a leaving group such as a chlorine atom, a bromine atom, an iodine atom, trifluoromethanesulfonyloxy, methanesulfonyloxy, benzenesulfonyloxy, ρ-toluenesulfonyloxy, or 2-nitrobenzenesulfonyloxy; T represents a hydrogen atom or a nitro group; Ac represents an acetyl group; DMTr represents a dimethoxytrityl group; and Po represents a solid-phase reagent such as CPG (controlled pore glass) or a polymer.

### Step 1

Compound (A2) can be produced by reacting compound (A1) with 1 equivalent or more of p,p'-dimethoxytrityl chloride in a pyridine solvent, if necessary in the presence of a cosolvent, at a temperature between 0°C and the boiling point of the solvent for 5 minutes to 100 hours.

Examples of the cosolvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide (DMF), N,N-dimethylacetamide, N-methylpyrrolidone, and water. These cosolvents are used alone or as a mixture.

Compound (A1) can be obtained as a commercially available product or by a method known in the art (e.g., The Fourth Series of Experimental Chemistry 19 "Organic Compound Synthesis I", Maruzen Co., Ltd. (1992) and The Fourth Series of Experimental Chemistry 20 "Organic Compound Synthesis II", Maruzen Co., Ltd. (1992)) or a method equivalent thereto.

### Step 2

Compound (A3) can be produced by reacting compound (A2) with 1 equivalent or more of a halogenating reagent or a sulfonylating reagent in a solvent in the presence of 1 equivalent or more of a base at a temperature between -20°C and the boiling point of the solvent used for 5 minutes to 24 hours.

Examples of the halogenating reagent and the sulfonylating reagent include thionyl chloride, sulfuryl chloride, phosphorus trichloride, phosphorus pentachloride, phosphorus oxychloride, phosphorus tribromide, hydrogen bromide, hydrogen iodide, methanesulfonyl chloride (MsCl), methanesulfonic anhydride, p-toluenesulfonyl chloride (TsCl), o-nitrobenzenesulfonyl chloride (NsCl), and trifluoromethanesulfonic anhydride.

Examples of the solvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide (DMF), N,N-dimethylacetamide, N-methylpyrrolidone, pyridine, and water. These solvents are used alone or as a mixture.

Examples of the base include cesium carbonate, potassium carbonate, potassium hydroxide, sodium hydroxide, sodium methoxide, potassium tert-butoxide, triethylamine, diisopropylethylamine, N-methylmorpholine, pyridine, 1,8-diazabicyclo[5.4.0]-7-undecene (DBU), and N,N-dimethyl-4-aminopyridine (DMAP).

### Step 3

Compound (A4) can be produced by reacting compound (A3) with 1 equivalent or more of thioacetic acid or thioacetic acid S-potassium salt in a solvent, if necessary in the presence of 0.01 to 30 equivalents of an additive, at a temperature between room temperature and the boiling point of the solvent used for 5 minutes to 100 hours.

Examples of the solvent include those listed in step 2.

Examples of the additive include sodium iodide, potassium iodide, tetrabutylammonium iodide (TBAI), tetrabutylammonium chloride, and 18-crown-6-ether.

### Step 4

Compound (A5) can be produced by reacting compound (A4) without a solvent or in a solvent in the presence of 1 to 100 equivalents of a primary or secondary amine at a temperature between room temperature and the boiling point of the solvent used for 5 minutes to 100 hours.

Examples of the solvent include those listed in step 2.

Examples of the primary amine include methylamine and ethylamine. Examples of the secondary amine include diethylamine, diisopropylamine, and piperidine.

### Step 5

Compound (A7) can be produced by reacting compound (A5) with 1 equivalent or more of compound (A6) in a solvent, if necessary in the presence of a base, at a temperature between room temperature and the boiling point of the solvent used for 5 minutes to 100 hours.

Examples of the solvent include those listed in step 2.

Examples of the base include those listed in step 2.

Compound (A6) can be produced by the following step 5-1: 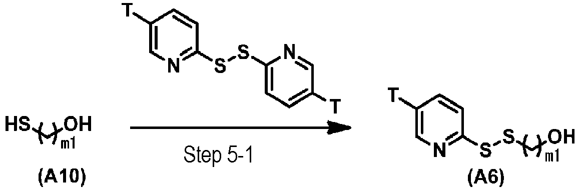 wherein m1 and T are as defined above.

Compound (A10) can be obtained as a commercially available product.

### Step 6

Compound (A8) can be produced by reacting compound (A7) with 1 equivalent or more of succinic anhydride in a solvent in the presence of 1 equivalent or more of a base at a temperature between room temperature and the boiling point of the solvent used for 5 minutes to 100 hours.

Examples of the solvent include those listed in step 2.

Examples of the base include those listed in step 2.

### Step 7

Compound (A9) can be produced by reacting compound (A8) with a terminally aminated solid-phase reagent without a solvent or in a solvent in the presence of 1 to 50 equivalents of a base, a condensing agent and, if necessary, 0.01 to 30 equivalents of an additive, at a temperature between room temperature and the boiling point of the solvent used for 5 minutes to 200 hours, then temporarily isolating the solid-phase reagent, and further reacting the resultant in an acetic anhydride/pyridine mixed solution at a temperature from room temperature to 200°C for 5 minutes to 100 hours.

Examples of the solvent include those listed in step 2.

Examples of the base include those listed in step 2.

Examples of the condensing agent include 1,3-dicyclohexanecarbodiimide (DCC), 1-ethyl-3-(3-dimethylaminopropyl)carbodiimide hydrochloride (EDC), N,N'-carbonyldiimidazole (CDI), N,N'-diisopropylcarbodiimide (DIC), benzotriazol-1-yloxytris(dimethylamino)phosphonium hexafluorophosphate, (benzotriazol-1-yloxy)tripyrrolidinophosphonium hexafluorophosphate, O-(7-azabenzotriazol-1-yl)-N,N,N',N'-tetramethyluronium hexafluorophosphate (HATU), O-(benzotriazol-1-yl)-N,N,N',N'-tetramethyluronium hexafluorophosphate (HBTU), and 2-chloro-1-methylpyridinium iodide.

Examples of the additive include 1-hydroxybenzotriazole (HOBt) and 4-dimethylaminopyridine (DMAP).

Examples of the aminated solid-phase reagent include long-chain alkylamine controlled pore glass (LCAA-CPG). Such an aminated solid-phase reagent can be obtained as a commercially available product.

Compound (A5) can also be produced by the following step 8: 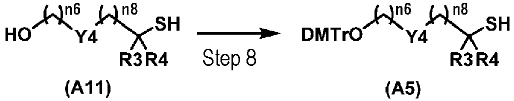 wherein R3, R4, Y4, n6, n8, and DMTr are each as defined above.

Compound (A5) can be produced in the same way as in step 1 using compound (A11).

Compound (A11) can be obtained as a commercially available product or by a method known in the art (e.g., Bioconjugate Chem., Vol. 22, No. 4, p. 717-727, 2011; and The Fourth Series of Experimental Chemistry 24 "Organic Compound Synthesis VI", Maruzen Co., Ltd. (1992)) or a method equivalent thereto.

Among compounds (A11), compound (All-a) wherein R4 is hydrogen, n6 is 1, n8 is 1, and Y4 is a bond can be produced by the following method: 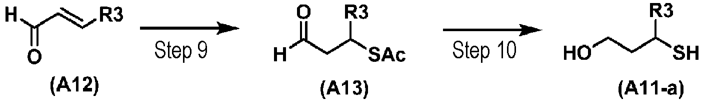 wherein R3 and Ac are as defined above.

### Step 9

Compound (A13) can be produced in the same way as in step 3 using compound (A12).

Compound (A12) can be obtained as a commercially available product.

### Step 10

Compound (All-a) can be produced by reacting compound (A13) with a reducing agent in a solvent at a temperature between -20°C and the boiling point of the solvent used for 5 minutes to 100 hours.

Examples of the reducing agent include lithium borohydride (LiBH₄), sodium borohydride (NaBH₄), sodium cyanoborohydride (NaBH₃CN), lithium triethylborohydride (LiBHEt₃), lithium aluminum hydride (LAH), and diisobutyl aluminum hydride (DIBAL).

Examples of the solvent include those listed in step 2.

Compound (A7) can also be produced by the following method: 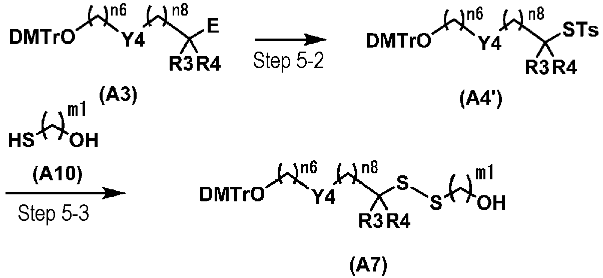 wherein R3, R4, Y4, E, n6, n8, m1 and DMTr are each as defined above, and Ts represents a p-toluenesulfonyl group.

### Step 5-2

Compound (A4') can be produced by reacting compound (A3) in a solvent in the presence of 1 to 100 equivalents of potassium p-toluenethiosulfonate and, if necessary, in the presence of an additive, at a temperature between 0°C and the boiling point of the solvent used for 5 minutes to 48 hours.

Examples of the solvent include those listed in step 2.

Examples of the additive include those listed in step 3.

### Step 5-3

Compound (A7) can be produced in the same way as in step 5 using compound (A4') and compound (A10).

### <Method B for producing solid-phase reagent>

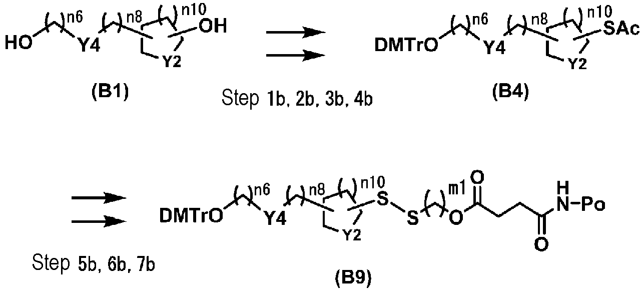

wherein Y2, Y4, n6, n8, n10, m1, DMTr, Ac and Po are each as defined above.

Compound (B9) can be produced in the same way as in method A for producing the solid-phase reagent except that compound (A1) and compound (A4) are changed to compound (B1) and compound (B4), respectively.

Compound (B1) can be obtained as a commercially available product or by a method known in the art (e.g., The Fourth Series of Experimental Chemistry 19 "Organic Compound Synthesis I", Maruzen Co., Ltd. (1992) and The Fourth Series of Experimental Chemistry 20 "Organic Compound Synthesis II", Maruzen Co., Ltd. (1992)) or a method equivalent thereto.

Among compounds (B4), compound (B4-a) wherein Y2 is a bond, n6 is 0, n8 is 0, and Y4 is a bond can be produced by the following method: 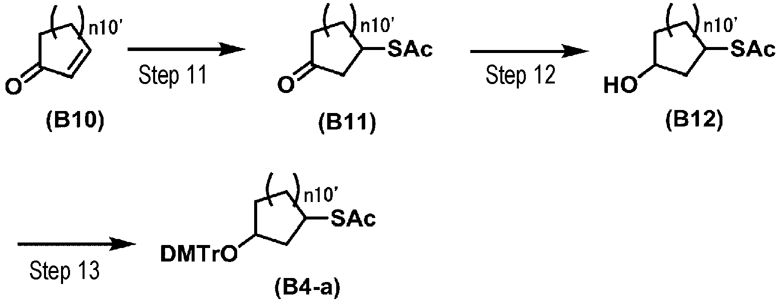 wherein n10' represents an integer of 1 to 3, and Ac and DMTr are each as defined above.

### Step 11

Compound (B11) can be produced in the same way as in step 3 of production method A using compound (B10).

Compound (B10) can be obtained as a commercially available product or by a method known in the art (e.g., The Fourth Series of Experimental Chemistry 21 "Organic Compound Synthesis III", Maruzen Co., Ltd. (1992); and J. Org. Chem., Vol. 38, No. 14, p. 2576-2578, 1973) or a method equivalent thereto.

### Step 12

Compound (B12) can be produced in the same way as in step 10 of production method A using compound (B11).

### Step 13

Compound (B4-a) can be produced in the same way as in step 1 of production method A using compound (B12).

### <Method C for producing solid-phase reagent having targeting compound>

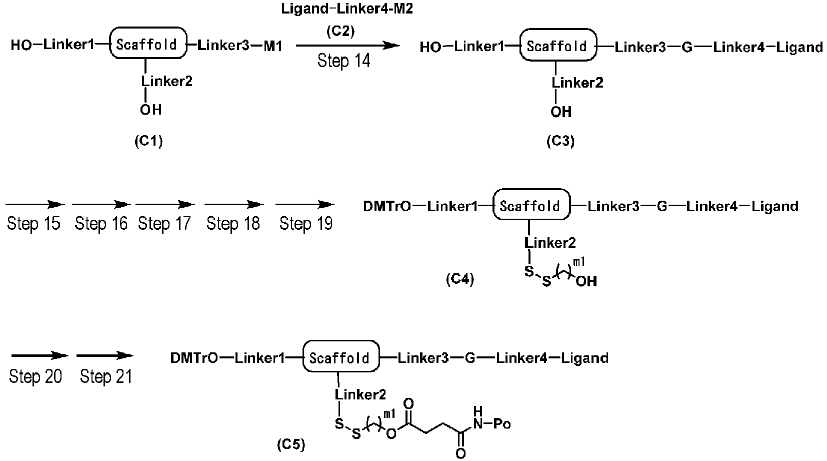

wherein m1 and Po are as defined above; M1 and M2 each represents -COOH, -NHRa, -OH, -N3, -C≡CH or -SH; G represents -C(O)-NRa-, -NRa-C(O)-, -C(O)-S-, -S-C(O)-, or triazolediyl; Ra represents a hydrogen atom or C1-C3 alkyl; Linker1, Linker2, Linker3, and Linker4 each represents a linker; Scaffold represents a structure having -Linker1-OH, -Linker2-OH, -Linker3-M1 as a substituent; and Ligand represents a targeting compound.

Scaffold is not particularly limited as long as its structure has -Linker1-OH, -Linker2-OH, -Linker3-M1 as a substituent.

### Step 14

Compound (C3) can be produced by reacting compound (C1) with compound (C2) in a solvent in the presence of 1 to 50 equivalents of a base, 1 equivalent or more of a condensing agent and, if necessary, 0.01 to 30 equivalents of an additive, at a temperature between 0°C and the boiling point of the solvent used.

Examples of the solvent include those listed in step 2.

Examples of the base include those listed in step 2.

Examples of the condensing agent include those listed in step 7.

Examples of the additive include those listed in step 7.

Compound (C1) can be any compound having two -OH groups and targeting compound-binding structure M1 in one molecule, and can be obtained as a commercially available product or by a method known in the art (e.g., corresponding structures are disclosed in International Publication Nos. WO 2015/006740 and WO 2015/105083) or a method equivalent thereto.

Compound (C2) can be any compound having a targeting compound, a linker and Scaffold-binding structure M2 in one molecule, and can be obtained as a commercially available product or by a method known in the art (e.g., Therapeutic Delivery, Vol. 4, p. 791-809 (2013)) or a method equivalent thereto.

When G in compound (C3) is triazolediyl, M1 in compound (C1) is -N3 or -C≡CH, and M2 in compound (C2) is -N3 or -C≡CH (wherein M1 and M2 are not -N3 or -C≡CH at the same time). This compound (C3) can be produced by reacting compound (C1) with compound (C2) in a solvent in the presence of 0.01 to 10 equivalents of a metal catalyst, 0.01 to 10 equivalents of a reducing agent and, if necessary, 0.01 to 10 equivalents of an added reaction accelerator, at a temperature between 0°C and the boiling point of the solvent used for 5 minutes to 48 hours.

Examples of the metal catalyst include copper(II) sulfate pentahydrate, copper(I) bromide, and pentamethylcyclopentadienylbis(triphenylphosphonium)ruthe nium(II) chloride.

Examples of the reducing agent include sodium ascorbate and tris(2-carboxyethyl)phosphine (TCEP).

Examples of the reaction accelerator include tris[(1-benzyl-1H-1,2,3-triazol-4-yl)methyl]amine (TBTA) and tris(2-benzimidazolylmethyl)amine ((BimH)₃).

### Step 15 to Step 19

Compound (C4) can be produced in the same way as in steps 1 to 5 or in the same way as in step 1, step 2, step 5-2 and step 5-3 using compound (C3).

### Step 20 and Step 21

Compound (C5) can be produced in the same way as in step 6 and step 7 using compound (C4).

### <Method D for producing amidite>

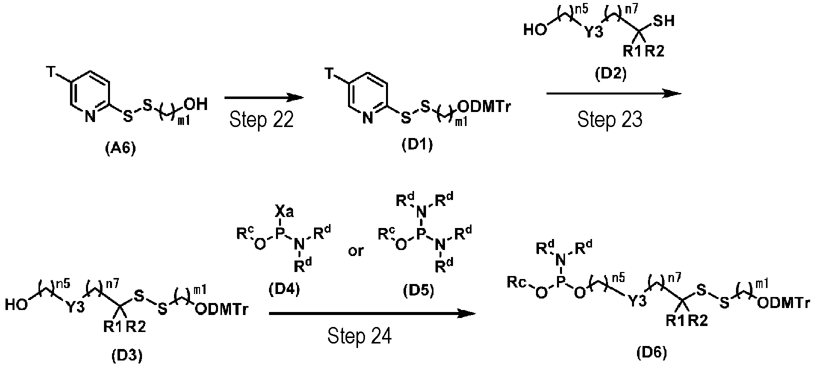

wherein R1, R2, Y3, n5, n7, m1, T, and DMTr are each as defined above; Xa represents a chlorine atom, a bromine atom or an iodine atom; R^{c} represents a protective group, for example, 2-cyanoethyl, which can be removed by base treatment; and R^{d} represents C1-C3 alkyl optionally having a substituent.

### Step 22

Compound (D1) can be produced in the same way as in step 1 using compound (A6).

### Step 23

Compound (D3) can be produced in the same way as in step 5 using compound (D1) and compound (D2).

Compound (D2) can be obtained as a commercially available product or by a method equivalent to the method for producing compound (A11).

### Step 24

Compound (D6) can be produced by reacting compound (D3) with compound (D4) in a solvent in the presence of a base at a temperature between 0°C and the boiling point of the solvent used for 10 seconds to 24 hours.

Examples of the solvent include dichloromethane, acetonitrile, toluene, ethyl acetate, THF, 1,4-dioxane, DMF, and NMP. These solvents are used alone or as a mixture.

Examples of the base include triethylamine, N,N-diisopropylethylamine, and pyridine. These bases are used alone or as a mixture.

Compound (D6) can also be produced by reacting compound (D3) with compound (D5) in a solvent in the presence of a reaction accelerator at a temperature between 0°C and the boiling point of the solvent used for 10 seconds to 24 hours.

Examples of the solvent include acetonitrile and THF. These solvents are used alone or as a mixture.

Examples of the reaction accelerator include 1H-tetrazole, 4,5-dicyanoimidazole, 5-ethylthiotetrazole, and 5-benzylthiotetrazole.

Compound (D4) and compound (D5) can be obtained as commercially available products.

### <Method E for producing amidite>

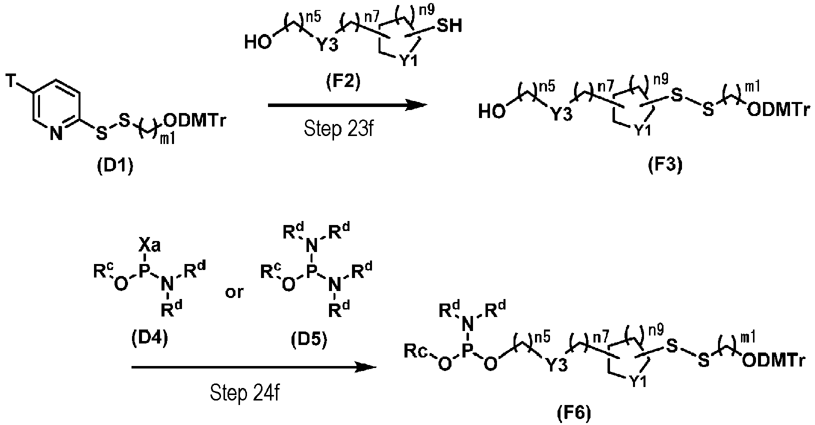

wherein Y1, Y3, n5, n7, n9, m1, T, DMTr, Xa, R^{c} and R^{d} are as defined above.

### Step 23f and Step 24f

Compound (F6) can be produced in the same way as in step 23 and step 24 except that compound (F2) is used instead of compound (D2).

Compound (F2) can be obtained as a commercially available product or by a method equivalent to the method for producing compound (A11).

### <Method F for producing amidite>

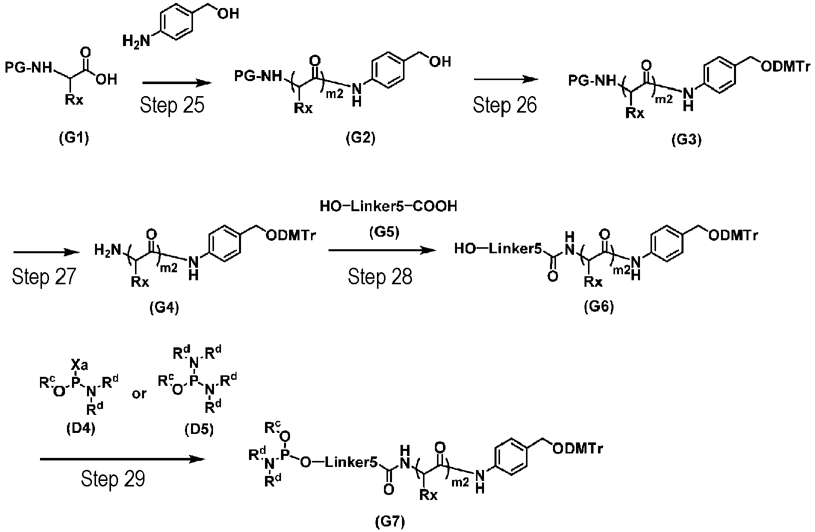

wherein DMTr, Xa, R^{c}, and R^{d} are as defined above; PG represents a protective group; m2 represents an integer of 1 to 10; Rx represents a substituent carried by a natural or non-natural α-amino acid residue; and Linkers represents a linker that connects the hydroxyl group to the carboxyl group.

### Step 25

Compound (G2) can be produced in the same way as in step 14 using α-amino acid (G1) with an amino group protected with an appropriate protective group, and p-aminobenzyl alcohol.

p-Aminobenzyl alcohol and compound (G1) can be obtained as commercially available products.

The obtained compound (G2) can be repetitively subjected to deprotection of the amino group and subsequent condensation reaction with compound (G1) to produce compound (G2) wherein m2 is adjusted to the desired value.

The amino group can be deprotected by use of an appropriate method commonly used in organic synthetic chemistry [e.g., a method described in Protective Groups in Organic Synthesis, Third Edition, T.W. Greene, John Wiley & Sons Inc. (1999)].

### Step 26

Compound (G3) can be produced in the same way as in step 1 using compound (G2).

### Step 27

Compound (G4) can be produced by use of the aforementioned appropriate deprotection of the amino group commonly used in organic synthetic chemistry.

### Step 28

Compound (G6) can be produced in the same way as in step 14 using compound (G4) and compound (G5).

Compound (G5) is not particularly limited as long as the compound has a hydroxyl group and a carboxyl group in one molecule. Compound (G5) can be obtained as a commercially available product.

### Step 29

Compound (G7) can be produced in the same way as in step 24 using compound (G6).

When moiety M comprising a chemical structure that is cleaved by an intracellular environment comprises -S-S-, the circular oligonucleotide can be produced by a method given below.

### <Method G for producing circular oligonucleotide>

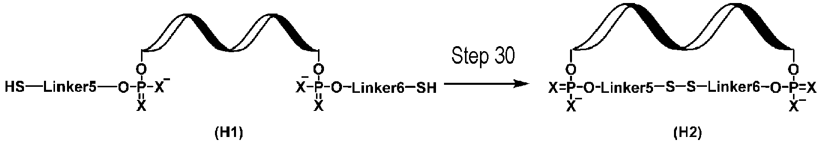

wherein X is as defined above; Linkers and Linker6 each represents a linker; and the ribbon-like structures each represents an oligonucleotide.

### Step 30

### Condition a

Compound (H2) can be produced by reacting compound (H1) in the presence of 1 equivalent or more of a base and, if necessary, 0.1 equivalents or more of an added additive, at a temperature between -20°C and the boiling point of the solvent used for 5 minutes to 120 hours.

Examples of the solvent include methanol, ethanol, acetonitrile, tetrahydrofuran, dioxane, N,N-dimethylformamide (DMF), N,N-dimethylacetamide, N-methylpyrrolidone, pyridine, water, PBS, a citrate buffer solution, and a Tris buffer solution. These solvents are used alone or as a mixture.

Examples of the base include cesium carbonate, potassium carbonate, sodium bicarbonate, triethylamine, diisopropylethylamine, N-methylmorpholine, and 1,8-diazabicyclo[5.4.0]-7-undecene (DBU).

Examples of the additive include urea, magnesium chloride, and potassium chloride.

### Condition b

Compound (H2) can be produced by dissolving compound (H1) in a solvent, adding 1 equivalent or more of 2-(methoxythio)-3-nitropyridine (Npys-OMe) (manufactured by Kokusan Chemical Co., Ltd.) to the solution, and reacting the mixture at a temperature between -20°C and the boiling point of the solvent used for 5 minutes to 120 hours.

Examples of the solvent include those described in condition a.

### Condition c

Compound (H2) can be produced by dissolving compound (H1) in a solvent, adding 1 equivalent or more of KSH-OMe (Npys-OMe-CHEMMATRIX Resin) (manufactured by Kokusan Chemical Co., Ltd.) to the solution, and reacting the mixture at a temperature between -20°C and the boiling point of the solvent used for 5 minutes to 120 hours.

Examples of the solvent include those described in condition a.

Compound (H1) can be produced by use of a chemical synthesis method known in the art or a method equivalent thereto, using a commercially available reagent or the solid-phase reagent and the amidite reagent described above in the production methods.

Examples of the chemical synthesis method for oligonucleotides known in the art include methods described in the following literatures:
(i) Tetrahedron, Vol. 48, No. 12, p. 2223-2311 (1992);
(ii) Current Protocols in Nucleic Acids Chemistry, John Wiley & Sons, Inc. (2000-2017);
(iii) Protocols for Oligonucleotides and Analogs, Human Press, Inc. (1993); and
(iv) Chemistry and Biology of Artificial Nucleic Acids, Wiley-VCH (2012).

The oligonucleotide can be purified using a C18 reverse-phase column or an anion-exchange column, preferably these two columns in combination.

The purity of the oligonucleotide thus purified is desirably 90% or higher, preferably 95% or higher.

In the present invention, the oligonucleotide derivative is produced by mixing a circular oligonucleotide with a linear oligonucleotide. This production can be performed through usual annealing reaction such that the circular oligonucleotide and the linear oligonucleotide form a hydrogen bond between their complementary base sequences.

Preferably, the oligonucleotide derivative can be produced by mixing equal amounts of the circular oligonucleotide and the linear oligonucleotide, for example, in a buffer solution, leaving the mixture standing, for example, at 60 to 95°C for 1 to 15 minutes, and then gradually lowering the temperature to room temperature. Then, the oligonucleotide derivative may be converted to a salt by a common method.

The pharmaceutical composition of the present invention comprises the oligonucleotide derivative or a salt thereof. The pharmaceutical composition of the present invention is administered as a nucleic acid complex, then recognized by a target cell, and introduced into the cell.

A circular oligonucleotide having a chemical structure that is cleaved by an intracellular environment is converted to a linear double-stranded oligonucleotide by intracellular cleavage at a structure other than the oligonucleotide. The linear double-stranded oligonucleotide is usually incorporated into a complex called RNA induced silencing complex (RISC), which in turn cleaves target mRNA and thereby silences a target gene by reducing or halting the expression of the target gene. Thus, such an oligonucleotide can be used in the treatment of a disease associated with the target gene.

The oligonucleotide derivative or a salt thereof of the present invention can be used as an agent for suppressing an expression of a target gene utilizing RNA interference. Also, the linear oligonucleotide represented by formula 7 can be used as an agent for suppressing an expression of target gene utilizing RNA interference.

In the case of using the oligonucleotide derivative or a salt thereof of the present invention, or the pharmaceutical product of the present invention as a therapeutic agent or a prophylactic agent, the administration route is not particularly limited, and an administration route most effective for treatment is desirably used. Examples thereof include intravenous administration, subcutaneous administration, intrathecal administration, intratracheal administration, administration by eye drops, intraocular administration, administration onto the skin, oral administration and intramuscular administration. Intravenous administration or subcutaneous administration is preferred.

The dose differs depending on the medical state or age of a recipient, the administration route, etc. The daily dose in terms of, for example, an antisense strand can be 0.1 µg to 1000 mg for administration and is more preferably 1 to 100 mg for administration.

Examples of a preparation appropriate as the pharmaceutical composition include an injection. A prepared liquid formulation may be directly used, for example, in the form of an injection. A solvent may be removed from the liquid formulation by, for example, filtration or centrifugation, and the residue can be used; the liquid formulation may be freeze-dried and used; and/or a liquid formulation supplemented with, for example, an excipient such as mannitol, lactose, trehalose, maltose or glycine may be freeze-dried and used.

The injection is preferably prepared by mixing the liquid formulation or the solvent-removed or freeze-dried preparation with, for example, water, an acid, an alkali, various buffer solutions, saline or an amino acid transfusion. Alternatively, the injection may be prepared by the addition of, for example, an antioxidant such as citric acid, ascorbic acid, cysteine or ethylenediaminetetraacetic acid (EDTA), or a tonicity agent such as glycerin, glucose or sodium chloride. The injection may be supplemented with, for example, a cryopreserving agent such as glycerin and cryopreserved.

Also described is a method for treating or preventing a disease associated with a target gene by silencing the target gene through reduction or halt of its expression *in vivo,* comprising administering the oligonucleotide derivative as it is, or in the form of a salt or in a form contained in a pharmaceutical composition to a patient in need thereof.

Specific examples of the circular oligonucleotide of the present invention are shown in Tables 2 to 6. However, the circular oligonucleotide of the present invention and the oligonucleotide derivative or a salt thereof comprising the same are not limited thereby. The ribbon-like structures in the tables each represent an oligonucleotide.

**[Table 2]**

| | | | | | |
|---|---|---|---|---|---|
| 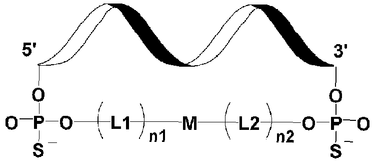 | | | | | |

| **Circular structure** | **L1** | **n1** | **M** | **n2** | **L2** |
|---|---|---|---|---|---|
| **A** | - | **0** | 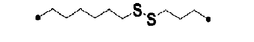 | **0** | **-** |
| **B** | 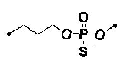 | **4** | 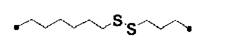 | **4** | 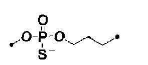 |
| **C** | 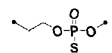 | **4** | 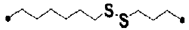 | **4** | 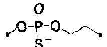 |
| **D** | 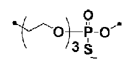 | **3** | 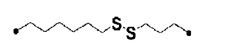 | **3** | 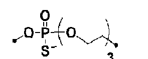 |
| **E** | 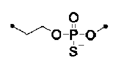 | **4** | 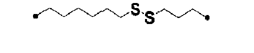 | **0** | **-** |
| **F** | 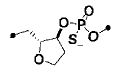 | **4** | 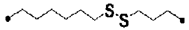 | **0** | **-** |
| **G** | 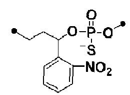 | **4** | 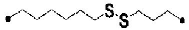 | **0** | **-** |
| **H** | 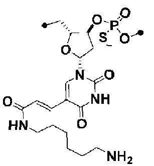 | **4** | 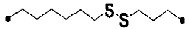 | **0** | - |

**[Table 3]**

| | | | | | |
|---|---|---|---|---|---|
| 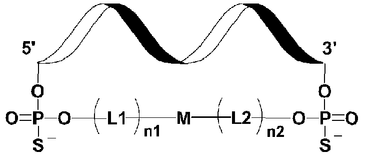 | | | | | |

| **Circular structure** | **L1** | **n1** | **M** | **n2** | **L2** |
|---|---|---|---|---|---|
| **I** | 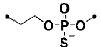 | **8** | 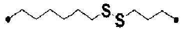 | **0** | **-** |
| **J** | 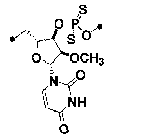 | **2** | 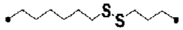 | **0** | |
| **K** | 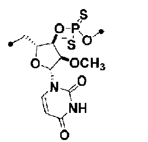 | **4** | 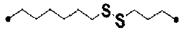 | **0** | |
| **L** | 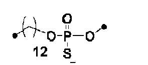 | **1** | 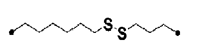 | **0** | |
| **M** | 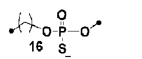 | **1** | 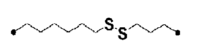 | **0** | |
| **N** | 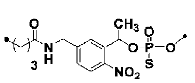 | **4** | 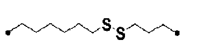 | **0** | |
| **O** | 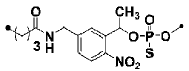 | **4** | 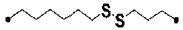 | **4** | 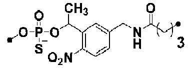 |
| **P** | 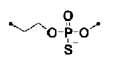 | **8** | 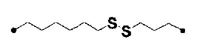 | **8** | 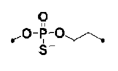 |
| **Q** | 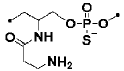 | **4** | 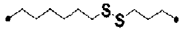 | **0** | |

**[Table 4]**

| | | | | | |
|---|---|---|---|---|---|
| 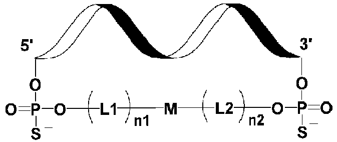 | | | | | |

| **Circular structure** | **L1** | **n1** | **M** | **n2** | **L2** |
|---|---|---|---|---|---|
| **R** | 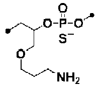 | **1** | 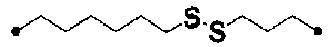 | **0** | - |
| **S** | 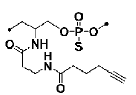 | **4** | 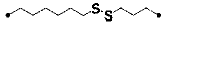 | **0** | - |
| **T** | 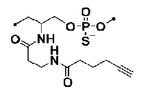 | **4** | 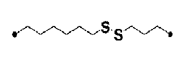 | **4** | 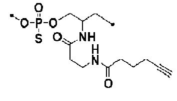 |
| **U** | - | **0** | 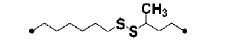 | **0** | - |
| **V** | - | **0** | 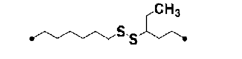 | **0** | - |
| **W** | - | **0** | 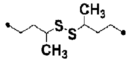 | **0** | - |
| **X** | - | **0** | 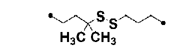 | **0** | - |
| **Y** | - | **0** | 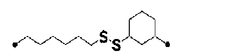 | **0** | - |
| **Z** | - | **0** | 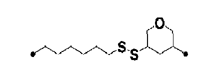 | **0** | - |
| **AA** | - | **0** | 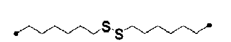 | **0** | - |
| **AB** | - | **0** | 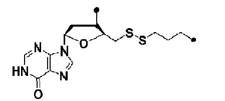 | **0** | - |

**[Table 5]**

| | | | | | |
|---|---|---|---|---|---|
| 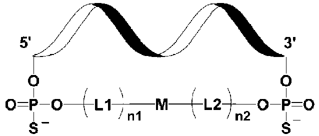 | | | | | |

| | **L1** | **n1** | **M** | **n2** | **L2** |
|---|---|---|---|---|---|
| **AC** | - | **0** | 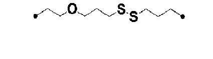 | **0** | **-** |
| **AD** | **-** | **0** | 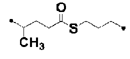 | **0** | **-** |
| **AE** | | **0** | 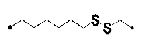 | **1** | 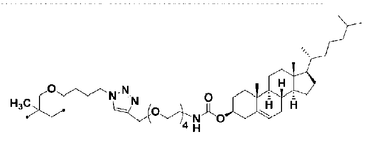 |
| **AF** | **-** | **0** | 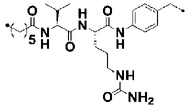 | **1** | 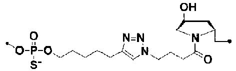 |

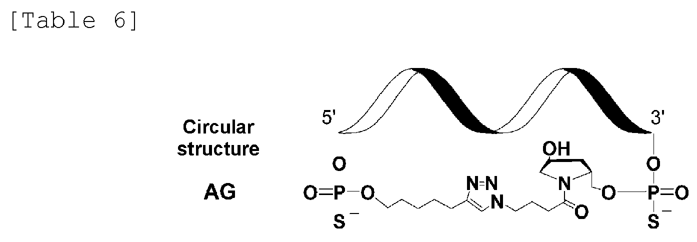

### Examples

Next, the present invention will be specifically described with reference to Examples. However, the present invention is not limited by these Examples.

The oligonucleotide derivative of the present invention was synthesized according to the synthesis route shown in the following scheme 1: 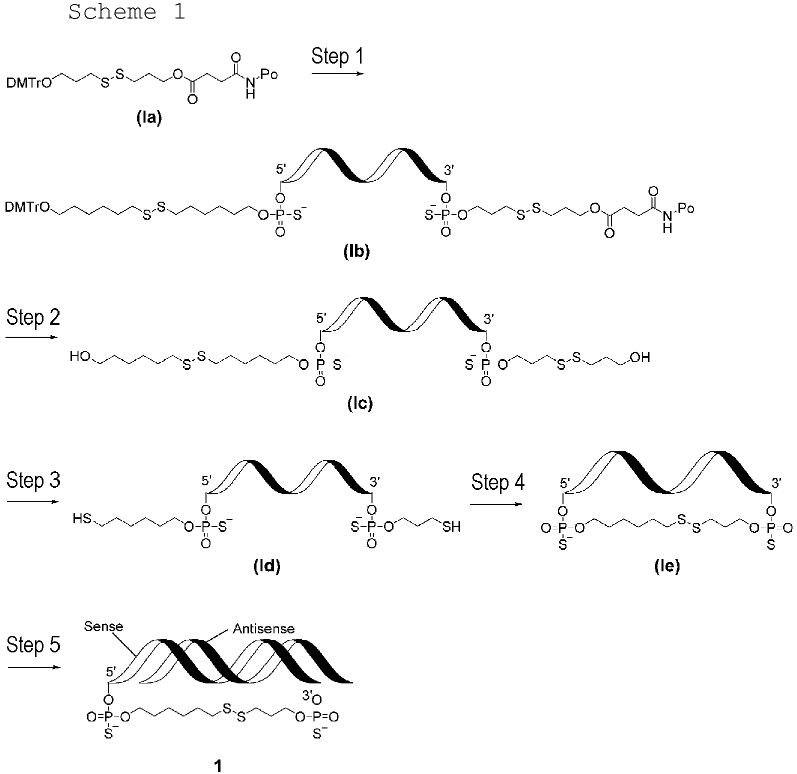 wherein Po is as defined above.

### Example 1

HPRT1_csRNA1 (compound Ie) targeting HPRT1 was synthesized according to the following steps:

### Step 1

Commercially available 2'-OMe-A-CE phosphoramidite, 2'-OMe-G-CE phosphoramidite, 2'-OMe-C-CE phosphoramidite, 2'-OMe-U-CE phosphoramidite, 2'-F-A-CE phosphoramidite, 2'-F-G-CE phosphoramidite, 2'-F-Ac-C-CE phosphoramidite, 2'-F-U-CE phosphoramidite, and Thiol-Modifier C6 S-S (all of these 9 reagents were obtained from Glen Research Corp.) were each prepared into a 0.1 mol/L solution in acetonitrile. Compound Ib was obtained as a crude product by nucleic acid synthesis using 3'-Thiol-Modifier C3 S-S CPG (compound Ia, Glen Research Corp.) as a solid-phase reagent and each of the prepared phosphoramidite solutions in acetonitrile. Activator 42 (SAFC-PROLIGO) was used in a nucleotide elongation step, and the reaction time was set to 10 minutes. nS-8 manufactured by GeneDesign, Inc. was used as a nucleic acid synthesis apparatus.

### Step 2

Crude product Ib obtained in step 1 was deprotected through reaction with trichloroacetic acid to remove the trityl group. Then, the resultant was treated with a reagent containing a 28% aqueous ammonia solution and a 40% aqueous methylamine solution mixed in equal amounts and thereby excised from the solid-phase reagent. The obtained crude product was purified by reverse-phase liquid chromatography (Waters Corp., Xbridge(Registered Trademark) C18, 4.6 mm × 250 mm, solution A: 0.1% triethylammonium acetate buffer solution, solution B: acetonitrile on a gradient) to obtain compound Ic.

ESI-MS calcd: 8775, found: 8778

### Step 3

To compound Ic (45 nmol) obtained in step 2, 50 mmol/L dithiothreitol (DTT) in a Tris buffer solution was added, and the mixture was left standing at room temperature for 18 hours. The reaction solution was purified using NAP-10 column (manufactured by GE Healthcare Japan Corp., product No. 17-0854-01) to obtain compound Id (1.5 mL).

### Step 4

To an aqueous solution (1.5 mL) of compound Id obtained in step 3, 5 mol/L NaCl, triethylamine, and water were added such that the final concentrations were 70 µmol/L compound Id, 150 mmol/L NaCl, and 8 mol% triethylamine. Then, the mixture was left standing at 55°C for 18 hours. The reaction solution was concentrated under reduced pressure and purified by reverse-phase liquid chromatography (Waters Corp., Xbridge (Registered Trademark ) C18, 4.6 mm × 250 mm, solution A: 0.1% triethylammonium acetate buffer solution, solution B: acetonitrile on a gradient) to obtain compound Ie (18 nmol, yield from 2 steps: 40%).

ESI-MS calcd: 8551, found: 8850

### Step 5

Compound Ie obtained in step 4 and oligonucleotide HPRT1_asRNA1 separately prepared using a nucleic acid synthesis apparatus were mixed in equal amounts, dissolved in a citrate buffer solution, and then left standing at 85°C for 5 minutes. Then, the temperature was gradually lowered to obtain compound 1.

In order to identify that compound 1 forms a complex, size-exclusion chromatography (SEC) analysis and polyacrylamide gel electrophoresis (PAGE) analysis were conducted in the same way as in usual identification of siRNA.

### Measurement conditions for SEC analysis

Column: TOSOH G2000SWXL (5 µm, 7.8 mm I.D. × 30 cm)
Solvent: 1 X PBS Buffer (manufactured by Nacalai Tesque, Inc.)
Flow rate: 1 mL/min
Gradient, time: isocratic, 20 min
Column temperature: 25°C
Detection wavelength: 260 nm
Injection volume: 200 pmol/sample
(Reference: Journal of Pharmaceutical and Biomedical Analysis 136 (2017) 55-65)

### Measurement conditions for PAGE analysis

Gel: Multi Gel II Mini 10/20 (13W) (414893, Cosmo Bio Co., Ltd.)
Loading buffer: GelPilot(Registered Trademark) DNA Loading Dye, 5X (239901, Qiagen N.V.)
Marker: DynaMarker(Registered Trademark) dsRNA Easy Load (DM185, BDL)
Electrophoresis tank: AE-6500 (ATTO Corp.)
Migration conditions: 150 V, 200 mA, 20.0 W, 60 min
Migrating solvent: 1 X TAE Buffer
Gel-staining reagent: SYBR(Registered Trademark) Green II Nucleic Acid Gel Stain (50522, Lonza Group AG)

### Example 2

Compound 2 was obtained in the same way as in Example 1 except that the nucleic acid sequences were changed to HPRT1_csRNA2 and HPRT1_asRNA2 described in Table 7.

### Example 3

Compound 3 was obtained in the same way as in Example 1 except that the nucleic acid sequences were changed to B2M_csRNA and B2M_asRNA described in Table 7.

### Example 4

Compound 4 was obtained in the same way as in Example 1 except that the nucleic acid sequences were changed to HPRT1_csRNA3 and HPRT1_asRNA3 described in Table 8.

### Example 5

Compound 5 was obtained in the same way as in Example 1 except that the nucleic acid sequences were changed to HPRT1_csRNA4 and HPRT1_asRNA4 described in Table 8.

### Example 6

Compound 6 was obtained in the same way as in Example 1 except that the nucleic acid sequences were changed to HPRT1_csRNA5 and HPRT1_asRNA5 described in Table 8.

In the tables given below, the column "Name" shows the name of a nucleic acid complex on the upper side and shows the name of circular oligonucleotide/linear oligonucleotide or sense strand/antisense strand constituting the nucleic acid complex on the lower side. The column "Sequence" shows the circular oligonucleotide or the sense strand on the upper side and shows the linear oligonucleotide or the antisense strand on the lower side.

The base sequences of the oligonucleotide derivatives of Examples 1 to 6 and an oligonucleotide as a negative control group are shown in Tables 7 and 8.

The abbreviations, etc. in Tables 7 and 8 are as defined below.

The terminal V represents a bond and means that the S atoms each adjacent to V were bonded to each other (i.e., -S-S-) to form a circular structure.
SC6 = -(CH₂)₆-S-
C3S = -(CH₂)₃-S-
p = Phosphorylated
^ = Phosphorothioate-modified
m = 2'-OMe-modified
f = 2'-F-modified

The molecular weights of circular oligonucleotide Ie of each Example and its pre-precursor compound Ic are shown in Table 9. The molecular weights were measured on the basis of a common method using ESI-MS (manufactured by Agilent Technologies, Inc., 1200 series).

**[Table 7]**

| | Name | Sequence (5' -> 3') |
|---|---|---|
| Negative control group 1 | HPRT1_dsRNA1 HPRT1_ssRNA 1 /HPRT1_asRNA 1 | fA^mA^fGmCfCmAfGmAfCmUfUmUfGmUfUmGfGmAfUmUfU |
| | | mA^fA^mAfUmCfCmAfAmCfAmAfAmGfUmCfUmGfGmC^fU^mU |
| Negative control group 2 | HPRT1_dsRNA2 HPRT1_ssRNA1/HPRT1_asRNA2 | fA^mA^fGmCfCmAfGmAfCmUfUmUfGmUfUmGfGmAfUmUfU |
| | | mA^fA^mAfUmCfCmAfAmCfAmAfAmGfUmCfUmGfGmCfUmU^fA |
| Example 1 | Compound 1 HPRT1_csRNA1/HPRT1_ asRNA1 | V-SC6^fA^mU^fAmUfAmAfGmCfCmAfGmAfCmUfUmUfGmUfUmGfGmAfUmUfU^C3S-V |
| | | mA^fA^mAfUmCfCmAfAmCfAmAfAmGfUmCfUmGfGmC^fU^mU |
| Example 2 | Compound 2 HPRT1_csRNA2/HPRT1_asRNA1 | V-SC6^fG^mG^fAmUfAmUfAmAfGmCfCmAfGmAfCmUfUmUfGmUfUmGfGmAfUmUfU^C3S-V |
| | | mA^fA^mAfUmCfCmAfAmCfAmAfAmGfUmCfUmGfGmC^fU^mU |
| Negative control group 3 | B2M_dsRNA B2M_ssRNA1/B2M_asRNA1 | fAmGfGmAfCmUfGmGfUmCfUfUmUfCmUfAmUfAmU^fC^mU |
| | | fA^mG^fAmUfAmUfAmGfAmAmAfGmAfCmCfAmGfUmC^fC^mU |
| Example 3 | Compound 3 B2M_csRNA1/B2M_asRNA1 | V-SC6^fAmGfCmAfAmGfGmAfCmUfGmGfUmCfUfUmUfCmUfAmUfAmU^fC^mU^C3S-V |
| | | fA^mG^fAmUfAmUfAmGfAmAmAfGmAfCmCfAmGfUmC^fC^mU |

**[Table 8]**

| | Name | Sequence (5' -> 3') |
|---|---|---|
| Negative control group 4 | HPRT1_dsRNA3 HPRT1_ssRNA2/HPRT1_asRNA3 | fUmCfCmUfAmUfGmAfCmUfGmUfAmGfAmUfUmUfUmAfU |
| | | pmAfUmAfAmAfAmUfCmUfAmCfAmGfUmCfAmUfAmG^fG^mA |
| Negative control group 5 | HPRT1_dsRNA4 HPRT1_ssRMA2/HPRT1_asRNA4 | fUmCfCmUfAmUfGmAfCmUfGmUfAmGfAmUfUmUfUmAfU |
| | | pmAfUmAfAmAfAmUfCmUfAmCfAmGfUmCfAmUfAmGfGmA^fA^mU |
| Example 4 | Compound 4 HPRT1_csRNA3/HPRT1_asRNA3 | V-SC6^fCmCfAmUfUmCfCmUfAmUfGmAfCmUfGmUfAmGfAmUfUmUfUmAfU^C3S-V |
| | | pmAfUmAfAmAfAmUfCmUfAmCfAmGfUmCfAmUfAmG^fG^mA |
| Example 5 | Compound 5 HPRT1_csRNA4/HPRT1_asRNA3 | V-SC6^fAmUfCmCfAmUfUmCfCmUfAmUfGmAfCmUfGmUfAmGfAmUfUmUfUmAfU^C3S-V |
| | | pmAfUmAfAmAfAmUfCmUfAmCfAmGfUmCfAmUfAmG^fG^mA |
| Negative control group 6 | HPRT1_dsRNA5 HPRT1_ssRNA3/HPRT1_asRNA5 | fU^mC^fCmUfAmUfGmAfCmUfGmUfAmGfAmUfUmUfU^mA^fU |
| | | pmA^fU^mAfAmAfAmUfCmUfAmCfAmGfUmCfAmUfAmG^fG^mA |
| Example 6 | Compound 6 HPRT1_csRNA5/HPRT1_asRNA5 | V-SC6^fA^mU^fCmCfAmUfUmCfCmUfAmUfGmAfCmUfGmUfAmGfAmUfUmUfU^mA^fU^C3S-V |
| | | pmA^fU^mAfAmAfAmUfCmUfAmCfAmGfUmCfAmUfAmG^fG^mA |

**[Table 9]**

| | MS calcd/found of compound Ic | MS calcd/found of circular oligonucleotide (compound Ie) |
|---|---|---|
| HPRT1_csRNA1 | 8775/8778 | 8551/8550 |
| HPRT1_csRNA2 | 9481/9485 | 9257/9255 |
| B2M_csRNA1 | 8750/8753 | 8526/8526 |
| HPRT1_csRNA3 | 8640/8641 | 8415/8416 |
| HPRT1_csRNA4 | 9291/9292 | 9066/9068 |
| HPRT1_csRNA5 | 9357/9356 | 9133/9131 |

### Example 7

Compound 7 was obtained in the same way as in Example 1 except that the nucleic acid sequences were changed to PTEN_csRNA1 and PTEN_asRNA2 described in Table 10.

### Example 8

Compound 8 was obtained in the same way as in Example 1 except that the nucleic acid sequences were changed to Factor9_csRNA1 and Factor9_asRNA2 described in Table 10.

### Example 9

Compound 9 was obtained in the same way as in Example 1 except that the nucleic acid sequences were changed to HPRT1_csRNA6 and HPRT1_asRNA5 described in Table 11.

### Example 10

Compound 10 was obtained in the same way as in Example 1 except that the nucleic acid sequences were changed to HPRT1_csRNA15 and HPRT1_asRNA5 described in Table 12.

### Example 11

Compound 11 was obtained in the same way as in Example 1 except that the nucleic acid sequences were changed to HPRT1_csRNA16 and HPRT1_asRNA5 described in Table 12.

### Example 12

Compound 12 was obtained in the same way as in Example 1 except that the nucleic acid sequences were changed to HPRT1_csRNA23 and HPRT1_asRNA5 described in Table 14.

### Example 13

Compound 13 was obtained in the same way as in Example 1 except that the nucleic acid sequences were changed to HPRT1_csRNA24 and HPRT1_asRNA5 described in Table 14.

### Example 14 (Circular oligonucleotide B and circular siRNA thereof)

Compound 14 was synthesized in the same way as in Example 1 except that step 1 of Example 1 was performed using Spacer Phosphoramidite C3 (Glen Research Corp.) in addition to the amidite described in step 1, and the following method was performed instead of step 4 of Example 1.

To an aqueous solution (1.5 mL) of compound 14d corresponding to compound Id of scheme 1, a 5 mol/L aqueous NaCl solution, sodium bicarbonate, and water were added such that the final concentrations were 100 µmol/L compound 14d, 150 mmol/L NaCl, and 5 wt% sodium bicarbonate. Then, the mixture was left standing at 65°C for 48 hours. The reaction solution was concentrated under reduced pressure and purified by reverse-phase liquid chromatography (Waters Corp., Xbridge(Registered Trademark) C18, 4.6 mm × 250 mm, solution A: 0.1% triethylammonium acetate buffer solution, solution B: acetonitrile on a gradient) to obtain compound 14e (yield from 2 steps: 47%).

### Example 15 (Circular oligonucleotide C and circular siRNA thereof)

Compound 15 was synthesized in the same way as in Example 1 except that DMT-ethane-Diol phosphoramidite (ChemGenes Corp.) was used in addition to the amidite described in step 1 of Example 1, and the method described in Example 14 was performed instead of step 4 of Example 1.

### Example 16 (Circular oligonucleotide D and circular siRNA thereof)

Compound 16 was synthesized in the same way as in Example 1 except that Spacer Phosphoramidite 9 (Glen Research Corp.) was used in addition to the amidite described in step 1 of Example 1, and the method described in Example 14 was performed instead of step 4 of Example 1.

### Example 17 (Circular oligonucleotide E and circular siRNA thereof)

Compound 17 was synthesized in the same way as in Example 15 except that the nucleic acid sequences were changed to HPRT1_csRNA10 and HPRT1_asRNA5 described in Table 11.

### Example 18 (Circular oligonucleotide E and circular siRNA thereof)

Compound 18 was synthesized in the same way as in Example 15 except that the nucleic acid sequences were changed to HPRT1_csRNA17 and HPRT1_asRNA5 described in Table 12.

### Example 19 (Circular oligonucleotide F and circular siRNA thereof)

Compound 19 was synthesized in the same way as in Example 1 except that dSpacer CE Phosphoramidite (Glen Research Corp.) was used in addition to the amidite described in step 1 of Example 1, and the method described in Example 14 was performed instead of step 4 of Example 1.

### Example 20 (Circular oligonucleotide G and circular siRNA thereof)

Compound 20 was synthesized in the same way as in Example 1 except that PC Linker Phosphoramidite (Glen Research Corp.) was used in addition to the amidite described in step 1 of Example 1, and the method described in Example 14 was performed instead of step 4 of Example 1.

### Example 21 (Circular oligonucleotide H and circular siRNA thereof)

Compound 21 was synthesized in the same way as in Example 1 except that Amino-Modifier C6 dT (Glen Research Corp.) was used in addition to the amidite described in step 1 of Example 1, and the method described in Example 14 was performed instead of step 4 of Example 1.

### Example 22 (Circular oligonucleotide I and circular siRNA thereof)

Compound 22 was synthesized in the same way as in Example 15 except that the nucleic acid sequences were changed to HPRT1_csRNA14 and HPRT1_asRNA5 described in Table 11.

### Example 23 (Circular oligonucleotide I and circular siRNA thereof)

Compound 23 was synthesized in the same way as in Example 15 except that the nucleic acid sequences were changed to HPRT1_csRNA18 and HPRT1_asRNA5 described in Table 12.

### Example 24 (Circular oligonucleotide J and circular siRNA thereof)

Compound 24 was synthesized in the same way as in Example 1 except that 2'-OMe-U-Thiophosphoramidite (Glen Research Corp.) was used in addition to the amidite described in step 1 of Example 1, and the method described in Example 14 was performed instead of step 4 of Example 1.

### Example 25 (Circular oligonucleotide K and circular siRNA thereof)

Compound 25 was synthesized in the same way as in Example 24 except that the nucleic acid sequences were changed to HPRT1_csRNA20 and HPRT1_asRNA5 described in Table 13.

### Example 26 (Circular oligonucleotide L and circular siRNA thereof)

Compound 26 was synthesized in the same way as in Example 1 except that Spacer C12 CE Phosphoramidite (Glen Research Corp.) was used in addition to the amidite described in step 1 of Example 1, and the method described in Example 14 was performed instead of step 4 of Example 1.

### Example 27 (Circular oligonucleotide M and circular siRNA thereof)

Compound 27 was synthesized in the same way as in Example 1 except that amidite Rf3 synthesized in Reference Example 1 was used in addition to the amidite described in step 1 of Example 1, and the method described in Example 14 was performed instead of step 4 of Example 1.

### Example 28 (Circular oligonucleotide N and circular siRNA thereof)

Compound 28 was synthesized in the same way as in Example 1 except that PC Spacer Phosphoramidite (Glen Research Corp.) was used in addition to the amidite described in step 1 of Example 1, and the method described in Example 14 was performed instead of step 4 of Example 1.

### Example 29 (Circular oligonucleotide O and circular siRNA thereof)

PC Spacer Phosphoramidite (Glen Research Corp.) was used in addition to the amidite described in step 1 of Example 1, and the following method was performed instead of step 4 of Example 1.

To an aqueous solution (1.5 mL) of compound 29d corresponding to compound Id of scheme 1, a 5 mol/L aqueous NaCl solution, sodium bicarbonate, and water were added such that the final concentrations were 100 µmol/L compound 29d, 2 mol/L NaCl, and 5 wt% sodium bicarbonate. Then, the mixture was left standing at 65°C for 72 hours. The reaction solution was concentrated under reduced pressure and purified by reverse-phase liquid chromatography (Waters Corp., Xbridge(Registered Trademark) C18, 4.6 mm × 250 mm, solution A: 0.1% triethylammonium acetate buffer solution, solution B: acetonitrile on a gradient) and size-exclusion chromatography (Tosoh Corp., TSKgel G2000SWXL, 7.8 mm X 300 mm, 1 X PBS isocratic) to obtain compound 29e (yield from 2 steps: 20%).

Compound 29 was synthesized in the same way as in Example 1 except for the procedures described above.

### Example 30 (Circular oligonucleotide P and circular siRNA thereof)

Compound 30 was synthesized in the same way as in Example 15 except that the nucleic acid sequences were changed to HPRT1_csRNA34 and HPRT1_asRNA5 described in Table 16.

### Example 31 (Circular oligonucleotide Q and circular siRNA thereof)

Compound 31 was synthesized in the same way as in Example 1 except that Amino-Modifier Serinol Phosphoramidite (Glen Research Corp.) was used in addition to the amidite described in step 1 of Example 1, and the method described in Example 14 was performed instead of step 4 of Example 1.

### Example 32 (Circular oligonucleotide R and circular siRNA thereof)

Compound 32 was synthesized in the same way as in Example 1 except that Fmoc-Amino-DMT C-7 CE phosphoramidite (ChemGenes Corp.) was used in addition to the amidite described in step 1 of Example 1, and the method described in Example 14 was performed instead of step 4 of Example 1.

### Example 33 (Circular oligonucleotide S and circular siRNA thereof)

Compound 33 was synthesized in the same way as in Example 1 except that Alkyne-Modifier Serinol Phosphoramidite (Glen Research Corp.) was used in addition to the amidite described in step 1 of Example 1, and the method described in Example 14 was performed instead of step 4 of Example 1.

### Example 34 (Circular oligonucleotide T and circular siRNA thereof)

Compound 34 was synthesized in the same way as in Example 33 except that the nucleic acid sequences were changed to HPRT1_csRNA37 and HPRT1_asRNA5 described in Table 16.

### Example 35 (Circular oligonucleotide U and circular siRNA thereof)

Compound 35 was synthesized in the same way as in Example 1 except that the solid-phase reagent 3'-Thiol-Modifier C3 S-S CPG described in step 1 of Example 1 was changed to modified CPG Rf12 synthesized in Reference Example 2, and the method described in Example 14 was performed instead of step 4 of Example 1.

### Example 36 (Circular oligonucleotide V and circular siRNA thereof)

Compound 36 was synthesized in the same way as in Example 1 except that the solid-phase reagent 3'-Thiol-Modifier C3 S-S CPG described in step 1 of Example 1 was changed to modified CPG Rf12 synthesized in Reference Example 3, and the method described in Example 14 was performed instead of step 4 of Example 1.

### Example 37 (Circular oligonucleotide W and circular siRNA thereof)

Thiol-Modifier C6 S-S described in step 1 of Example 1 was changed to amidite Rf41 synthesized in Reference Example 6, the solid-phase reagent 3'-Thiol-Modifier C3 S-S CPG was changed to modified CPG Rf12 synthesized in Reference Example 2, and the following method was performed instead of step 4 of Example 1.

To an aqueous solution (1.5 mL) of compound 37d corresponding to compound Id of scheme 1, a 5 mol/L aqueous NaCl solution, triethylamine, and water were added such that the final concentrations were 100 µmol/L compound 37d, 2 mol/L NaCl, and 5 vol% triethylamine. Then, the mixture was left standing at 65°C for 72 hours. The reaction solution was purified by reverse-phase liquid chromatography (Waters Corp., Xbridge(Registered Trademark) C18, 4.6 mm × 250 mm, solution A: 0.1% triethylammonium acetate buffer solution, solution B: acetonitrile on a gradient) and size-exclusion chromatography (Tosoh Corp., TSKgel G2000SWXL, 7.8 mm X 300 mm, 1 X PBS isocratic) to obtain compound 37e (yield from 2 steps: 16%).

Compound 37 was synthesized in the same way as in Example 1 except for the procedures described above.

### Example 38 (Circular oligonucleotide X and circular siRNA thereof)

Compound 38 was synthesized in the same way as in Example 1 except that Thiol-Modifier C6 S-S described in step 1 of Example 1 was changed to amidite Rf46 synthesized in Reference Example 7, and the method described in Example 14 was performed instead of step 4 of Example 1.

### Example 39 (Circular oligonucleotide Y and circular siRNA thereof)

Compound 39 was synthesized in the same way as in Example 1 except that the solid-phase reagent 3'-Thiol-Modifier C3 S-S CPG described in step 1 of Example 1 was changed to modified CPG Rf29 synthesized in Reference Example 4, and the method described in Example 14 was performed instead of step 4 of Example 1.

### Example 40 (Circular oligonucleotide Z and circular siRNA thereof)

Compound 40 can be synthesized in the same way as in Example 1 except that the solid-phase reagent 3'-Thiol-Modifier C3 S-S CPG described in step 1 of Example 1 is changed to modified CPG Rf37 synthesizable in Reference Example 5, and the method described in Example 14 is performed instead of step 4 of Example 1.

### Example 41 (Circular oligonucleotide AA and circular siRNA thereof)

Compound 41 was synthesized in the same way as in Example 1 except that the solid-phase reagent 3'-Thiol-Modifier C3 S-S CPG described in step 1 of Example 1 was changed to DMT-C6 Disulfide lcaa CPG 500 Angstrom (ChemGenes Corp.), and the method described in Example 14 was performed instead of step 4 of Example 1.

### Example 42 (Circular oligonucleotide AB and circular siRNA thereof)

Compound 42 was synthesized in the same way as in Example 1 except that Thiol-Modifier C6 S-S described in step 1 of Example 1 was changed to 5'-Thio-dI CEP (Berry & Associates, Inc.), and the method described in Example 14 was performed instead of step 4 of Example 1.

### Example 43 (Circular oligonucleotide AC and circular siRNA thereof)

Compound 43 was synthesized in the same way as in Example 1 except that Thiol-Modifier C6 S-S described in step 1 of Example 1 was changed to Thiol-modifier-oxa-C6-S-S CEP (Berry & Associates, Inc.), and the method described in Example 14 was performed instead of step 4 of Example 1.

### Example 44 (Circular oligonucleotide AD and circular siRNA thereof)

Compound 44 was synthesized according to the following scheme 2: 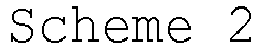 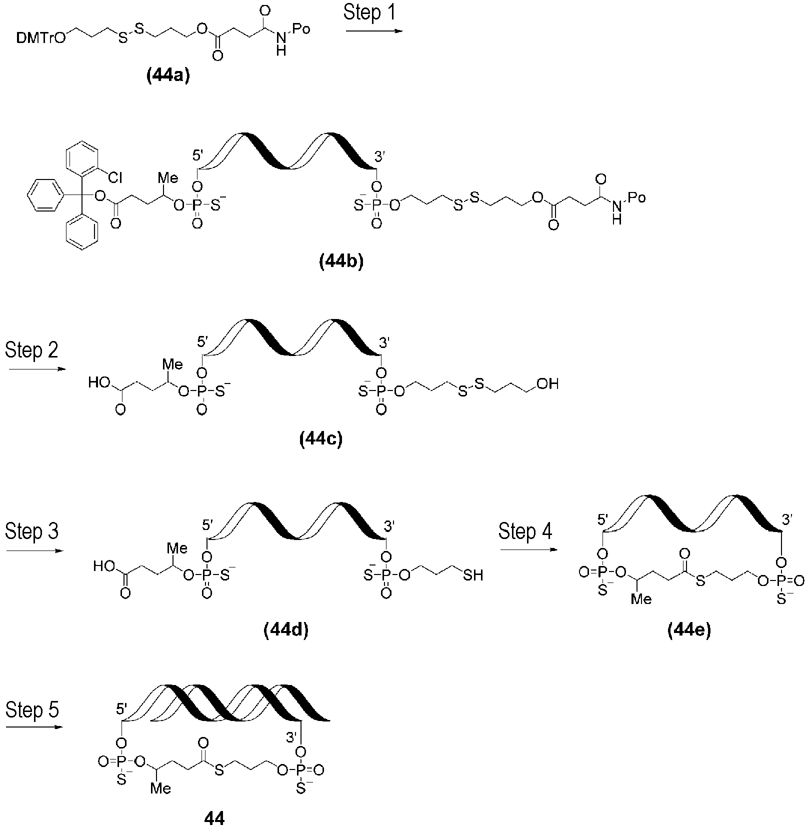 wherein Po is as defined above.

### Step 1

Crude product of compound 44b was obtained in the same way as in step 1 of Example 1 except that Thiol-Modifier C6 S-S described in step 1 of Example 1 was changed to 5'-Carboxy-Modifier C5 (Glen Research Corp.).

### Step 2

Compound 44c was obtained in the same way as in step 2 of Example 1.

ESI-MS calcd: 9208, found: 9207

### Step 3

Compound 44c (100 nmol) obtained in step 2 was dissolved in 200 uL of RNase-free water. To the solution, 200 uL of separately prepared 30 mmol/L tris(2-carboxyethyl)phosphine hydrochloride (TCEP·HCl)/10 mmol/L triethylamine acetate buffer solution was added, and the mixture was left standing at room temperature for 12 hours. After reaction, a crude product (400 uL) of compound 44d was obtained and used directly in the next step.

ESI-MS calcd: 9117, found: 9118

### Step 4

To the reaction solution (400 uL) of compound 44d obtained in step 3, 5 mol/L NaCl and water were added such that the final concentrations were 100 µmol/L compound 44d and 450 mmol/L NaCl. Then, the mixture was left standing at 60°C for 48 hours. The reaction solution was purified by reverse-phase liquid chromatography (Waters Corp., XBridge(Registered Trademark) C18, 4.6 mm × 250 mm, solution A: 0.1% triethylammonium acetate buffer solution, solution B: acetonitrile on a gradient) to obtain compound 44e (15 nmol, yield from 2 steps: 15%).

ESI-MS calcd: 9100, found: 9102

### Step 5

Compound 44 was obtained in the same way as in step 5 of Example 1 except that the nucleic acid sequence was changed to HPRT1_asRNA5 described in Table 8.

### Example 45 (Circular oligonucleotide AE and circular siRNA thereof)

Compound 45 was synthesized in the same way as in Example 1 except that the solid-phase reagent 3'-Thiol-Modifier C3 S-S CPG described in step 1 of Example 1 was changed to modified CPG Rf59 synthesized in Reference Example 9.

### Example 46 (Circular oligonucleotide AF and circular siRNA thereof)

Compound 46 can be synthesized according to the following scheme 3: 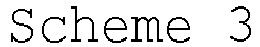 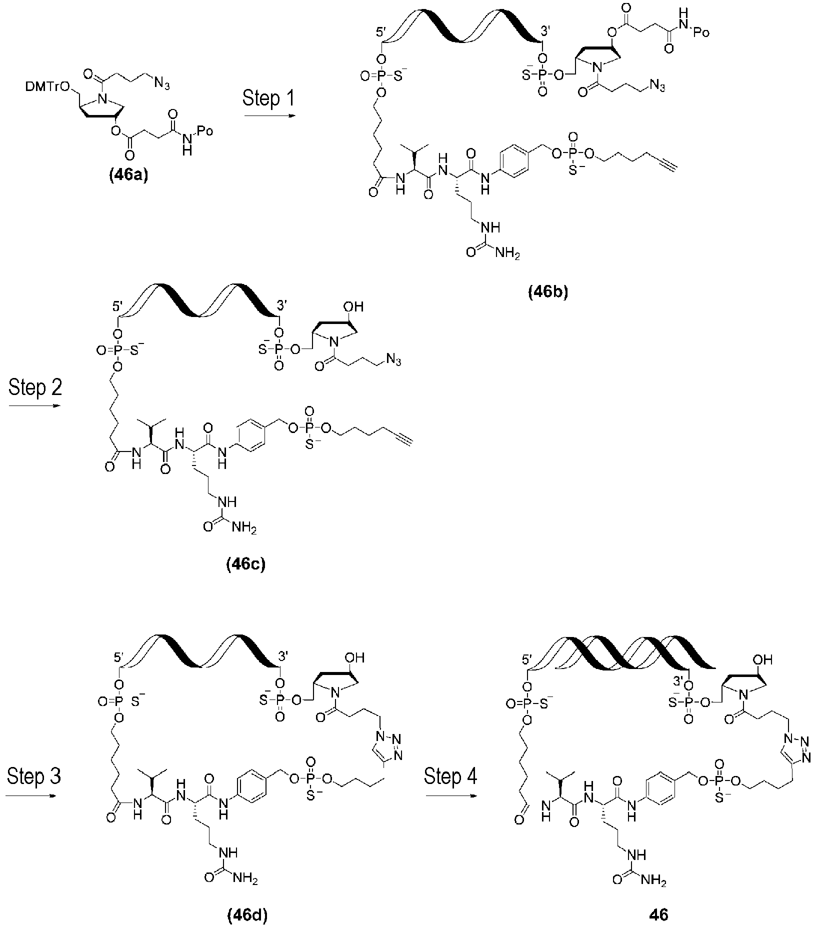 wherein Po is as defined above.

### Step 1

A crude product of compound 46b is obtained in the same way as in step 1 of Example 1 except that Thiol-Modifier C6 S-S described in step 1 of Example 1 is changed to 5'-Hexynyl Phosphoramidite (Glen Research Corp.), the solid-phase reagent 3'-Thiol-Modifier C3 S-S CPG is changed to Azide-modified CPG (compound (46a), PRIMETECH Corp.), and dipeptide amidite Rf65 synthesized in Reference Example 10 is used.

### Step 2

Crude product 46b obtained in step 1 is treated with a reagent containing a 28% aqueous ammonia solution and a 40% aqueous methylamine solution mixed in equal amounts and thereby excised from the solid-phase reagent. The obtained crude product is purified by reverse-phase liquid chromatography (Waters Corp., XBridge(Registered Trademark) C18, 4.6 mm x 250 mm, solution A: 0.1% triethylammonium acetate buffer solution, solution B: acetonitrile on a gradient) to obtain compound 46c.

### Step 3

Compound 46d is obtained by the method described in Journal of Organic Chemistry, Vol. 73, p. 287-290, 2008 or a synthesis method equivalent thereto using compound 46c obtained in step 2.

### Step 4

Compound 46 is obtained in the same way as in step 5 of Example 1 except that the nucleic acid sequence is changed to HPRT1_asRNA5 described in Table 8.

### Example 47 (Circular oligonucleotide AG and circular siRNA thereof)

Compound 47 can be synthesized according to the following scheme 4: 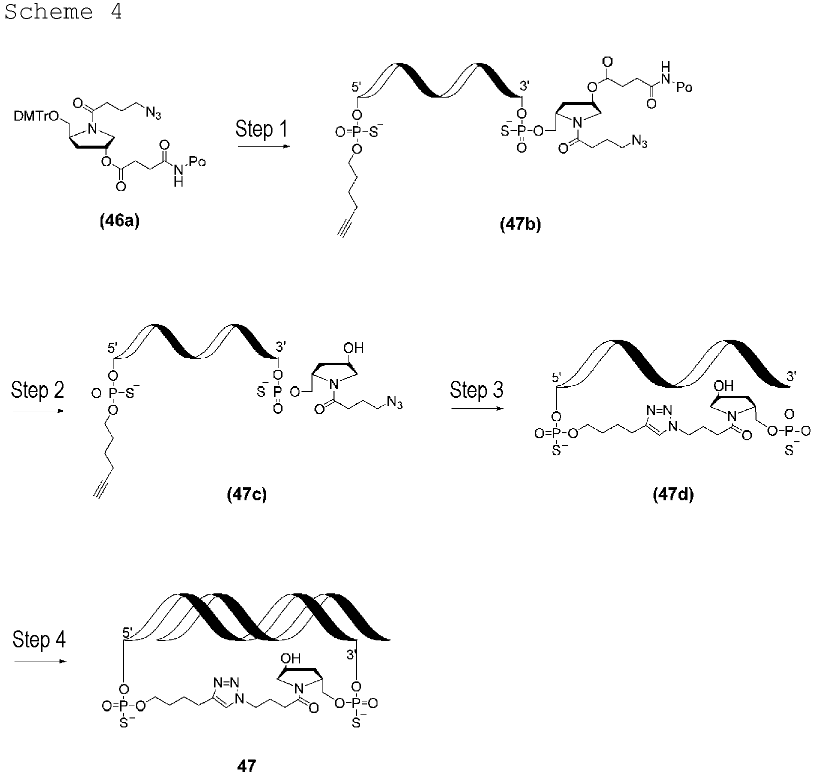 wherein Po is as defined above.

### Step 1

A crude product of compound 47b was obtained in the same way as in step 1 of Example 1 except that Thiol-Modifier C6 S-S described in step 1 of Example 1 was changed to 5'-Hexynyl Phosphoramidite (Glen Research Corp.), and the solid-phase reagent 3'-Thiol-Modifier C3 S-S CPG was changed to Azide-modified CPG (compound 46a, PRIMETECH Corp.).

### Step 2

Crude product 47b obtained in step 1 was treated with a reagent containing a 28% aqueous ammonia solution and a 40% aqueous methylamine solution mixed in equal amounts and thereby excised from the solid-phase reagent. The obtained crude product was purified by reverse-phase liquid chromatography (Waters Corp., Xbridge(Registered Trademark) C18, 4.6 mm x 250 mm, solution A: 0.1% triethylammonium acetate buffer solution, solution B: acetonitrile on a gradient) to obtain compound 47c.

### Step 3

Compound 47c (70 nmol) obtained in step 2 was divided into seven 1.5 mL sample tubes (10 nmol of compound 47c per tube). To each tube, 5 mol/L NaCl and water were added such that the final concentrations were 50 µmol/L compound 47c and 200 mmol/L NaCl. Then, Cu wires (10 to 20 wires, manufactured by Wako Pure Chemical Industries, Ltd.) were added to each tube, which was then placed in a heat block prewarmed to 80°C, left standing for 3 minutes, and left standing until the temperature became room temperature by the termination of heating. The reaction solutions in these tubes were combined, simply purified using NAP-10 column (manufactured by GE Healthcare Japan Corp.), and then purified by reverse-phase liquid chromatography (Waters Corp., Xbridge (Registered Trademark) C18, 4.6 mm x 250 mm, solution A: 0.1% triethylammonium acetate buffer solution, solution B: acetonitrile on a gradient) to obtain compound 47d (27.4 nmol, yield: 39%).

### ESI-MS calcd: 9235, found: 9234

### Step 4

Compound 47 was obtained in the same way as in step 5 of Example 1 except that the nucleic acid sequence was changed to HPRT1_asRNA5 described in Table 8.

The base sequences of the oligonucleotide derivatives of Examples 7 to 46 and an oligonucleotide as a negative control group, and the molecular weights thereof are shown in Tables 10 to 19. For each Example, the upper column shows the circular oligonucleotide, and the lower column shows the linear nucleotide. Each alphabet in the column "Circular structure" in the tables means that the sequence takes a circular structure corresponding to each alphabet in the column "Circular structure" in Tables 2 to 6. For each negative control group, the upper column shows the sense strand, and the lower column shows the antisense strand.

The abbreviations, etc. in Tables 10 to 19 are as defined below.

V at both ends means that these V moieties were bonded to each other to form a circular structure corresponding to the alphabet shown in the column "Circular structure" in the tables.
p = Phosphorylated
^ = Phosphorothioate-modified
m = 2'-OMe-modified
f = 2'-F-modified

**[Table 10]**

| | Name | Circular structure | Sequence (5' -> 3') | MS (calcd) | MS (found) |
|---|---|---|---|---|---|
| Negative control group 7 | PTEN_dsRNA1 PTEN_ssRNA1/PTEN_asRNA1 | - | fA^mC^fCmUfGmAfUmCfAmUfUmAfUmAfGmAfU^mA^fA | 6224 | 6226 |
| | | - | pmU^fU^mAfUmCfUmAfUmAfAmUfGmAfUmCfAmGfGmU^fA^mA | 6984 | 6987 |
| Example 7 | Compound 7 PTEN_csRNA1/PTEN_asRNA2 | A | V^fG^mA^fCmAfAmUfGmAfAmCfCmUfGmAfUmCfAmUfUmAfUmAfGmAfU^mA^fA^V | 9290 | 9289 |
| | | - | pmU^fU^mAfUmCfUmAfUmAfAmUfGmAfUmCfAmGfGmU^fU^mC | 6939 | 6940 |
| Negative control group 8 | Factor9_dsRNA1 Factor9_ssRNA1/Factor9_asRNA1 | - | fU^mG^fGmAfAmGfCmAfGfUfAmUfGmUfUmGfAmUfGmGfA | 6998 | 7000 |
| | | - | pmU^fC^mCfAmUfCmAfAmCfAmUmAmCfUmGfCmUfUmCfCmA^mA^mA | 7552 | 7556 |
| Example 8 | Compound 8 Factor9_csRNA1/Factor9_ asRNA2 | A | V^fG^mA^fAmUfUmUfUmGfGmAfAmGfCmAfGfUfAmUfGmUfUmGfAmUfGmGfA^V | 9352 | 9347 |
| | | - | pmU^fC^mCfAmUfCmAfAmCfAmUmAmCfUmGfCmUfUmC^fC^mA | 6868 | 6867 |

**[Table 11]**

| | Name | Circular structure | Sequence (5' -> 3') | **MS** (calcd) | MS (found) |
|---|---|---|---|---|---|
| Example 9 | Compound 9 HPRT1_csRNA6/HPRT1_asRNA5 | A | V^fU^mU^fUmUfAmUfUmCfCmUfAmUfGmAfCmUfGmUfAmGfAmUfUmUfU^mA^fU^V | 9111 | 9112 |
| | | - | pmA'fU'mAfAmAfAmUfCmUfAmCfAmGfUmCfAmUfAmG'fG'mA | 7029 | 7030 |
| Example 14 | Compound 14 HPRT1_csRNA7/HPRT1_asRNA5 | B | V^fA^mU^fUmCfCmUfAmUfGmAfCmUfGmUfAmGfAmUfUmUfU^mA^fU^V | 9088 | 9088 |
| | | - | pmA^fU^mAfAmAfAmUfCmUfAmCfAmGfUmCfAmUfAmG^fG^mA | 7029 | 7030 |
| Example 15 | Compound 15 HPRT1_csRNA8/HPRT1_asRNA5 | c | V^mU^fUmCfCmUfAmUfGmAfCmUfGmUfAmGfAmUfUmUfU^mA^fU^V | 8976 | 8975 |
| | | - | pmA^fU^mAfAmAfAmUfCmUfAmCfAmGfUmCfAmUfAmG^fG^mA | 7029 | 7030 |
| Example 16 | Compound 16 HPRT1_csRNA9/HPRT1_asRNA5 | D | V^fA^mU^fUmCfCmUfAmUfGmAfCmUfGmUfAmGfAmUfUmUfU^mA^fU^V | 9224 | 9224 |
| | | - | pmA^fU^mAfAmAfAmUfCmUfAmCfAmGfUmCfAmUfAmG^fG^mA | 7029 | 7030 |
| Example 17 | Compound 17 HPRT1_csRNA10/HPRT1_asRNA5 | E | V^fA^mU^fCmCfAmUfUmCfCmUfAmUfGmAfCmUfGmUfAmGfAmUfUmUfU^mA^fU^V | 9693 | 9692 |
| | | - | pmA^fU^mAfAmAfAmUfCmUfAmCfAmGfUmCfAmUfAmG^fG^mA | 7029 | 7030 |
| Example 19 | Compound 19 HPRT1_csRNA11/HPRT1_ asRNA5 | F | V^mU^fCmCfAmUfUmCfCmUfAmUfGmAfCmUfGmUfAmGfAmUfUmUfU^mA^fU^V | 9918 | 9917 |
| | | - | pmA^fU^mAfAmAfAmUfCmUfAmCfAmGfUmCfAmUfAmG^fG^mA | 7029 | 7030 |
| Example 20 | Compound 20 HPRT1_csRNA12/HPRT1_asRNA5 | G | V^mU^fCmCfAmUfUmCfCmUfAmUfGmAfCmUfGmUfAmGfAmUfUmUfU^mA^fU^V | 10231 | 10231 |
| | | - | pmA^fU^mAfAmAfAmUfCmUfAmCfAmGfUmCfAmUfAmG^fG^mA | 7029 | 7030 |
| Example 21 | Compound 21 HPRT1_csRNA13/HPRT1_asRNA5 | H | V^fA^mU^fCmCfAmUfUmCfCmUfAmUfGmAfCmUfGmUfAmGfAmUfUmUfU^mA^fU^V | 11031 | 11028 |
| | | - | pmA^fU^mAfAmAfAmUfCmUfAmCfAmGfUmCfAmUfAmG^fG^mA | 7029 | 7030 |
| Example 22 | Compound 22 HPRT1_csRNA14/HPRT1_asRNA5 | I | V^fA^mU^fCmCfAmUfUmCfCmUfAmUfGmAfCmUfGmUfAmGfAmUfUmUfU^mA^fU^V | 10254 | 10253 |
| | | - | pmA^fU^mAfAmAfAmUfCmUfAmCfAmGfUmCfAmUfAmG^fG^mA | 7029 | 7030 |

**[Table 12]**

| | Name | Circular structure | Sequence (5' -> 3') | MS (calcd) | MS (found) |
|---|---|---|---|---|---|
| Negative control group 9 | HPRT1_dsRNA6 | - | fU^mC^fC^mU^fA^mU^fG^mAfCmUfGmUfA^mG^fA^mU^fU^mU^fU^mA^fU | 7000 | 6999 |
| | HPRT1_ssRNA4/HPRT1_asRNA5 | - | pmA^fU^mAfAmAfAmUfCmUfAmCfAmGfUmCfAmUfAmG^fG^mA | 7029 | 7030 |
| Example 10 | Compound 10 | A | V^fA^mU^fC^mC^fA^mU^fU^mCfCmUfAmUfGmAfCmUfGmUfA^mG^fU^mU^fU^mU^fU^mA^fU^V | 9310 | 9311 |
| | HPRT1_csRNA15/HPRT1_asRNA5 | - | pmA^fU^mAfAmAfAmUfCmUfAmCfAmGfUmCfAmUfAmG^fG^mA | 7029 | 7030 |
| Example 11 | Compound 11 | A | V^fU^mU^fU^mU^fA^mU^fU^mC^fCmUfAmUfGmAfCmUfGmUfAmG^fA^mU^fU^mU^fU^mA^fU^V | 9287 | 9287 |
| | HPRT1_csRNA 16/HPRT1_asRNA5 | - | pmA^fU^mAfAmAfAmUfCmUfAmCfAmGfUmCfAmUfAmG^fG^mA | 7029 | 7030 |
| Example 18 | Compound 18 | E | V^fA^mU^fC^mC^fA^mU^fU^mC^fCmUfAmUfGmAfCmUfGmUfAmG^fA^mU^fU^mU^fU^mA^fU^V | 9869 | 9868 |
| | HPRT1_csRNA17/HPRT1_asRNA5 | - | pmA^fU^mAfAmAfAmUfCmUfAmCfAmGfUmCfAmUfAmG^fG^mA | 7029 | 7030 |
| Example 23 | Compound 23 | I | V^fA^mU^fC^mC^fA^mU^fU^mC^fCmUfAmUfGmAfCmUfGmUfAmG^fA^mU^fU^mU^fU^mA^fU^V | 10431 | 10431 |
| | HPRT1csRNA18/HPRT1_asRNA5 | - | pmA^fU^mAfAmAfAmUfCmUfAmCfAmGfUmCfAmUfAmG^fG^mA | 7029 | 7030 |

**[Table 13]**

| | Name | Circular structure | Sequence (5' -> 3') | MS (calcd) | MS (found) |
|---|---|---|---|---|---|
| Example 24 | Compound 24 | J | V^fUmUfAmUfUmCfCmUfAmUfGmAfCmUfGmUfAmGfAmUfUmUfU^mA^fU^V | 9153 | 9154 |
| | HPRT1_csRNA 19/HPRT1_asRNA5 | - | pmA^fU^mAfAmAfAmUfCmUfAmCfAmGfUmCfAmUfAmG^fG^mA | 7029 | 7030 |
| Example 25 | Compound 25 | K | V^fA^mU^fU^mC^fCmUfAmUfGmAfCmUfGmUfAmG^fA^mU^fU^mU^fU^mA^fU^V | 9373 | 9373 |
| | HPRT1_csRNA20/HPRT1_asRNA5 | - | pmA^fU^mAfAmAfAmUfCmUfAmCfAmGfUmCfAmUfAmG^fG^mA | 7029 | 7030 |
| Example 26 | Compound 26 | L | V^fA^mU^fCmCfAmUfUmCfCmUfAmUfGmAfCmUfGmUfAmGfAmUfUmUfU^mA^fU^V | 9413 | 9411 |
| | HPRT1_csRNA21/HPRT1_asRNA5 | - | pmA^fU^mAfAmAfAmUfCmUfAmCfAmGfUmCfAmUfAmG^fG^mA | 7029 | 7030 |
| Example 35 | Compound 35 | U | V^fA^mU^fCmCfAmUfUmCfCmUfAmUfGmAfCmUfGmUfAmGfAmUfUmUfU^mA^fU^V | 9147 | 9146 |
| | HPRT1_csRNA22/HPRT1_asRNA5 | - | pmA^fU^mAfAmAfAmUfCmUfAmCfAmGfUmCfAmUfAmG^fG^mA | 7029 | 7030 |

**[Table 14]**

| | Name | Circular structure | Sequence (5' -> 3') | MS (calcd) | MS (found) |
|---|---|---|---|---|---|
| Example 12 | Compound 12 | A | V^mA^fG^mUfGfAmUfAmGfAmUfCmCfAmUfUmCfCmUfAmUfGmAfCmUfGmUfAmGfAmUfUm UfU^mA^fU^V | 11833 | 11831 |
| | HP RT1 _csRN A23/HP RT1 _asRNA5 | - | pmA^fU^mAfAmAfAmUfCmUfAmCfAmGfUmCfAmUfAmG^fG^mA | 7029 | 7030 |
| Example 13 | Compound 13 | A | V^fG^mA^fCmUfGmUfAmGfAmUfUmUfU^mA^fU^V | 5298 | 5296 |
| | HPRT1_csRNA24/HPRT1_asRNA5 | - | pmA^fU^mAfAmAfAmUfCmUfAmCfAmGfUmCfAmUfAmG^fG^mA | 7029 | 7030 |
| Example 32 | Compound 32 | R | V^fA^mU^fCmCfAmUfUmCfCmUfAmUfGmAfCmUfGmUfAmGfAmUfUmUfU^mA^fU^V | 9360 | 9357 |
| | HPRT1_csRNA25/HPRT1_asRNA5 | - | pmA^fU^mAfAmAfAmUfCmUfAmCfAmGfUmCfAmUfAmG^fG^mA | 7029 | 7030 |
| Example 41 | Compound 41 | AA | V^fA^mU^fCmCfAmUfUmCfCmUfAmUfGmAfCmUfGmUfAmGfAmUfUmUfU^mA^fU^V | 9175 | 9171 |
| | HPRT1_csRNA26/HPRT1_asRNA5 | - | pmA^fU^mAfAmAfAmUfCmUfAmCfAmGfUmCfAmUfAmG^fG^mA | 7029 | 7030 |
| Example 44 | Compound 44 | AD | V^fA^mU^fCmCfAmUfUmCfCmUfAmUfGmAfCmUfGmUfAmGfAmUfUmUfU^mA^fU^V | 9100 | 9102 |
| | HPRT1_csRNA27/HPRT1_asRNA5 | - | pmA^fU^mAfAmAfAmUfCmUfAmCfAmGfUmCfAmUfAmG^fG^mA | 7029 | 7030 |

**[Table 15]**

| | Name | Circular structure | Sequence (5' -> 3') | MS (calcd) | MS (found) |
|---|---|---|---|---|---|
| Example 38 | Compound 38 | X | V^fA^mU^fCmCfAmUfUmCfCmUfAmUfGmAfCmUfGmUfAmGfAmUfUmUfU^mA^fU^V | 9117 | 9116 |
| | HPRT1_csRNA28/HPRT1_asRNA5 | - | pmA^fU^mAfAmAfAmUfCmUfAmCfAmGfUmCfAmUfAmG^fG^mA | 7029 | 7030 |
| Example 39 | Compound 39 | Y | V^fA^mU^fCmCfAmUfUmCfCmUfAmUfGmAfCmUfGmUfAmGfAmUfUmUfU^mA^fU^V | 9172 | 9170 |
| | HPRT1_csRNA29/HPRT1_asRNA5 | - | pmA^fU^mAfAmAfAmUfCmUfAmCfAmGfUmCfAmUfAmG^fG^mA | 7029 | 7030 |
| Example 42 | Compound 42 | AB | V^fA^mU^fCmCfAmUfUmCfCmUfAmUfGmAfCmUfGmUfAmGfAmUfUmUfU^mA^fU^V | 9265 | 9263 |
| | HPRT1_csRNA30/HPRT1_asRNA5 | - | pmA^fU^mAfAmAfAmUfCmUfAmCfAmGfUmCfAmUfAmG^fG^mA | 7029 | 7030 |
| Example 43 | Compound 43 | AC | V^fA^mUfCmCfAmUfUmCfCmUfAmUfGmAfCmUfGmUfAmGfAmUfUmUfUmA^fU^V | 9103 | 9101 |
| | HPRT1_csRNA31/HPRT1_asRNA5 | - | pmA^fU^mAfAmAfAmUfCmUfAmCfAmGfUmCfAmUfAmG^fG^mA | 7029 | 7030 |

**[Table 16]**

| | Name | Circular structure | Sequence (5' -> 3') | MS (calcd) | MS (found) |
|---|---|---|---|---|---|
| Example 28 | Compound 28 | N | V^fA^mU^fCmCfAmUfUmCfCmUfAmUfGmAfCmUfGmUfAmGfAmUfUmUfU^mA^fU^V | 10571 | 10570 |
| | HPRT1_csRNA32/HPRT1_asRNA5 | - | pmA^fU^mAfAmAfAmUfCmUfAmCfAmGfUmCfAmUfAmG^fG^mA | 7029 | 7030 |
| Example 29 | Compound 29 | O | V^fA^mU^fUmCfCmUfAmUfGmAfCmUfGmUfAmGfAmUfUmUfU^mA^fU^V | 10735 | 10736 |
| | HPRT1_csRNA33/HPRT1_asRNA5 | - | pmA^fU^mAfAmAfAmUfCmUfAmCfAmGfUmCfAmUfAmG^fG^mA | 7029 | 7030 |
| Example 30 | Compound 30 | P | V^fA^mU^fUmCfCmUfAmUfGmAfCmUfGmUfAmGfAmUfUmUfU^mA^fU^V | 10097 | 10095 |
| | HPRT1_csRNA34/HPRT1_asRNA5 | - | pmA^fU^mAfAmAfAmUfCmUfAmCfAmGfUmCfAmUfAmG^fG^mA | 7029 | 7030 |
| Example 31 | Compound 31 | Q | V^fA^mU^fCmCfAmUfUmCfCmUfAmUfGmAfCmUfGmUfAmGfAmUfUmUfU^mA^fU^V | 10091 | 10088 |
| | HPRT1_csRNA35/HPRT1_asRNA5 | - | pmA^fU^mAfAmAfAmUfCmUfAmCfAmGfUmCfAmUfAmG^fG^mA | 7029 | 7030 |
| Example 33 | Compound 33 | s | V^fA^mU^fCmCfAmUfUmCfCmUfAmUfGmAfCmUfGmUfAmGfAmUfUmUfU^mA^fU^V | 10467 | 10468 |
| | HPRT1_csRNA36/HPRT1_asRNA5 | - | pmA^fU^mAfAmAfAmUfCmUfAmCfAmGfUmCfAmUfAmG^fG^mA | 7029 | 7030 |
| Example 34 | Compound 34 | T | V^fA^mU^fUmCfCmUfAmUfGmAfCmUfGmUfAmGfAmUfUmUfU^mA^fU^V | 10527 | 10527 |
| | HPRT1_csRNA37/HPRT1_asRNA5 | - | pmA^fU^mAfAmAfAmUfCmUfAmCfAmGfUmCfAmUfAmG^fG^mA | 7029 | 7030 |

**[Table 17]**

| | Name | Circular structure | Sequence (5' -> 3') | MS (calcd) | MS (found) |
|---|---|---|---|---|---|
| Example 27 | Compound 27 | M | V^fA^mU^fCmCfAmUfUmCfCmUfAmUfGmAfCmUfGmUfAmGfAmUfUmUfU^mA^fU^V | 9470 | 9469 |
| | HPRT1_csRNA38/HPRT1_asRNA5 | - | pmA^fU^mAfAmAfAmUfCmUfAmCfAmGfUmCfAmUfAmG^fG^mA | 7029 | 7030 |
| Example 36 | Compound 36 | V | V^fA^mU^fCmCfAmUfUmCfCmUfAmUfGmAfCmUfGmUfAmGfAmUfUmUfU^mA^fU^V | 9160 | 9158 |
| | HPRT1_csRNA39/HPRT1_asRNA5 | - | pmA^fU^mAfAmAfAmUfCmUfAmCfAmGfUmCfAmUfAmG^fG^mA | 7029 | 7030 |
| Example 37 | Compound 37 | W | V^fA^mU^fCmCfAmUfUmCfCmUfAmUfGmAfCmUfGmUfAmGfAmUfUmUfU^mA^fU^V | 9118 | 9118 |
| | HPRT1_csRNA40/HPRT1_asRNA5 | - | pmA^fU^mAfAmAfAmUfCmUfAmCfAmGfUmCfAmUfAmG^fG^mA | 7029 | 7030 |
| Example 45 | Compound 45 | AE | V^fG^mG^fAmUfAmUfAmAfGmCfCmAfGmAfCmUfUmUfGmUfUmGfGmAfUmUfU^V | 10044 | 10043 |
| | HPRT1_csRNA41/HPRT1_asRNA1 | - | mA^fA^mAfUmCfCmAfAmCfAmAfAmGfUmCfUmGfGmC^fU^mU | 6901 | 6903 |

**[Table 18]**

| | Name | Circular structure | Sequence (5' -> 3') | MS (calcd) | MS (found) |
|---|---|---|---|---|---|
| Negative control group 6 | HPRTI_dsRNA5 | - | fU^mC^fCmUfAmUfGmAfCmUfGmUfAmGfAmUfUmUfU^mA^fU | 6823 | 6823 |
| | HPRT1_ssRNA3/HPRT1_asRNA5 | - | pmA^fU^mAfAmAfAmUfCmUfAmCfAmGfUmCfAmUfAmG^fG^mA | 7029 | 7030 |
| Example 47 | Compound 47 | AG | V^fA^mU^fCmCfAmUfUmCfCmUfAmUfGmAfCmUfGmUfAmGfAmUfUmUfU^mA^fU^V | 9235 | 9234 |
| | HPRT1_csRNA42/HPRT1 asRNA5 | - | pmA^fU^mAfAmAfAmUfCmUfAmCfAmGfUmCfAmUfAmG^fG^mA | 7029 | 7030 |

**[Table 19]**

| | Name | Circular structure | Sequence (5' -> 3') | MS (calcd) | MS (found) |
|---|---|---|---|---|---|
| Example 40 | Compound 40 | Z | V^fA^mU^fCmCfAmUfUmCfCmUfAmUfGmAfCmUfGmUfAmGfAmUfUmUfU^mA^fU^V | | |
| | HPRT1_csRNA43/HPRT1_asRNA5 | - | pmA^fU^mAfAmAfAmUfCmUfAmCfAmGfUmCfAmUfAmG^fG^mA | 7029 | 7030 |
| Example 46 | Compound 46 | AF | V^fA^mU^fCmCfAmUfUmCfCmUfAmUfGmAfCmUfGmUfAmGfAmUfUmUfU^mA^fU^V | | |
| | HPRT1_osRNA44/HPRT1_asRNA5 | - | pmA^fU^mAfAmAfAmUfCmUfAmCfAmGfUmCfAmUfAmG^fG^mA | 7029 | 7030 |

### Reference Example 1

### Synthesis of amidite Rf3

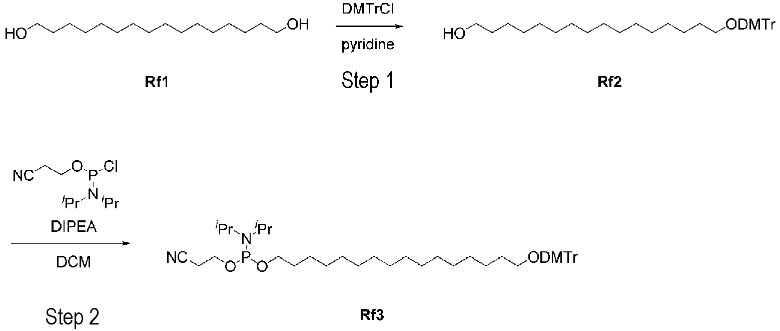

### Step 1:

Commercially available hexadecane-1,16-diol Rf1 (5.24 g, 20.3 mmol) was suspended in dehydrated pyridine (82 mL). To the suspension, 4,4'-dimethoxytrityl chloride (5.72 g, 16.9 mmol) was added, and the mixture was stirred at room temperature for 2 hours and 30 minutes. After the completion of reaction, the reaction solution was quenched by the addition of dichloromethane and a saturated aqueous solution of sodium bicarbonate. The organic layer was washed once with water and saturated saline and dried over anhydrous sodium sulfate. The solvent was distilled off under reduced pressure to obtain a crude product (15.7 g) as a yellowish-white solid. The obtained crude product was purified four times by column chromatography (Yamazen Corp., Hi-Flash Column 3L, developing solvent: n-heptane/ethyl acetate (2% triethylamine) = 90/10 → 70/30 → 50/50). The solvent was distilled off under reduced pressure. Then, the residue was subjected to azeotropy with acetonitrile to obtain 16-(bis(4-methoxyphenyl)(phenyl)methoxy)hexadecan-1-ol Rf2 (4.25 g, 7.13 mmol, 5.9 wt% acetonitrile) as a yellow oil (yield: 42%) .

ESI-MS (m/z): 584 [M + Na].

¹H-NMR (CDCl₃, 400 MHz) δ (ppm): 7.43 (d, J = 7.2 Hz, 2H), 7.33 - 7.16 (m, 7H), 6.84-6.81 (m, 4H), 3.78 (s, 6H), 3.64 (q, J = 6.4 Hz, 2H), 3.02 (t, J = 6.4 Hz, 2H), 1.64-1.53 (m, 4H), 1.42-1.20 (m, 24H).

### Step 2:

In 20 mL eggplant-shaped flask in an argon atmosphere, compound Rf2 (3.00 g, 5.35 mmol) was dissolved in dehydrated dichloromethane (41 mL). To the solution, N,N-diisopropylethylamine (4.67 mL, 26.7 mmol) and 2-cyanoethyl diisopropylchlorophosphoramidite (1.90 g, 8.02 mmol) were added in an ice bath, and the mixture was stirred at room temperature for 1 hour. After the completion of reaction, the reaction solution was quenched by the addition of a saturated aqueous solution of sodium bicarbonate, followed by extraction with dichloromethane twice. Combined organic layers were washed with water and then dried over anhydrous sodium sulfate. The solvent was distilled off under reduced pressure to obtain a crude product (6.45 g) as a white oil. The crude product was purified by column chromatography (Yamazen Corp., NH Column L, developing solvent: n-heptane/ethyl acetate = 90/10 -> 70/30 → 50/50) to obtain 16-(bis(4-methoxyphenyl)(phenyl)methoxy)hexadecyl(2-cyanoethyl)diisopropylphosphoramidite Rf3 (3.78 g, 4.09 mmol) as a colorless oil (yield: 77%).

ESI-MS (m/z): undetected.

¹H-NMR (CDCl₃, 400 MHz) δ (ppm): 7.45-7.43 (m, 2H), 7.43-7.25 (m, 6H), 7.21-7.19 (m, 1H), 6.84-6.81 (m, 4H), 3.90-3.80 (m, 2H), 3.79 (s, 6H), 3.68-3.55 (m, 4H), 3.02 (t, J = 6.8 Hz, 2H), 2.64 (t, J =6.4 Hz, 2H), 1.64-1.56 (m, 4H), 1.35-1.24 (m, 24H), 1.18 (dd, J = 4.0, 6.8 Hz, 12H). ³¹P-NMR (CDCl₃, 162 MHz) δ (ppm): 147.8

### Reference Example 2

### Synthesis of modified CPG Rf12

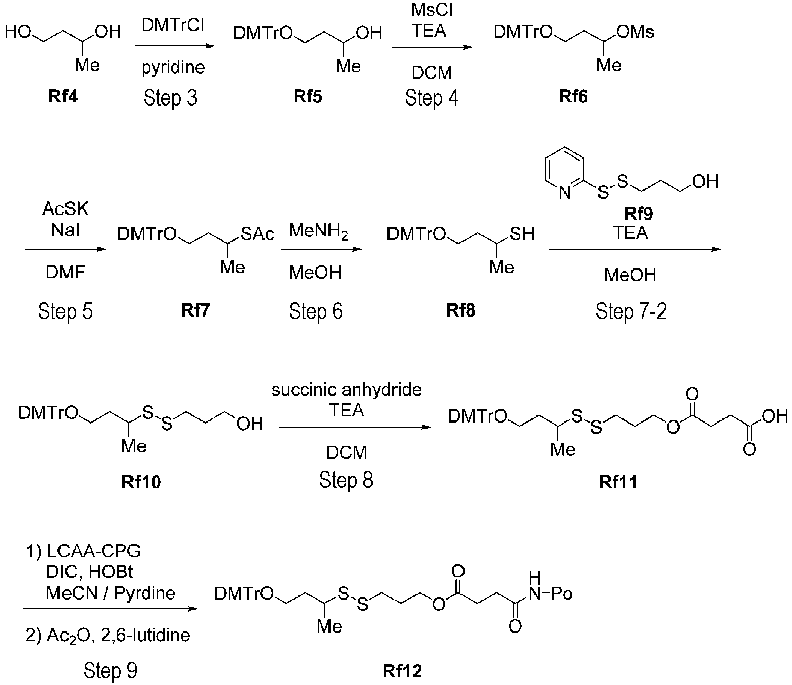

wherein Po is as defined above.

Compound Rf9 was synthesized by the following step 7-1:

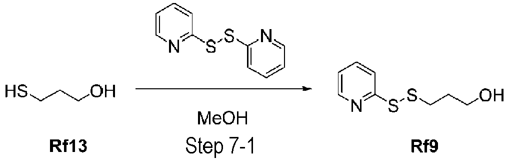

### Step 3:

4-(Bis(4-methoxyphenyl)(phenyl)methoxy)butan-2-ol Rf5 (4.00 g, 9.51 mmol, 2.7 wt% ethyl acetate, 4.3 wt% n-heptane) was obtained as a pale yellow oil (yield: 86%) in the same way as in step 1 of Reference Example 1 using commercially available butane-1,3-diol Rf4 (1.0 g, 11.1 mmol) .

ESI-MS (m/z): 415.0 (M + Na).

¹H-NMR (CDCl₃, 400 MHz) δ (ppm): 7.42 (d, J = 7.2 Hz, 2H), 7.32-7.19 (m, 7H), 6.83 (d, J = 8.8 Hz, 4H), 4.00-3.95 (m, 1H), 3.79 (s, 6H), 3.38-3.19 (m, 2H), 2.95 (d, J = 2.8 Hz, 1H), 1.83-1.66 (m, 2H), 1.16 (d, J = 6.4 Hz, 3H).

### Step 4:

To a 300 mL flask in an argon atmosphere, compound Rf5 (4.00 g, 10.2 mmol) obtained in step 3 and dehydrated dichloromethane (50 mL) were added. Triethylamine (2.84 mL, 20.4 mmol) and methanesulfonyl chloride (1.20 mL, 15.3 mmol) were added thereto under ice cooling, and the mixture was stirred at room temperature for 1 hour.

After the completion of reaction, the reaction solution was separated into aqueous and organic layers by the addition of a saturated aqueous solution of sodium bicarbonate and chloroform. The organic layer was dried over anhydrous sodium sulfate, and the solvent was concentrated under reduced pressure to obtain a crude product containing 4-(bis(4-methoxyphenyl)(phenyl)methoxy)butan-2-yl methanesulfonate Rf6 (5.30 g) as an orange oil. The obtained crude product was used directly in the next step (yield: quant.).

ESI-MS (m/z): 493 [M + Na].

¹H-NMR (CDCl₃, 400 MHz) δ (ppm): 7.40 (d, J = 7.6 Hz, 2H), 7.31-7.20 (m, 7H), 6.82 (d, J = 9.2 Hz, 4H), 5.04-5.00 (m, 1H), 3.79 (s, 6H), 3.23-3.14 (m, 2H), 2.75 (s, 3H), 1.97-1.88 (m, 2H), 1.43 (d, J = 6.4 Hz, 3H).

### Step 5:

To a 200 mL Kolben, the compound Rf6-containing crude product (5.30 g) obtained in step 4 and dehydrated N,N-dimethylformamide (96 mL) were added and suspended. To the suspension, potassium thioacetate (5.14 g, 45.1 mmol) and sodium iodide (0.170 g, 1.13 mmol) were added, and the mixture was stirred at 50°C for 3 hours. After the completion of reaction, water was added to the reaction solution, followed by extraction with n-heptane/ethyl acetate (1/1) twice. Combined organic layers were dried over anhydrous sodium sulfate, and the solvent was concentrated under reduced pressure to obtain a crude product (4.57 g) as an orange oil. The obtained residue was purified by silica gel column chromatography (Yamazen Corp., Hi-Flash Column L, developing solvent: n-heptane/ethyl acetate = 100/0 → 90/10 → 80/20) to obtain S-(4-(bis(4-methoxyphenyl)(phenyl)methoxy)butan-2-yl) ethanethioate Rf7 (3.98 g, 7.83 mmol, 11.3 wt% ethyl acetate) as an orange oil (2-stage yield: 70%).

ESI-MS (m/z): 473.2 [M + Na].

¹H-NMR (CDCl₃, 400 MHz) δ (ppm): 7.44-7.42 (m, 2H), 7.33-7.18 (m, 7H), 6.82 (d, J = 8.8 Hz, 4H), 3.79 (s, 6H), 3.76-3.71 (m, 1H), 3.18-3.07 (m, 2H), 2.26 (s, 3H), 1.90-1.79 (m, 2H), 1.25 (d, J = 7.2 Hz, 3H).

### Step 6:

To a 30 mL flask in an argon atmosphere, compound Rf7 (400 mg, 0.888 mmol) obtained in step 5 and dehydrated methanol (3.6 mL) were added and dissolved. To the solution, a solution of 40% methylamine in methanol (870 µL) was added, and the mixture was stirred for 1 hour. After the completion of reaction, the solvent was concentrated under reduced pressure to obtain a crude product (430 mg) as an orange oil. The residue was purified by silica gel column chromatography (Yamazen Corp., Hi-Flash Column M, developing solvent: n-heptane/ethyl acetate = 90/10 → 70/30) to obtain 4-(bis(4-methoxyphenyl)(phenyl)methoxy)butane-2-thiol Rf8 (324 mg, 0.717 mmol, 9.5 wt% ethyl acetate) as a colorless oil (yield: 81%).

ESI-MS (m/z): undetected.

¹H-NMR (CDCl₃, 400 MHz) δ (ppm): 7.44 - 7.42 (m, 2H), 7.33 - 7.20 (m, 7H), 6.82 (d, J = 8.8 Hz, 4H), 3.79 (s, 6H), 3.25 - 3.15 (m, 3H), 1.91 - 1.72 (m, 2H), 1.44 (d, J = 6.4 Hz,, 1H), 1.29 (d, J = 3.6 Hz, 3H).

### Step 7-1:

In a 100 mL flask in an argon atmosphere, 2, 2'- dipyridyl disulfide (5.00 g, 22.7 mmol) was dissolved in dehydrated methanol (33 mL). To the solution, commercially available 3-mercaptopropan-1-ol Rf13 (1.39 g, 15.1 mmol) was added, and the mixture was stirred at room temperature for 4 hours. After the completion of reaction, the solvent was concentrated under reduced pressure to obtain a yellow oil of a crude product (6.40 g). The crude product was purified twice by silica gel column chromatography (Yamazen Corp., Hi-Flash Column L, developing solvent: n-heptane/ethyl acetate = 90/10 → 70/30 → 50/50 → 30/70) to obtain 3-(pyridin-2-yldisulfanyl)propan-1-ol Rf9 (2.20 g, 10.9 mmol) as a colorless oil (yield: 72%).

ESI-MS (m/z): undetected.

¹H-NMR (CDCl₃, 400 MHz) δ (ppm): 8.48 - 8.46 (m, 1H), 7.66 - 7.61 (m, 2H), 7.14 - 7.09 (m, 1H), 3.81 (t, J = 6.0 Hz, 2H), 2.98 (t, J = 4.8 Hz, 2H), 2.41 (br, 1H), 1.99 - 1.92 (m, 2H).

### Step 7-2:

To a 50 mL flask in an argon atmosphere, compound Rf8 (480 mg, 1.18 mmol) obtained in step 6 and dehydrated methanol (12 mL) were added and suspended. To this mixed solution, compound Rf9 (710 mg, 3.52 mmol) obtained in step 7-1 and triethylamine (491 µL, 3.52 mmol) were added, and the mixture was stirred at room temperature for 2 hours. After the completion of reaction, the solvent was concentrated under reduced pressure to obtain a crude product (1.29 g) as a yellow oil. The residue was purified by column chromatography (Yamazen Corp., Hi-Flash Column M, developing solvent: n-heptane/ethyl acetate = 90/10 → 70/30 → 50/50) to obtain 3-((4-(bis(4-methoxyphenyl)(phenyl)methoxy)butan-2-yl)disulfanyl)propan-1-ol Rf10 (492 mg, 0.839 mmol, 15.0 wt% ethyl acetate) as a colorless oil (yield: 62%). ESI-MS (m/z): 521.3 [M+Na].

¹H-NMR (CDCl₃, 400 MHz) δ (ppm): 7.43 (d, J = 7.9 Hz, 2H), 7.33-7.20 (m, 7H), 6.83 (d, J = 8.4 Hz, 4H), 3.79 (s, 6H), 3.71 (br, 2H), 3.24-3.03 (m, 3H), 2.73 (t, J = 7.2 Hz, 2H), 2.02-1.95 (m, 1H), 1.88 (quin, J = 6.8 Hz, 2H), 1.78-1.69 (m, 1H), 1.28-1.23 (m, 3H).

### Step 8:

To a 50 mL flask in an argon atmosphere, compound Rf10 (492 mg, 0.987 mmol) obtained in step 7 and dehydrated dichloromethane (10 mL) were added and dissolved. To this solution, triethylamine (2.75 µL, 1.97 mmol) and succinic anhydride (150 mg, 1.48 mmol) were added, and the mixture was stirred at room temperature for 2 hours. After the completion of reaction, the solvent was concentrated under reduced pressure to obtain a crude product (1.20 g) as a yellow oil. The obtained residue was purified by silica gel column chromatography (Yamazen Corp., Hi-Flash Column M, developing solvent: chloroform/methanol (1% triethylamine) = 100/0 → 98/2 → 95/5 -> 90/10 → 80/20) and then subjected to azeotropy with acetonitrile to obtain 4-(3-((4-(bis(4-methoxyphenyl)(phenyl)methoxy)butan-2-yl)disulfanyl)propoxy)-4-oxobutanoic acid Rf11 (630 mg, 0.863 mmol, 18.0 wt% triethylamine (1.3 eq.)) as a light white oil (yield: 87%).

ESI-MS (m/z): 597 [M-H].

¹H-NMR (CDCl₃, 400 MHz) δ (ppm): 7.42 (d, J = 7.6 Hz, 2H), 7.32 - 7.20 (m, 7H), 6.82 (d, J = 8.8 Hz, 4H), 4.12 (t, J = 6.0 Hz, 2H), 3.79 (s, 6H), 3.23 - 3.02 (m, 3H), 2.67 (d, J = 7.6 Hz, 2H), 2.61 - 2.50 (m, 4H), 2.01 - 1.93 (m, 3H), 1.76 - 1.68 (m, 1H), 1.30 - 1.28 (m, 3H).

### Step 9:

To CPG resin (LCAA Controlled Pore Glass, 2.20 g), dehydrated acetonitrile (8.0 mL) was added, further diisopropylcarbodiimide (52 µL, 334 µmol) and 1-hydroxybenzotriazole (5.42 mg, 40 µmol) were added, and the mixture was shaken and stirred at room temperature for 10 minutes after hermetical sealing. Compound Rf11 (81 mg (6.6 wt%, triethylamine (1.3 eq.), 111 µmol) obtained in step 8 was dissolved in a mixed solvent of dehydrated pyridine (0.67 mL) and anhydrous acetonitrile (2.0 mL). The solution was added to the solution of the CPG resin, and the mixture was shaken and stirred at room temperature for 34 hours and 20 minutes after hermetical sealing. Further, diisopropylcarbodiimide (52 µL, 334 µmol) and 1-hydroxybenzotriazole (5.42 mg, 40 µmol) were added thereto, and the mixture was shaken and stirred at room temperature for 19 hours and 40 minutes after hermetical sealing. Further, diisopropylcarbodiimide (52 µL, 334 µmol) and 1-hydroxybenzotriazole (5.42 mg, 40 µmol) were added thereto, and the mixture was shaken and stirred at room temperature for 16 hours and 50 minutes after hermetical sealing. The CPG solution was filtered to remove the solution. Then, the CPG resin was washed in the order of methylene chloride → acetonitrile → methylene chloride and dried through argon gas to obtain modified CPG resin (1.75 g). A portion (12.0 mg) of the obtained CPG resin was treated with a solution of 2 wt% trichloroacetic acid in methylene chloride (25 mL), followed by the measurement of absorbance (absorbance (503 nm) = 2.051, loading 56.4 µmol/g; a calculation method for the loading abided by Current Protocol in Nucleic Acid Chemistry, Unit 3.2.14).

This operation was further performed in 2 batches using the same amounts (2.20 g) of the CPG resin to each obtain modified CPG resin (1.85 g (loading 55.0 µmol/g), 1.87 g, (loading 57.4 µmol/g)).

To the obtained CPG resin (1.75 g), a mixed solution of N-methylimidazole/tetrahydrofuran = 1/5.25 (22.0 mL) and a mixed solution of acetic anhydride/2,6-lutidine/tetrahydrofuran = 1/1/8 (21.9 mL) were added, and the mixture was shaken and stirred at room temperature for 16 hours. The solution was removed by filtration. Then, the CPG resin was washed in the order of methylene chloride → acetonitrile → methylene chloride and dried through argon gas to obtain modified CPG Rf12 (1.64 g). A portion (12.5 mg) of the obtained CPG resin was treated with a solution of 2 wt% trichloroacetic acid in methylene chloride (25 mL), followed by the measurement of absorbance (absorbance (503 nm) = 2.117, loading 55.9 µmol/g).

### Reference Example 3

### Synthesis of modified CPG Rf21

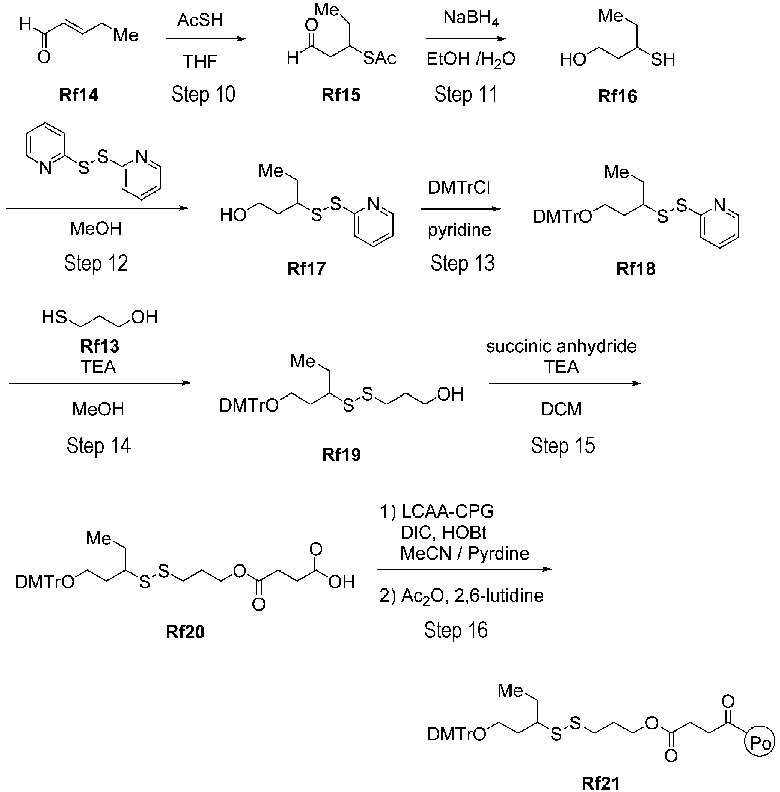

wherein Po is as defined above.

### Step 10:

In an argon atmosphere, commercially available trans-2-pentenal Rf14 (500 mg, 5.94 mmol) was dissolved in dehydrated tetrahydrofuran (3.0 mL). To this solution, thioacetic acid (635 µL, 8.92 mmol) was added, and the mixture was stirred at room temperature for 3 hours. After the completion of reaction, the reaction solution was quenched by the addition of a saturated aqueous solution of sodium bicarbonate, followed by extraction with ethyl acetate. The organic layer was washed with saturated saline, and the organic layer was dried over anhydrous sodium sulfate and then concentrated under reduced pressure to obtain S-(1-oxopentan-3-yl)ethanethioate Rf15 (875 mg) as a yellow oil (yield: 86%) .

ESI-MS (m/z): undetected.

¹H-NMR (CDCl₃, 400 MHz) δ (ppm): 9.70 (t, J = 2.0Hz, 1H), 3.92-3.86 (m, 1H), 2.74-2.71 (m, 2H), 2.33 (s, 3H), 1.77-1.62 (m, 2H), 0.98 (t, J = 7.2 Hz, 3H).

### Step 11:

In an argon atmosphere, sodium borohydride (340 mg, 8.91 mmol) and compound Rf15 (875 mg, 5.94 mmol) obtained in step 10 were added to a mixed solution of ethanol/water = 4/1 (20 mL) in an ice bath, and the mixture was stirred at room temperature for 40 minutes. After the completion of reaction, a saturated aqueous solution of ammonium chloride was added to the reaction solution, followed by extraction with ethyl acetate. The organic layer was washed with saturated saline and dried over anhydrous sodium sulfate. The obtained crude product was purified by column chromatography (Yamazen Corp., Hi-Flash Column L, developing solvent: n-heptane/ethyl acetate = 90/10 -> 50/50) to obtain 3-mercaptopentan-1-ol Rf16 (160 mg) as a colorless oil (yield: 21%).

ESI-MS (m/z): undetected.

¹H-NMR (CDCl₃, 400 MHz) δ (ppm): 3.89-3.78 (m, 2H), 2.93-2.85 (m, 1H), 2.05-1.94 (m, 1H), 1.79-1.50 (m, 4H), 1.38 (d, J = 8.4 Hz, 1H), 1.02 (t, J = 7.2 Hz, 3H).

### Step 12:

3-(Pyridin-2-yldisulfanyl)pentan-1-ol Rf17 (250 mg) was obtained as a colorless oil (yield: 79%) in the same way as in step 7-1 of Reference Example 2 using compound Rf16 (160 mg, 1.33 mmol) obtained in step 11.

ESI-MS (m/z): 230 [M+H].

¹H-NMR (CDCl₃, 400 MHz) δ (ppm): 8.47 (d, J = 4.4 Hz, 1H), 7.62-7.51 (m, 2H), 7.13-7.10 (m, 1H), 4.13-4.01 (m, 1H), 3.85-3.77 (m, 2H), 3.08-3.01 (m, 1H), 2.00-1.91 (m, 1H), 1.82-1.66 (m, 3H), 1.02 (t, J = 8.0 Hz, 3H).

### Step 13:

2-((1-(Bis(4-methoxyphenyl)(phenyl)methoxy)pentan-3-yl)disulfanyl)pyridine Rf18 (333 mg) was obtained as a colorless oil (yield: 51%) in the same way as in step 1 of Reference Example 1 using compound Rf17 (250 mg, 1.09 mmol) obtained in step 12.

ESI-MS (m/z): 570 [M + K].

¹H-NMR (CDCl₃, 400 MHz) δ (ppm): 8.40 (d, J = 4.8 Hz, 1H), 7.65 (d, J = 7.6 Hz, 1H), 7.51 (t, J = 7.6 Hz, 1H), 7.41 (d, J =7.6 Hz, 2H), 7.31-7.25 (m, 6H), 7.22-7.20 (m, 1H), 7.30 (t, J =7.6 Hz, 1H), 6.81 (d, J = 8.8 Hz, 4H), 3.79 (s, 6H), 3.24-3.14 (m, 2H), 2.99 (quin, J = 6.4 Hz, 1H), 1.89 (q, J = 6.0 Hz, 2H), 1.65-1.57 (m, 2H), 0.96 (t, J = 8.8 Hz, 3H).

### Step 14:

Compound Rf18 (333 mg, 0.626 mmol) obtained in step 13 was dissolved in dehydrated methanol (5.0 mL). To this solution, commercially available 3-mercapto-1-propanol Rf13 (43 µL, 0.501 mmol) was added, and the mixture was stirred at room temperature for 1 hour.

Since the starting materials remained, compound Rf13 (11 µL, 0.128 mmol) was added thereto, and the mixture was further stirred at room temperature for 3 hours. After the completion of reaction, the reaction solution was concentrated to obtain a crude product as a yellow oil (0.69 g). The obtained crude product was purified by column chromatography (Yamazen Corp., Hi-Flash Column M, developing solvent: n-heptane/ethyl acetate (2% triethylamine) = 95/5 → 80/20 → 70/30) to obtain 3-((1-(bis(4-methoxyphenyl)(phenyl)methoxy)pentan-3-yl)disulfanyl)propan-1-ol Rf19 (215 mg) as a colorless oil (yield: 57%).

ESI-MS (m/z): 552 [M + K].

¹H-NMR (CDCl₃, 400 MHz) δ (ppm): 7.42 (d, J = 7.2 Hz, 1H), 7.33-7.20 (m, 7H), 6.82 (d, J = 8.8 Hz, 4H), 3.79 (s, 6H),

3.69 (q, J = 5.6 Hz, 2H), 3.20 (dt, J = 2.4, 7.2 Hz, 2H), 2.81 (quin, J = 6.4 Hz, 1H), 2.68 (t, J = 7.2 Hz, 2H), 1.94-1.83 (m, 4H), 1.63-1.57 (m, 2H), 1.35 (t, J = 5.6 Hz, 1H), 0.968 (t, J = 8.0 Hz, 3H).

### Step 15:

4-(3-((1-(Bis(4-methoxyphenyl)(phenyl)methoxy)pentan-3-yl)disulfanyl)propoxy)-4-oxobutanoic acid Rf20 (210 mg, 6.6 wt% triethylamine (0.43 eq.)) was obtained as a colorless oil (yield: 90%) in the same way as in step 8 of Reference Example 2 using compound Rf19 (215 mg, 0.354 mmol) obtained in step 14.

ESI-MS (m/z): 612 [M-H].

¹H-NMR (CDCl₃, 400 MHz) δ (ppm): 7.41 (d, J = 7.6 Hz, 2H), 7.32-7.20 (m, 7H), 6.82 (d, J = 9.2 Hz, 4H), 4.13 (t, J = 6.0 Hz, 2H), 3.79 (s, 6H), 3.21-3.17 (m, 2H), 2.83-2.77 (m, 1H), 2.64-2.57 (m, 6H), 1.98-1.85 (m, 4H), 1.64-1.56 (m, 2H), 0.962 (t, J = 6.8 Hz, 3H).

### Step 16:

Modified CPG Rf21 (2.10 g) was obtained in the same way as in step 9 of Reference Example 2 using compound Rf20 (61 mg, 0.1 mmol) obtained in step 15 and CPG resin (LCAA Controlled Pore Glass, 2.20 g). A portion (24.3 mg) of the obtained CPG resin was treated with a solution of 2 wt% trichloroacetic acid in methylene chloride (25 mL), followed by the measurement of absorbance (absorbance (503 nm) = 2.490, loading 33.7 µmol/g).

### Reference Example 4

### Synthesis of modified CPG Rf29

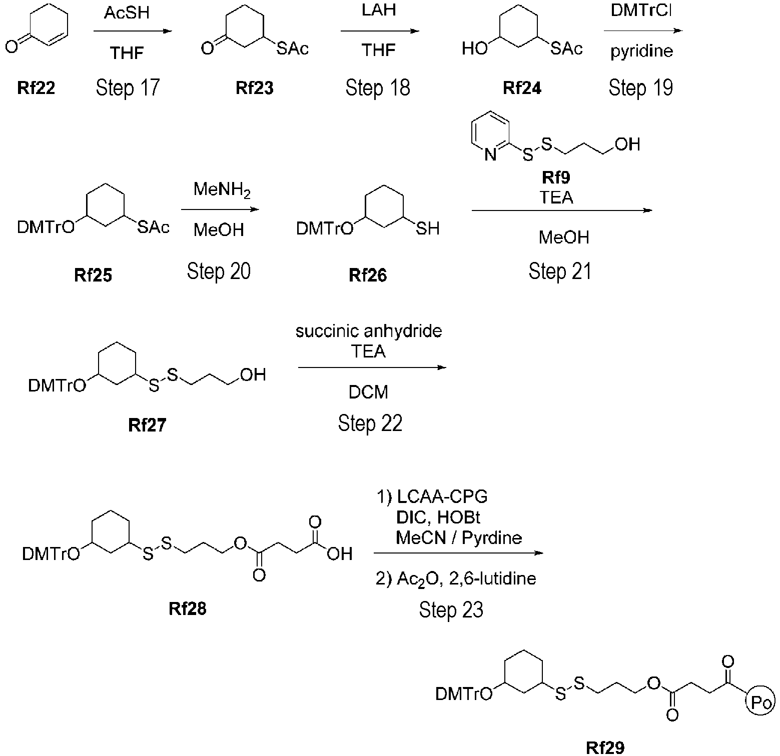

wherein Po is as defined above.

### Step 17:

S-(3-Oxocyclohexyl)ethanethioate Rf23 (8.83 g, 49.0 mmol, 4.4 wt% ethyl acetate) was obtained as an orange oil (yield: 94%) in the same way as in step 10 of Reference Example 3 using commercially available 2-cyclohexen-1-one Rf22 (5.00 g, 52 mmol).

ESI-MS (m/z): undetected.

¹H-NMR (CDCl₃, 400 MHz) δ (ppm): 3.89-3.82 (m, 1H), 2.72 (dd, J = 3.2, 14.4 Hz, 1H), 2.49-2.29 (m, 3H), 2.32 (s, 3H), 2.13 (br, 1H), 2.09-1.99 (m, 1H), 1.89-1.75 (m, 2H).

### Step 18:

To a 500 mL Kolben in an argon atmosphere, dehydrated tetrahydrofuran (100 mL) was added. Lithium aluminum hydride (1.95 g, 51.3 mmol) was added thereto under ice cooling, and then, compound Rf23 (8.83 g, 51.3 mmol) obtained in step 17 was added over 20 minutes in a salt ice bath. After the addition, the mixture was stirred for 1 hour in an ice bath (inside temperature: 21°C). After the completion of reaction, the reaction solution was quenched by the addition of a saturated aqueous solution of ammonium chloride under ice cooling (inside temperature: 5 to 10°C), followed by extraction with ethyl acetate. The organic layer was washed with water and saturated saline and dried over anhydrous sodium sulfate. The solvent was distilled off under reduced pressure to obtain a crude product (7.18 g) as an orange oil. The residue was purified by column chromatography (Yamazen Corp., Hi-Flash Column 3L, developing solvent: n-heptane/ethyl acetate = 80/20 → 70/30 → 50/50) to obtain S-(3-hydroxycyclohexyl)ethanethioate Rf24 (5.81 g, 30.0 mmol, 10 wt% ethyl acetate) as an orange oil (yield: 59%). ESI-MS (m/z): 197.9 [M + Na].

### Step 19:

S-(3-(Bis(4-methoxyphenyl)(phenyl)methoxy)cyclohexyl)ethanethioate Rf25 (1.70 g, 3.44 mmol, 3.5 wt% acetonitrile) was obtained as a colorless amorphous product (yield: 21%) in the same way as in step 1 of Reference Example 1 using compound Rf24 (2.90 g, 16.6 mmol) obtained in step 18. ESI-MS (m/z): 499.1 [M + Na].

¹H-NMR (CDCl₃, 400 MHz) δ (ppm): 7.53-7.47 (m, 2H), 7.42-7.35 (m, 4H), 7.30-7.17 (m, 3H), 6.83-6.80 (m, 4H), 3.79 (s, 6H), 3.46-3.41 (m, 1H), 3.16-3.10 (m, 1H), 2.24 (s, 3H), 1.74 (br, 1H), 1.62-1.56 (s, 2H), 1.38-1.04 (m, 5H).

### Step 20:

3-(Bis(4-methoxyphenyl)(phenyl)methoxy)cyclohexane-1-thiol Rf26 (1.41 g, 2.80 mmol, 13.7 wt% ethyl acetate) was obtained as a colorless oil (yield: 78%) in the same way as in step 6 of Reference Example 2 using compound Rf25 (1.70 g, 3.57 mmol) obtained in step 19.

ESI-MS (m/z): undetected.

¹H-NMR (CDCl₃, 400 MHz) δ (ppm): 7.45-7.47 (m, 2H), 7.39-7.36 (m, 4H), 7.29-7.18 (m, 3H), 6.83-6.80 (m, 4H), 3.79 (s, 6H), 3.36-3.29 (m, 1H), 2.47-2.38 (m, 1H), 1.85-1.71 (m, 2H), 1.60-1.50 (m, 1H), 1.43 (d, J = 7.2 Hz, 1H), 1.31-1.05 (m, 4H), 1.18-0.90 (m, 1H).

### Step 21:

3-((3-(Bis(4-methoxyphenyl)(phenyl)methoxy)cyclohexyl)disulfanyl)propa n-1-ol Rf27 (457 mg, 0.785 mmol, 9.8 wt% ethyl acetate) was obtained as a colorless oil (yield: 41%) in the same way as in step 7-2 of Reference Example 2 using compound Rf26 (780 mg, 3.87 mmol) obtained in step 20.

ESI-MS (m/z): 564.6 [M + K].

¹H-NMR (CDCl₃, 400 MHz) δ (ppm): 7.51-7.48 (m, 2H), 7.41-7.37 (m, 4H), 7.30-7.18 (m, 3H), 6.84-6.80 (m, 4H), 3.79 (s, 6H), 3.75-3.69 (m, 2H), 3.40-3.32 (m, 1H), 2.70-2.61 (m, 2H), 2.39-2.31 (m, 1H), 1.96-1.80 (m, 2H), 1.69-1.43 (m, 4H), 1.28-0.983 (m , 4H).

### Step 22:

4-(3-((3-(Bis(4-methoxyphenyl)(phenyl)methoxy)cyclohexyl)disulfanyl)propo xy)-4-oxobutanoic acid Rf28 (227 mg, 0.360 mmol, 7.2 wt% triethylamine (0.53 eq.)) was obtained as a colorless oil (yield: 35%) in the same way as in step 8 of Reference Example 2 using compound Rf27 (215 mg, 0.354 mmol) obtained in step 21.

ESI-MS (m/z): 623 [M-H].

¹H-NMR (CDCl₃, 400 MHz) δ (ppm): 7.53-7.47 (m, 2H), 7.42-7.35 (m, 4H), 7.30-7.18 (m, 3H), 6.85-6.79 (m, 4H), 4.16-4.10 (m, 2H), 3.79 (s, 6H), 3.40-3.30 (m, 1H), 3.02-2.96 (m, 1H), 2.63-2.56 (m, 6H), 2.41-2.30 (m, 1H), 2.01-1.90 (m, 2H), 1.84-1.78 (m, 1H), 1.70-1.58 (m, 2H), 1.45-1.39 (m, 1H), 1.28-0.96 (m, 4H).

### Step 23:

Modified CPG Rf29 (1.08 g) was obtained in the same way as in step 9 of Reference Example 2 using compound Rf28 (42.8 mg, 68.5 µmol) obtained in step 22 and CPG resin (LCAA Controlled Pore Glass, 1.20 g). A portion (14.9 mg) of the obtained CPG resin was treated with a solution of 2 wt% trichloroacetic acid in methylene chloride (25 mL), followed by the measurement of absorbance (absorbance (503 nm) = 2.146, loading 47.3 µmol/g).

### Reference Example 5

### Synthesis of modified CPG Rf37

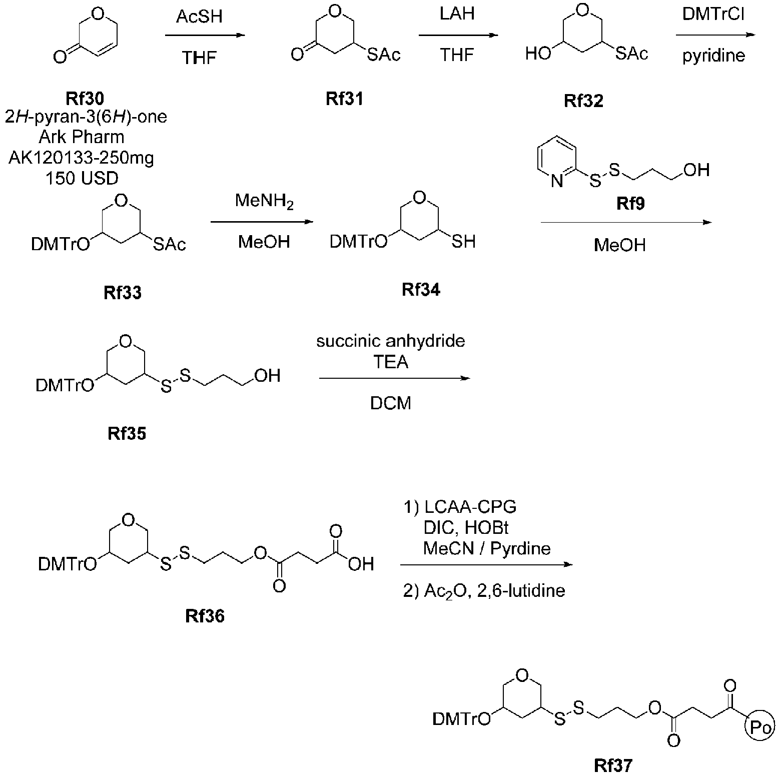

wherein Po is as defined above.

Modified CPG Rf37 can be synthesized by the same synthesis method as in Reference Example 4 using commercially available 2H-pyran-3(6H)-one.

### Reference Example 6

### Synthesis of amidite Rf41

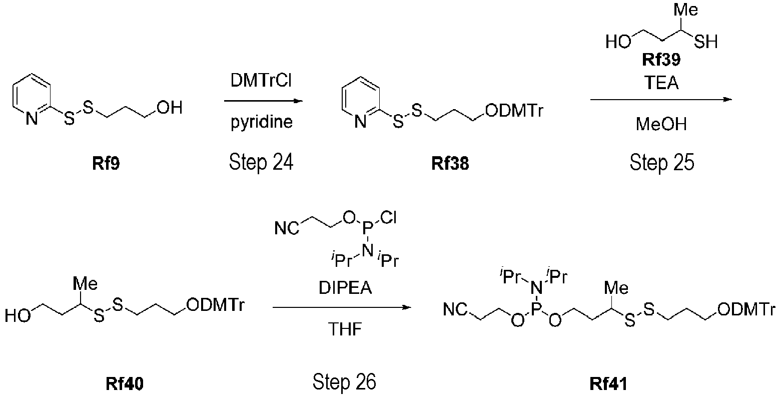

### Step 24:

2-((3-(Bis(4-methoxyphenyl)(phenyl)methoxy)propyl)disulfanyl)pyridine Rf38 (2.96 g) was obtained as a colorless oil (yield: 90%) in the same way as in step 1 of Reference Example 1 using compound Rf9 (1.32 g, 6.56 mmol) obtained in step 7-1 of Reference Example 2.

### ESI-MS (m/z): undetected.

¹H-NMR (CDCl₃, 400 MHz) δ (ppm): 8.45-8.44 (m, 1H), 7.69 (d, J = 0.8 Hz, , 1H), 7.67-7.58 (m, 1H), 7.42-7.40 (m, 2H), 7.31-7.25 (m, 6H), 7.22-7.16 (m, 1H), 7.08-7.04 (m, 1H), 6.84-6.79 (m, 4H), 3.78 (s, 6H), 3.15 (t, J = 6.0 Hz, 2H), 2.93 (t, J = 7.6 Hz, 2H), 2.00-1.96 (m, 2H).

### Step 25:

3-((3-(Bis(4-methoxyphenyl)(phenyl)methoxy)propyl)disulfanyl)butan-1-ol Rf40 (1.86 g) was obtained as a colorless oil (yield: 67%) in the same way as in step 7-2 of Reference Example 2 using compound Rf38 (2.49 g, 4.94 mmol) obtained in step 24 and commercially available 3-mercaptobutan-1-ol Rf39 (787 mg, 7.42 mmol).

ESI-MS (m/z): 521.7 [M + Na].

¹H-NMR (CDCl₃, 400 MHz) δ (ppm): 7.44-7.41 (m, 2H), 7.33-7.26 (m, 6H), 7.22-7.19 (m, 1H), 6.84-6.80 (m, 4H), 3.83-3.68 (m, 2H), 3.79 (s, 6H), 3.15 (t, J = 6.4 Hz, 2H) , 3.02-2.94 (m, 1H), 2.80 (t, J = 6.8 Hz, 2H), 2.60 (t, J = 6.4 Hz, 1H), 2.01-1.92 (m, 2H), 1.80-1.71 (m, 1H), 1.41 (t, J = 5.6 Hz, 1H), 1.34 (d, J = 6.8 Hz, 3H).

### Step 26:

3-((3-(Bis(4-methoxyphenyl)(phenyl)methoxy)propyl)disulfanyl)butyl(2-cyanoethyl)diisopropylphosphoramidite Rf41 (230 mg, 0.300 mmol, 8.9 wt% AcOEt) was obtained as a colorless oil (yield: 39%) in the same way as in step 2 of Reference Example 1 using compound Rf40 (380 mg, 0.762 mmol) obtained in step 25.

ESI-MS (m/z): undetected.

¹H-NMR (CDCl₃, 400 MHz) δ (ppm): 7.40-7.41 (m, 2H), 7.32-7.26 (m, 6H), 7.22-7.20 (m, 1H), 6.84-6.79 (m, 4H), 3.86-3.53 (m, 4H), 3.80 (s, 6H), 3.55-3.61 (m, 2H), 3.18-3.13 (m, 2H) , 3.00-2.94 (m, 1H), 2.81-2.58 (m, 4H), 2.01-1.74 (m, 4H), 1.34 (dd, 1.2, 6.8 Hz, 3H), 1.29-1.22 (m, 2H), 1.17 (dt, J = 1.2, 4.8 Hz, 10H)

³¹P-NMR (CDCl₃, 162 MHz) δ (ppm): 148.3

### Reference Example 7

### Synthesis of amidite Rf46

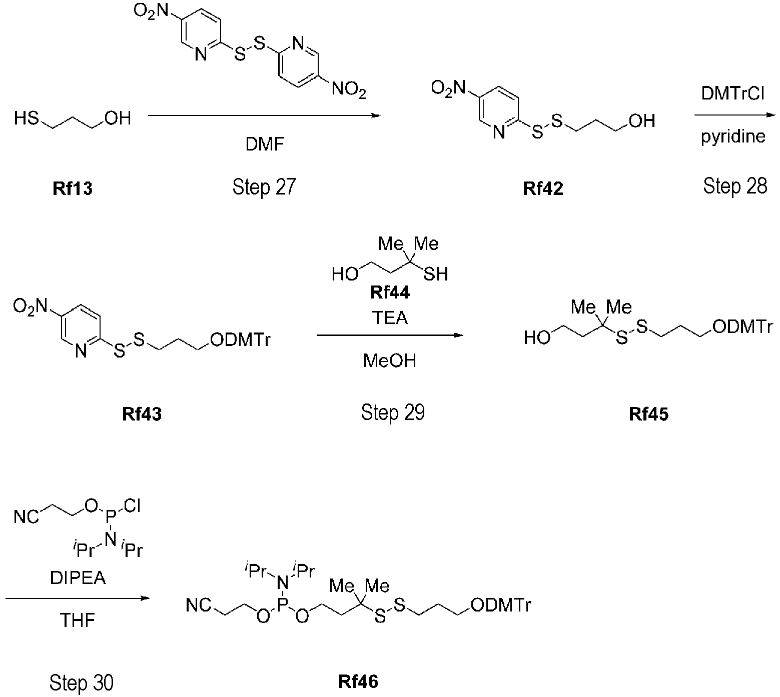

### Step 27:

2,2'-Dithiobis(5-nitropyridine) (5.95 g, 19.2 mmol) was dissolved in dehydrated N,N-dimethylformamide (118 mL). To the solution, commercially available 3-mercaptopropan-1-ol Rf13 (1.18 g, 12.8 mmol) was added, and the mixture was then stirred at room temperature for 4 hours. Water was added to the reaction solution, followed by extraction with isopropyl ether. Then, water was added to the organic layer, followed by extraction with ethyl acetate. The organic layer was washed with saturated saline, then dried over anhydrous sodium sulfate, and concentrated under reduced pressure to obtain a yellow solid of a crude product (7.01 g). The obtained crude product was suspended and washed with n-heptane/ethyl acetate = 50/50 to obtain an orange solid of a crude product (4.98 g). The crude product was purified by column chromatography (amount of silica: 90 g, developing solvent: n-heptane/ethyl acetate = 2/1 -> 1/1 (1% triethylamine)) to obtain 3-((5-nitropyridin-2-yl)disulfanyl)propan-1-ol Rf42 (2.09 g) as a yellow oil (yield: 66%).

ESI-MS (m/z): undetected.

¹H-NMR (CDCl₃, 400 MHz) δ (ppm): 9.28 (d, J = 1.6 Hz, 1H), 8.41 (dd, J = 2.4, 8.8 Hz, 1H), 7.89 (d, J = 8.8 Hz, 1H), 3.79 (t, J = 5.6 Hz, 2H), 2.99 (t, J = 8.8 Hz, 2H), 2.00-1.94 (m, 2H).

### Step 28:

2-((3-(Bis(4-methoxyphenyl)(phenyl)methoxy)propyl)disulfanyl)-5-nitropyridine Rf43 (3.99 g) was obtained as a pale yellow oil (yield: 91%) in the same way as in step 1 of Reference Example 1 using compound Rf42 (1.97 g, 8.00 mmol) obtained in step 27.

ESI-MS (m/z): undetected.

¹H-NMR (CDCl₃, 400 MHz) δ (ppm): 9.24 (d, J = 1.6 Hz, 1H), 8.3 (dd, J = 2.8, 8.8 Hz, 1H), 7.85 (d, J = 8.8 Hz, 1H), 7.41-7.38 (m, 2H), 7.31-7.16 (m, 7H), 6.84-6. 80 (m, 4H), 3.79 (s, 6H), 3.18 (t, J = 6.0 Hz, 2H), 2.78 (t, J = 7.2 Hz, 2H), 1.97 (quin, J = 7.2 Hz, 2H).

### Step 29:

3-((3-(Bis(4-methoxyphenyl)(phenyl)methoxy)propyl)disulfanyl)-3-methylbutan-1-ol Rf45 (3.72 g, 6.98 mmol) was obtained as a colorless liquid (yield: 93%) in the same way as in step 7-2 of Reference Example 2 using compound Rf43 (4.13 g, 7.53 mmol) obtained in step 28 and commercially available 3-mercapto-3-methylbutan-1-ol Rf44 (1.36 g, 11.3 mmol) .

ESI-MS (m/z): 535.0 [M + Na].

¹H-NMR (CDCl₃, 400 MHz) δ (ppm): 7.34-7.40 (m, 2H), 7.33-7.26 (m, 6H), 7.22-7.18 (m, 1H), 6.84-6.80 (m, 4H), 3.79 (s, 6H), 3.79-3.75 (m, 2H), 3.14 (t, J = 6.4 Hz, 2H), 2.82 (t, J = 7.2 Hz, 2H), 1.96-1.86 (m, 4H), 1.44 (br, 1H), 1.32 (s, 6H).

### Step 30:

3-((3-(Bis(4-methoxyphenyl)(phenyl)methoxy)propyl)disulfanyl)-3-methylbutyl(2-cyanoethyl)diisopropylphosphoramidite Rf46 (1.15 g, 1.46 mmol) was obtained as a colorless oil (yield: 75%) in the same way as in step 2 of Reference Example 1 using compound Rf45 (1.00 g, 1.95 mmol) obtained in step 29.

ESI-MS (m/z): undetected.

¹H-NMR (CDCl₃, 400 MHz) δ (ppm): 7.43-7.41 (m, 2H), 7.32-7.28 (m, 6H), 7.22-7.18 (m, 1H), 6.84-6.80 (m, 4H), 3.86-3.69 (m, 4H), 3.79 (s, 6H), 3.63-3.56 (m, 2H), 3.14 (t, J = 6.0 Hz, 2H) , 2.81 (t, J =7.6 Hz, 2H), 2.61 (t, J = 6.4 Hz, 2H), 1.94 (quin, J = 7.6 Hz, 4H), 1.32 (s, 6H), 1.18 (dd, J = 4.8, 6.8 Hz, 12H).

³¹P-NMR (CDCl₃, 162 MHz) δ (ppm): 147.9

### Reference Example 8

### Synthesis of carboxylic acid triethylamine salt Rf54

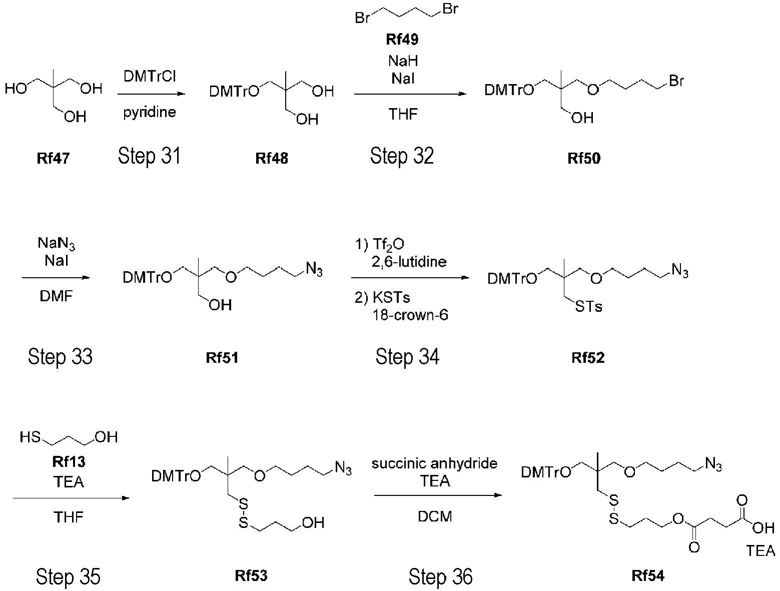

### Step 31:

2-((Bis(4-methoxyphenyl)(phenyl)methoxy)methyl)-2-methylpropane-1,3-diol Rf48 (4.88 g, containing 5.4 wt% ethyl acetate, 10.9 mmol) was obtained as a pale yellow amorphous product (yield: 44%) in the same way as in step 1 of Reference Example 1 using commercially available 2-(hydroxymethyl)-2-methylpropane-1,3-diol Rf47 (3.00 g, 25.0 mmol).

ESI-MS (m/z): undetected.

¹H-NMR (CDCl₃, 400 MHz) δ (ppm): 7.43-7.41 (m, 2H), 7.34-7.19 (m, 7H), 6.85-6.82 (m, 4H), 3.79 (s, 6H), 3.69 (d, J = 11.2 Hz, 2H), 3.58 (d, J = 11.2 Hz, 2H), 3.14 (s, 2H), 2.32 (br, 2H), 0.834 (s, 3H).

### Step 32:

In an argon atmosphere, compound Rf48 (4.88 g, containing 5.4 wt% ethyl acetate, 10.9 mmol) obtained in step 31 was dissolved in anhydrous tetrahydrofuran (50 mL), and the solution was cooled in ice. Sodium hydride (60% in mineral oil, 436 mg, 10.9 mmol), sodium iodide (168 mg, 1.12 mmol), and 1,4-dibromobutane Rf49 (1.29 ml, 10.9 mmol) were added thereto in this order, and the mixture was stirred for 100 minutes under ice cooling. The ice bath was removed, and the reaction mixture was stirred for 17 hours while spontaneously warmed to room temperature. Since the reaction proceeded only by 30%, the reaction solution was cooled in ice again, and sodium hydride (60% in mineral oil, 436 mg, 10.9 mmol) and 1,4-dibromobutane Rf49 (1.29 ml, 10.9 mmol) were added thereto. The ice bath was immediately removed, and the reaction mixture was stirred for 3 hours while spontaneously warmed to room temperature. Although the starting materials remained, the reaction solution was cooled in ice and quenched by the slow addition of a saturated aqueous solution of ammonium chloride (50 mL). Water (50 mL) was further thereto, followed by extraction with ethyl acetate (0.15 L). The organic layer was washed with water (50 mL) and further washed with saturated saline (50 mL). The organic layer was dried over anhydrous sodium sulfate and then concentrated under reduced pressure to obtain a yellow liquid of a crude product. The obtained crude product was purified by column chromatography (Yamazen Corp., Hi-Flash Column 3L, developing solvent: n-heptane/ethyl acetate = 100/0 -> 0/100) to obtain 3-(bis(4-methoxyphenyl) (phenyl)methoxy)-2-((4-bromobutoxy)methyl)-2-methylpropan-1-ol Rf50 (3.24 g, containing 12 wt% ethyl acetate, 5.09 mmol) as a colorless viscous liquid (yield: 47%).

ESI-MS (m/z): undetected.

¹H-NMR (CDCl₃, 400 MHz) δ (ppm): 7.43-7.41 (m, 2H), 7.32-7.16 (m, 7H), 6.85-6.82 (m, 4H), 3.79 (s, 6H), 3.55-3.38 (m, 6H), 3.08 (q, J = 10.8 Hz, 2H), 2.70 (t, J = 6.0 Hz, 1H), 1.91-1.84 (m, 2H), 1.72-1.65 (m, 2H), 1.23 (br, 2H), 0.884 (s, 3H).

### Step 33:

In an argon atmosphere, compound Rf50 (1.00 g, containing 12 wt% ethyl acetate, 1.58 mmol) obtained in step 32 was dissolved in anhydrous N,N-dimethylformamide (18 mL). To the solution, sodium iodide (134 mg, 0.897 mmol) and sodium azide (233 mg, 3.59 mmol) were added, and the mixture was stirred at room temperature for 8 hours. The reaction solution was cooled in ice, and water (20 mL) was added thereto. After extraction with a mixed solvent of n-heptane/ethyl acetate = 1/1 (50 mL), the extract was washed twice with water (50 mL x 2). The organic layer was dried over anhydrous sodium sulfate and then concentrated under reduced pressure to obtain a crude product (0.89 g) as a colorless viscous liquid. The obtained crude product was purified by column chromatography (Yamazen Corp., Hi-Flash Column M, developing solvent: n-heptane/ethyl acetate = 100/0 → 30/70) to obtain 3-(4-azidobutoxy)-2-((bis(4-methoxyphenyl)(phenyl)methoxy)methyl)-2-methylpropan-1-ol Rf51 (720 mg, containing 4.3 wt% ethyl acetate, 1.33 mmol) as a colorless liquid (yield: 84%).

ESI-MS (m/z): 542 [M + Na].

¹H-NMR (CDCl₃, 400 MHz) δ (ppm): 7.43-7.41 (m, 2H), 7.32-7.16 (m, 7H), 6.84-6.81 (m, 4H), 3.79 (s, 6H), 3.55-3.40 (m, 6H), 3.28-3.25 (m, 2H), 3.08 (q, J = 11.2 Hz, 2H), 1.63-1.57 (m, 4H), 0.882 (s, 3H).

### Step 34:

In an argon atmosphere, compound Rf51 (1.00 g, 1.92 mmol) obtained in step 33 was dissolved in dehydrated methylene chloride (10 mL), and the solution was cooled in an ice bath (inside temperature: 5°C). 2,6-Lutidine (1.21 mL, 10.4 mmol) and trifluoromethanesulfonic anhydride (0.39 ml, 2.31 mmol) were added thereto, and the mixture was stirred for 1 hour while cooled in an ice bath. The reaction mixture was quenched by the addition of ice water (0.10 L), followed by extraction with ethyl acetate (0.20 L). The organic layer was dried over anhydrous sodium sulfate and concentrated under reduced pressure to obtain a crude product (1.25 g) as a red liquid.

The crude product was dissolved in dehydrated N,N-dimethylformamide (10 mL). To the solution, 18-crown-6 (1.22 g, 4.60 mmol) and potassium p-toluenethiosulfonate (0.87 g, 3.84 mmol) were added, and the mixture was stirred at room temperature for 20 hours after hermetical sealing. The reaction mixture was quenched by the addition of water (0.10 L), followed by extraction with a solvent of heptane/ethyl acetate = 1/1 twice (0.15 L x 2). The organic layer was washed with water (0.10 L) and dried over anhydrous sodium sulfate. The residue was concentrated under reduced pressure to obtain a crude product (1.35 g). The obtained crude product was purified by column chromatography (Yamazen Corp., Hi-Flash Column L, developing solvent: n-heptane/ethyl acetate = 100/0 → 80/20) to obtain S-(3-(4-azidobutoxy)-2-((bis(4-methoxyphenyl)(phenyl)methoxy)methyl)-2-methylpropyl) 4-methylbenzene benzenesulfonothioate Rf52 (1.07 g, 1.55 mmol) as a yellow liquid (2-stage yield: 81%) .

ESI-MS (m/z): undetected.

¹H-NMR (CDCl₃, 400 MHz) δ( ppm): 7.78-7.75 (m, 2H), 7.38-7.33 (m, 2H), 7.29-7.18 (m, 9H), 6.85-6.78 (m, 4H), 3.80 (s, 6H), 3.31 (t, J = 5.6 Hz, 2H), 3.25-3.17 (m, 4H), 3.70 (d, J = 2.0 Hz, 2H), 2.88 (q, J = 8.8 Hz, 2H), 2.42 (s, 3H), 1.59-1.52 (m, 4H), 0.879 (s, 3H).

### Step 35:

3-((3-(4-Azidobutoxy)-2-((bis(4-methoxyphenyl)(phenyl)methoxy)methyl)-2-methylpropyl)disulfanyl)propan-1-ol Rf53 (858 mg, 1.37 mmol) was obtained as a colorless liquid (yield: 90%) in the same way as in step 7-2 of Reference Example 2 using compound Rf52 (1.05 g, 1.52 mmol) obtained in step 34 and commercially available 3-mercaptopropan-1-ol Rf13 (0.16 mL, 1.83 mmol).

ESI-MS (m/z): undetected.

¹H-NMR (CDCl₃, 400 MHz) δ (ppm): 7.43-7.40 (m, 2H), 7.32-7.18 (m, 7H), 6.84-6.80 (m, 4H), 3.79 (s, 6H), 3.72 (q, J = 6.0 Hz, 2H), 3.39 (t, J = 7.2 Hz, 2H), 3.34 (dd, J =5.6, 14.0 Hz, 2H), 3.26 (t, J = 6.4 Hz, 2H), 2.97 (q, J = 7.2 Hz, 2H), 2.91 (dd, J = 5.6, 18.0 Hz, 2H), 2.75 (t, J = 8.8 Hz, 2H), 1.92 (quin, J = 5.6 Hz, 2H), 1.63-1.58 (m, 4H), 1.44 (br, 1H), 0.999 (s, 3H).

### Step 36:

4-(3-((3-(4-Azidobutoxy)-2-((bis(4-methoxyphenyl)(phenyl)methoxy)methyl)-2-methylpropyl)disulfanyl)propoxy)-4-oxobutanoic acid triethylamine salt Rf54 (373 mg, 0.432 mmol) was obtained as a colorless liquid (yield: 90%) in the same way as in step 8 of Reference Example 2 using compound Rf53 (300 mg, 0.480 mmol) obtained in step 35.

ESI-MS (m/z): 725.0 [M-H].

¹H-NMR (CDCl₃, 400 MHz) δ (ppm): 7.43-7.41 (m, 2H), 7.31-7.18 (m, 7H), 6.84-6.80 (m, 4H), 4.14 (t, 6.4 Hz, 2H), 3.79 (s, 6H), 3.39-3.24 (m, 6H), 2.97 (q, J = 7.2 Hz, 2H), 2.92-2.85 (m, 2H), 2.69 (t, J = 7.2 Hz, 2H), 2.61-2.50 (m, 4H), 1.98 (quin, J = 6.4 Hz, 2H), 1.62-1.57 (m, 4H), 0.990 (s, 3H).

### Reference Example 9

### Synthesis of modified CPG Rf59

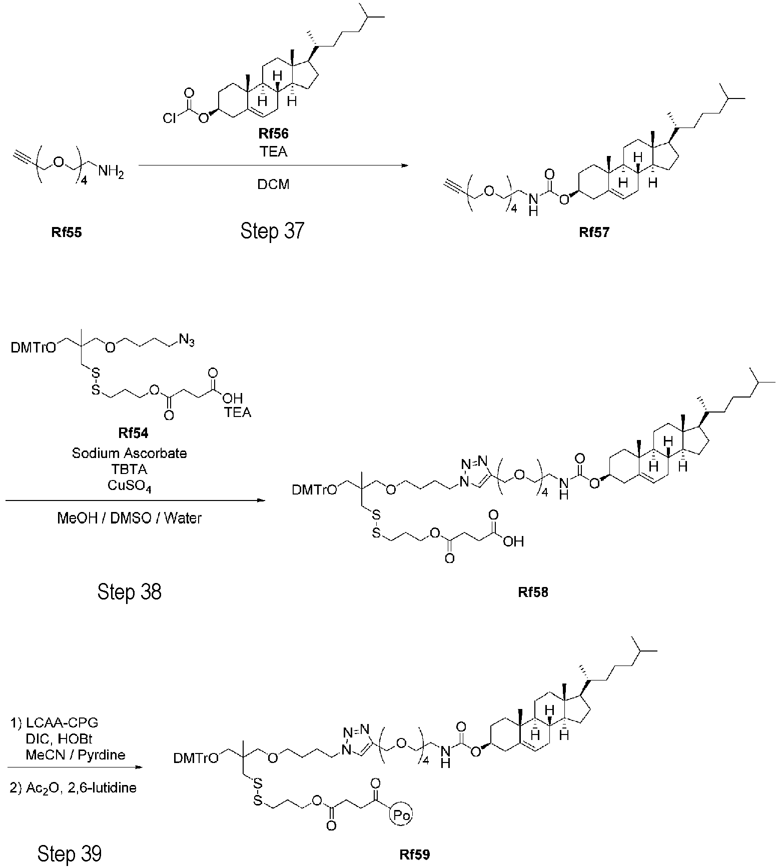

wherein Po is as defined above.

### Step 37:

Commercially available 3,6,9,12-tetraoxapentadec-14-yn-1-amine Rf55 (75.0 mg, 0.324 mmol) was dissolved in dehydrated methylene chloride (3.0 mL). To the solution, cholesterol chloroformate Rf56 (582 mg, 1.30 mmol) and triethylamine (294 µL, 2.11 mmol) were added, and the mixture was stirred at room temperature for 2 hours and 10 minutes after hermetical sealing. Methanol (10 mL) was added to the reaction solution, and the mixture was quenched by stirring at room temperature for 5 minutes and concentrated under reduced pressure to obtain a crude product (1.97 g). The obtained crude product was purified by column chromatography (Yamazen Corp., Hi-Flash Column M, developing solvent: n-heptane/ethyl acetate = 100/0 → 60/40) to obtain (3S,8S,9S,10R,13R,14S,17R)-10,13-dimethyl-17-((R)-6-methylheptan-2-yl)-2,3,4,7,8,9,10,11,12,13,14,15,16,17-tetradecahydro-1H-cyclopenta[a]phenanthren-3-yl (3,6,9,12-tetraoxapentadec-14-yn-1-yl)carbamate Rf57 (129 mg, 0.201 mmol) as a colorless viscous liquid (yield: 62%) .

ESI-MS (m/z): undetected.

¹H-NMR (CDCl₃, 400 MHz) δ (ppm): 5.37 (d, J = 5.2 Hz, 1H), 5.17 (br, 1H), 4.50 (br, 1H), 4.21 (d, J = 2.4 Hz, 2H), 3.73-3.60 (m, 12H), 3.55 (t, J = 5.2 Hz, 2H), 3.36 (br, 2H), 2.43 (t, J = 2.4 Hz, 1H), 2.39-2.24 (m, 2H), 2.04-1.78 (m, 5H), 1.59-0.939 (m, 21H), 1.00 (s, 3H), 0.911 (d, J = 6.4 Hz, 3H), (dd, J = 2.0, 6.4 Hz, 6H), 0.675 (s,3H).

### Step 38:

Compound Rf57 (73 mg, 0.114 mmol) obtained in step 37 and carboxylic acid triethylamine salt Rf54 (110 mg, 0.137 mmol) obtained in Reference Example 8 were dissolved in methanol (2.0 mL). To the solution, a solution of tris[(1-benzyl-1H-1,2,3-triazol-4-yl)methyl]amine (TBTA) (12.0 mg, 0.023 mmol), sodium ascorbate (4.50 mg, 0.023 mmol), and copper(II) sulfate (1.80 mg, 0.011 mmol) in dimethyl sulfoxide/water = 1/1 (2.0 mL) was added, and the mixture was stirred at room temperature for 15 minutes after hermetical sealing. Methanol (2 mL), water (1 mL), and dimethyl sulfoxide (1 mL) were further added thereto, and the mixture was stirred at room temperature for 1 hour and 15 minutes while insoluble matter was suspended by ultrasonic vibration performed once per 10 minutes. The reaction mixture was quenched by the addition of water (50 mL), followed by extraction with methylene chloride twice (80 mL x 2). The organic layer was dried over anhydrous sodium sulfate. The residue was concentrated under reduced pressure to obtain a crude product (548 mg). The obtained crude product was purified by column chromatography (Yamazen Corp., Hi-Flash Column M, developing solvent: chloroform (1% TEA)/methanol = 100/0 -> 85/15). Then, the product obtained in the column was dissolved in acetonitrile and freeze-dried to obtain 4-(3-((3-(bis(4-methoxyphenyl)(phenyl)methoxy)-2-((4-(4-(1-(((3S,8S,9S,10R,13R,14S,17R)-10,13-dimethyl-17-((R)-6-methylheptan-2-yl)-2,3,4,7,8,9,10,11,12,13,14,15,16,17-tetradecahydro-1H-cyclopenta[a]phenanthren-3-yl)oxy)-1-oxo-5,8,11,14-tetraoxa-2-azapentadecan-15-yl)-1H-1,2,3-triazol-1-yl)butoxy)methyl)-2-methylpropyl)disulfanyl)propoxy)-4-oxobutanoic acid Rf58 (144 mg, 98.0 mmol) as a colorless viscous liquid containing 3.8 wt% (0.55 eq.) triethylamine and 3.2 wt% dimethyl sulfoxide (yield: 86%).

ESI-MS (m/z): undetected.

¹H-NMR (CDCl₃, 400 MHz) δ (ppm): 7.57 (s, 1H), 7.42-7.40 (m, 2H), 7.31-7.17 (m, 7H), 6.83-6.79 (m, 4H), 5.36 (d, J = 5.2 Hz, 1H), 5.25 (br, 1H), 4.69 (s, 2H), 4.49 (br, 1H), 4.15 (t, J = 6.4 Hz, 2H), 3.79 (s, 6H), 3.71-3.59 (m, 12H), 3.55-3.53 (m, 2H), 3.40-3.36 (m, 8H), 3.03-2.86 (m, 4H), 2.67 (t, J = 7.2 Hz, 2H), 2.60 (s, 4H), 2.36-2.23 (m, 2H), 2.02-1.78 (m, 9H), 1.60-0.852 (m, 24H), 0.997 (s, 6H), 0.911 (d, J = 6.4 Hz, 3H), 0.863 (dd, J = 2.0, 6.8 Hz, 6H), 0.672 (s,3H).

### Step 39:

Modified CPG Rf59 (1.05 g) was obtained in the same way as in step 9 of Reference Example 2 using compound Rf58 (64 mg, 44 µmοl) obtained in step 38 and CPG resin (LCAA Controlled Pore Glass, 1.10 g). A portion (8.67 mg) of the obtained CPG resin was treated with a solution of 2 wt% trichloroacetic acid in methylene chloride (25 mL), followed by the measurement of absorbance (absorbance (503 nm) = 1.065, loading 40.4 µmol/g).

### Reference Example 10

### Synthesis of dipeptide amidite Rf65

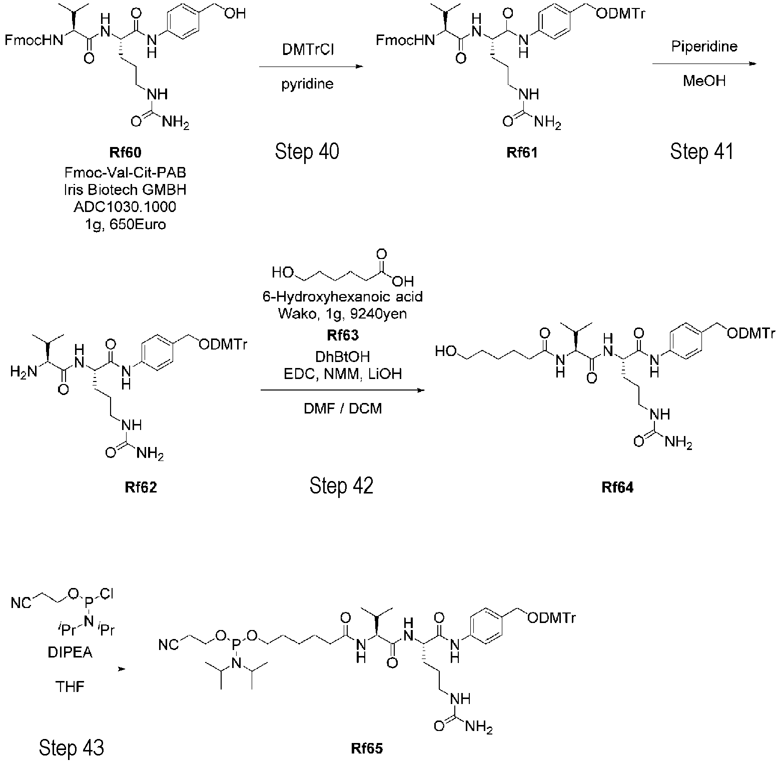

Dipeptide amidite Rf65 can be synthesized by step 40 according to the same synthesis method as in step 1 of Reference Example 1, step 41 according to a method commonly used in organic synthetic chemistry [e.g., a method described in Protective Groups in Organic Synthesis, third edition, T.W. Greene, John Wiley & Sons Inc. (1999)], step 42 according to the method described in Journal of Medicinal Chemistry, Vol. 48, p. 6229-6235, 2005 or a synthesis method equivalent thereto, and step 43 according to the same synthesis method as in step 2 of Reference Example 1 using commercially available Fmoc-Val-Cit-PAB [(9H-fluoren-9-yl)methyl ((S)-1-(((S)-1-((4-(hydroxymethyl)phenyl)amino)-1-oxo-5-ureidopentan-2-yl)amino)-3-methyl-1-oxobutan-2-yl)carbamate] Rf60.

### Example 48

### Synthesis of linear siRNA (compound 48)

Compound Ic obtained in step 2 of Example 1 and oligonucleotide HPRT1_asRNA1 separately prepared using a nucleic acid synthesis apparatus were mixed in equal amounts, dissolved in a citrate buffer solution, and then left standing at 85°C for 5 minutes. Then, the temperature was gradually lowered to obtain compound 48.

### Example 49

### Synthesis of linear siRNA (compound 49)

Compound 49 was obtained in the same way as in Example 48 using compound 2c obtained in step 2 of Example 2 and oligonucleotide HPRT1_asRNA1 separately prepared using a nucleic acid synthesis apparatus.

### Example 50

### Synthesis of linear siRNA (compound 50)

Compound 50 was obtained in the same way as in Example 48 using compound 3c obtained in step 2 of Example 3 and oligonucleotide B2M_asRNA1 separately prepared using a nucleic acid synthesis apparatus.

### Example 51

### Synthesis of false linker-attached linear siRNA (compound 51)

Compound 51 was obtained in the same way as in Example 48 using compound 6c obtained in step 2 of Example 6 and oligonucleotide HPRT1_asRNA5 separately prepared using a nucleic acid synthesis apparatus.

### Example 52

### Synthesis of linear siRNA (compound 52)

Compound 52 was obtained in the same way as in Example 48 using compound 7c obtained in step 2 of Example 7 and oligonucleotide PTEN_asRNA2 separately prepared using a nucleic acid synthesis apparatus.

### Example 53

### Synthesis of linear siRNA (compound 53)

Compound 53 was obtained in the same way as in Example 48 using compound 8c obtained in step 2 of Example 8 and oligonucleotide Factor9_asRNA2 separately prepared using a nucleic acid synthesis apparatus.

The base sequences of the oligonucleotide derivatives of Examples 48 to 53 and a negative control group, and the molecular weights thereof are shown in Tables 20 to 23. For each Example, the upper column shows the sense strand, and the lower column shows the antisense strand.

The abbreviations, etc. in Tables 20 to 23 are as defined below.
C6CSSC6 = CH₃-(CH₂)₅-S-S-(CH₂)₆-
C3SSC3 = CH₃-(CH₂)₂-S-S-(CH₂)₃-
p = Phosphorylated
^ = Phosphorothioate-modified
m = 2'-OMe-modified
f = 2'-F-modified

**[Table 20]**

| | Name | Sequence (5' → 3') | MS (calcd) | MS (found) |
|---|---|---|---|---|
| Negative control group 1 | HPRTI_dsRNA 1 | fA^mA^fGmCfCmAfGmAfCmUfUmUfGmUfUmGfGmAfUmUfU | 6868 | 6868 |
| | HPRT1_ssRNA1/HPRT1_asRNA1 | mA^fA^mAfUmCfCmAfAmCfAmAfAmGfUmCfUmGfGmC^fu^mU | 6901 | 6903 |
| Example 48 | Compound 48 | C6SSC6^fA^mU^fAmUfAmAfGmCfCmAfGmAfCmUfUmUfGmUfUmGfGmAfUmUfU^C3SSC3 | 8775 | 8778 |
| | HPRT1_ssRNA5/HPRT1_asRNA1 | mA^fA^mAfUmCfCmAfAmCfAmAfAmGfUmCfUmGfGmC^fU^mU | 6901 | 6903 |
| Example 49 | Compound 49 | C6SSC6^fG^mG^fAmUfAmUfAmAfGmCfCmAfGmAfCmUfUmUfGmUfUmGfGmAfUmUFU^C3SSC3 | 9481 | 9485 |
| | HPRT1_ssRNA6/HPRT1_asRNA1 | mA^fA^mAfUmCfCmAfAmCfAmAfAmGfUmCfUmGfUmC^fU^mU | 6901 | 6903 |

**[Table 21]**

| | Name | Sequence (5' → 3') | MS (calcd) | MS (found) |
|---|---|---|---|---|
| Negative control group 3 | B2M_dsRNA | fAmGfGmAfCmUfGmGfUmCfUfUmUfCmUfAmUfAmU^fC^mU | 6804 | 6806 |
| | B2M_ssRNA1/B2M_asRNA1 | fA^mG^fAmUfAmUfAmGfAmAmAfGmAfCmCfAmGfUmC^fC^mU | 6964 | 6965 |
| Example 50 | Compound 50 | C6SSC6^fAmGfCmAfAmGfGmAfCmUfGmGfUmCfUfUmUfCmUfAmUfAmU^fC^mUC3SSC3 | 8750 | 8753 |
| | B2M_ssRNA2/B2M_asRNA1 | fA^mG^fAmUfAmUfAmGfAmAmAfGmAfCmCfAmGfUmC^fC^mU | 6964 | 6965 |

**[Table 22]**

| | Name | Sequence (5' → 3') | MS (calcd) | MS (found) |
|---|---|---|---|---|
| Negative control group 6 | HPRT1_dsRNA5 | fU^mC^fCmUfAmUfGmAfCmUfGmUfAmGfAmUfUmUfU^mA^fU | 6823 | 6823 |
| | HPRT1_ssRNA3/HPRT1_asRNA5 | pmA^fU^mAfAmAfAmUfCmUfAmCfAmGfUmCfAmUfAmG^fG^mA | 7029 | 7030 |
| Example 51 | Compound 51 | C6SSC6^fA^mU^fCmCfAmUfUmCfCmUfAmUfGmAfCmUfGmUfAmGfAmUfUmUfU^mA^fU^C3SSC3 | 9357 | 9356 |
| | HPRT1_ssRNA7/HPRT1_asRNA5 | pmA^fU^mAfAmAfAmUfCmUfAmCfAmGfUmCfAmUfAmG^fG^mA | 7029 | 7030 |

**[Table 23]**

| | Name | Sequence (5' → 3') | MS (calcd) | MS (found) |
|---|---|---|---|---|
| Negative control group 7 | PTEN_dsRNA1 | fA^mC^fCmUfGmAfUmCfAmUfUmAfUmAfGmAfGmAfU^mA^fA | 6224 | 6226 |
| | PTEN_ssRNA1/PTEN_asRNA1 | pmU^fU^mAfUmCfUmAfUmAfAmUfGmAfUmCfAmGfGmU6fA^mA | 6984 | 6987 |
| Example 52 | Compound 52 | C6SSC6^fG^mA^fCmAfAmUfGmAfAmCfCmUfGmAfUmCfAmUfUmAfUmAfGmAfU^mA^fA^C3SSC3 | 9513 | 9514 |
| | PTEN_ssRNA2/PTEN_asRNA2 | pmU^fU^mAfUmCfUmAfUmAfAmUfGmAfUmCfAmGfGmU^fU^mC | 6939 | 6940 |
| Negative control group 8 | Factor9_dsRNA1 | fU^mG^fGmAfAmGfCmAfGfUfAmUfGmUfUmGfAmUfGmGfA | 6998 | 7000 |
| | Factor9_ssRNA1/Factor9_asRNA1 | pmU^fC^mCfAmUfCmAfAmCfAmUmAmCfUmGfCmUfUmCfCmA^mA^mA | 7552 | 7556 |
| Example 53 | Compound 53 | C6SSC6^fG^mA^fAmUfUmUfUmGfGmAfAmGfCmAfGfUfAmUfGmUfUmGfAmUfGmGfA^C3SSC3 | 9574 | 9577 |
| | Factor9_ssRNA2/Factor9_asRNA2 | pmU^fC^mCfAmUfCmAfAmCfAmUmAmCfUmGfCmUfUmC^fC^mA | 6868 | 6867 |

Test Example 1: mRNA knock-down in mouse primary hepatocyte by circular siRNA targeting hypoxanthine-guanine phosphoribosyltransferase 1 (HPRT1)

To 500 mL of William's E Medium (no phenol red, manufactured by Life Technologies Corp., A1217601), an additive (Primary Hepatocyte Thawing and Plating Supplements, manufactured by Thermo Fisher Scientific Inc., CM3000) was added to prepare a medium for mouse primary hepatocyte inoculation. Also, to 500 mL of Williams' Medium E (no phenol red), an additive (Hepatocyte Maintenance Supplements, manufactured by Thermo Fisher Scientific Inc., CM4000) was added to prepare a medium for mouse primary hepatocyte incubation.

Frozen commercially available mouse primary hepatocytes (Mouse (CD-1) Cryopreserved Hepatocytes, Plateable Male, manufactured by Invitrogen Corp., MSCP10) were thawed in a hot water bath of 37°C and then suspended in 10 mL of the medium for inoculation. The suspended cells were centrifuged. After removal of the supernatant, the resulting cells were diluted into 1.25 × 10⁵ cells/mL with a medium.

The test samples used were compound 1 and compound 2, and HPRT1_dsRNA1 and HPRT1_dsRNA2 were established as comparative controls. The test was conducted with four final concentrations of 1 µmol/L, 0.3 µmol/L, 0.1 µmol/L and 0.03 µmol/L at N = 3.

A nucleic acid complex solution was diluted by the following procedures: the nucleic acid complex solution was diluted with Opti-MEM(Registered Trademark) I Reduced Serum Medium (manufactured by Life Technologies Corp., 31985-070) and a citrate buffer solution (20 mM citrate (pH 7) and 150 mM NaCl). The diluted nucleic acid complex solution was added at 20 µL/well to a collagen I-coated 96-well flat-bottomed plate (manufactured by Becton, Dickinson and Company, 356407), and a nucleic acid-free diluted solution was added at 20 µL/well for a negative control group. The cell lysate was added at 80 µL/well to the plate, cultured at 37°C for 24 hours under 5% CO₂ conditions, and then subjected to RNA extraction.

A cell lysate containing RNA was prepared using SuperPrep(Registered Trademark) Cell Lysis & RT Kit for qPCR (manufactured by Toyobo Co., Ltd., SCQ-101), and cDNA was prepared through reverse transcription reaction according to the instruction attached to the kit using RT Kit for qPCR included in the kit.

This cDNA was used as a PCR reaction template in the PCR reaction of HPRT1 gene or Actin β (hereinafter, referred to as ACTB) gene as a control by the TaqMan probe method using QuantStudio 12K Flex real-time PCR system (manufactured by Applied Biosystems, Inc.). The amplified mRNA level of each gene was measured, and the semiquantitative value of HPRT1 mRNA was calculated with the amplified ACTB mRNA level as an internal control. Also, each of the amplified HPRT1 and ACTB mRNA levels in the negative control group was similarly measured, and the semiquantitative value of HPRT1 mRNA was calculated.

The HPRT1 gene was assayed using TaqMan probe Mm0154399_m1 (manufactured by Applied Biosystems, Inc.). The ACTB gene was assayed using Mm00607939_s1 (manufactured by Applied Biosystems, Inc.). The reaction was carried out using TaqMan Gene Expression Master Mix (manufactured by Applied Biosystems, Inc., 4369542) as a reaction reagent according to the attached protocol.

The target mRNA level of the siRNA-introduced specimen was calculated as a relative ratio when the HPRT1 mRNA level of the negative control group (siRNA-unintroduced group) was defined as 1. The results about the relative ratio of the mRNA level indicated by mean ± standard deviation are shown in Figure 1.

From these results, the test sample (compound 1 and compound 2) was confirmed to exhibit a stronger knock-down effect than that of the comparative control (HPRT1_dsRNA1 and HPRT1_dsRNA2).

### Test Example 2: mRNA knock-down in mouse primary hepatocyte by circular siRNA targeting beta 2-macroglobulin (B2M)

To 500 mL of William's E Medium (no phenol red, manufactured by Life Technologies Corp., A1217601), an additive (Primary Hepatocyte Thawing and Plating Supplements, manufactured by Thermo Fisher Scientific Inc., CM3000) was added to prepare a medium for mouse primary hepatocyte inoculation. Also, to 500 mL of Williams' E Medium, an additive (Hepatocyte Maintenance Supplements, manufactured by Thermo Fisher Scientific Inc., CM4000) was added to prepare a medium for mouse primary hepatocyte incubation.

Frozen commercially available mouse primary hepatocytes (Mouse (CD-1) Cryopreserved Hepatocytes, Plateable Male, manufactured by Invitrogen Corp., MSCP10) were thawed in a hot oil bath of 37°C and then suspended in 10 mL of the medium for inoculation. The suspended cells were centrifuged. After removal of the supernatant, the resulting cells were diluted into 1.25 × 10⁵ cells/mL with a medium.

The test sample used was compound 3, and B2M_dsRNA was established as a comparative control. The test was conducted with four final concentrations of 1 µmol/L, 0.3 µmol/L, 0.1 µmol/L and 0.03 µmol/L at N = 3.

A nucleic acid complex solution was diluted by the following procedures: the nucleic acid complex solution was diluted with Opti-MEM(Registered Trademark) I Reduced Serum Medium (manufactured by Life Technologies Corp., 31985-070) and a citrate buffer solution (20 mM citrate (pH 7) and 150 mM NaCl). The diluted nucleic acid complex solution was added at 20 µL/well to a collagen I-coated 96-well flat-bottomed plate (manufactured by Becton, Dickinson and Company, 356407), and a nucleic acid-free diluted solution was added at 20 µL/well for a negative control group. The cell lysate was added at 80 µL/well to the plate and cultured at 37°C for 6 hours under 5% CO₂ conditions. Then, the supernatant was removed, and the medium for incubation was added at 100 µL/well. The cell lysate was cultured at 37°C for 18 hours under 5% CO₂ conditions and then subjected to RNA extraction.

A cell lysate containing RNA was prepared using SuperPrep (Registered Trademark) Cell Lysis & RT Kit for qPCR (manufactured by Toyobo Co., Ltd., SCQ-101), and cDNA was prepared through reverse transcription reaction according to the instruction attached to the kit using RT Kit for qPCR included in the kit.

This cDNA was used as a PCR reaction template in the PCR reaction of B2M gene or Actin β (hereinafter, referred to as ACTB) gene as a control by the TaqMan probe method using QuantStudio 12K Flex real-time PCR system (manufactured by Applied Biosystems, Inc.). The amplified mRNA level of each gene was measured, and the semiquantitative value of B2M mRNA was calculated with the amplified ACTB mRNA level as an internal control. Also, each of the amplified B2M and ACTB mRNA levels in the negative control group was similarly measured, and the semiquantitative value of B2M mRNA was calculated.

The B2M gene was assayed using TaqMan probe Mm00437762_m1 (manufactured by Applied Biosystems, Inc.). The ACTB gene was assayed using Mm00607939_s1 (manufactured by Applied Biosystems, Inc.). The reaction was carried out using TaqMan Gene Expression Master Mix (manufactured by Applied Biosystems, Inc., 4369542) as a reaction reagent according to the attached protocol.

The target mRNA level of the siRNA-introduced specimen was calculated as a relative ratio when the B2M mRNA level of the negative control group (siRNA-unintroduced group) was defined as 1. The results about the relative ratio of the mRNA level indicated by mean ± standard deviation are shown in Figure 2.

From these results, the test sample compound 3 was confirmed to exhibit a stronger knock-down effect than that of the comparative control B2M_dsRNA.

### Test Example 3: mRNA knock-down in mouse primary hepatocyte by circular siRNA targeting hypoxanthine-guanine phosphoribosyltransferase 1 (HPRT1)

To 500 mL of William's E Medium (no phenol red, manufactured by Life Technologies Corp., A1217601), an additive (Primary Hepatocyte Thawing and Plating Supplements, manufactured by Thermo Fisher Scientific Inc., CM3000) was added to prepare a medium for mouse primary hepatocyte inoculation. Also, to 500 mL of Williams' E Medium, an additive (Hepatocyte Maintenance Supplements, manufactured by Thermo Fisher Scientific Inc., CM4000) was added to prepare a medium for mouse primary hepatocyte incubation.

Frozen commercially available mouse primary hepatocytes (Mouse (CD-1) Cryopreserved Hepatocytes, Plateable Male, manufactured by Invitrogen Corp., MSCP10) were thawed in a hot oil bath of 37°C and then suspended in 10 mL of the medium for inoculation. The suspended cells were centrifuged. After removal of the supernatant, the resulting cells were diluted into 1.25 × 10⁵ cells/mL with a medium.

The test samples used were compound 4 and compound 5, and HPRT1_dsRNA3 and HPRT1_dsRNA4 were established as comparative controls. The test was conducted with four final concentrations of 1 µmol/L, 0.3 µmol/L, 0.1 µmol/L and 0.03 µmol/L at N = 3.

A nucleic acid complex solution was diluted by the following procedures: the nucleic acid complex solution was diluted with Opti-MEM(Registered Trademark) I Reduced Serum Medium (manufactured by Life Technologies Corp., 31985-070) and a citrate buffer solution (20 mM citrate (pH 7) and 150 mM NaCl). The diluted nucleic acid complex solution was added at 20 µL/well to a collagen I-coated 96-well flat-bottomed plate (manufactured by Becton, Dickinson and Company, 356407), and a nucleic acid-free diluted solution was added at 20 µL/well for a negative control group. The cell lysate was added at 80 µL/well to the plate and cultured at 37°C for 6 hours under 5% CO₂ conditions. Then, the supernatant was removed, and the medium for incubation was added at 100 µL/well. The cell lysate was cultured at 37°C for 18 hours under 5% CO₂ conditions and then subjected to RNA extraction.

A cell lysate containing RNA was prepared using SuperPrep(Registered Trademark) Cell Lysis & RT Kit for qPCR (manufactured by Toyobo Co., Ltd., SCQ-101), and cDNA was prepared through reverse transcription reaction according to the instruction attached to the kit using RT Kit for qPCR included in the kit.

This cDNA was used as a PCR reaction template in the PCR reaction of HPRT1 gene or Actin β (hereinafter, referred to as ACTB) gene as a control by the TaqMan probe method using QuantStudio 12K Flex real-time PCR system (manufactured by Applied Biosystems, Inc.). The amplified mRNA level of each gene was measured, and the semiquantitative value of HPRT1 mRNA was calculated with the amplified ACTB mRNA level as an internal control. Also, each of the amplified HPRT1 and ACTB mRNA levels in the negative control group was similarly measured, and the semiquantitative value of HPRT1 mRNA was calculated.

The HPRT1 gene was assayed using TaqMan probe Mm0154399_m1 (manufactured by Applied Biosystems, Inc.). The ACTB gene was assayed using Mm00607939_s1 (manufactured by Applied Biosystems, Inc.). The reaction was carried out using TaqMan Gene Expression Master Mix (manufactured by Applied Biosystems, Inc., 4369542) as a reaction reagent according to the attached protocol.

The target mRNA level of the siRNA-introduced specimen was calculated as a relative ratio when the HPRT1 mRNA level of the negative control group (siRNA-unintroduced group) was defined as 1. The results about the relative ratio of the mRNA level indicated by mean ± standard deviation are shown in Figure 3.

From these results, the test sample (compound 3 and compound 4) was confirmed to exhibit a stronger knock-down effect than that of the comparative control (HPRT1_dsRNA3 and HPRT1_dsRNA4).

### Test Example 4: mRNA knock-down in mouse primary hepatocyte by circular siRNA targeting hypoxanthine-guanine phosphoribosyltransferase 1 (HPRT1)

The test was carried out by the same approach as in Test Example 3 except that the test sample was changed to compound 6, and the comparative control was changed to HPRT1_dsRNA5. The test results are shown in Figure 4.

From these results, the test sample (compound 6) was confirmed to exhibit a stronger knock-down effect than that of the comparative control (HPRT1_dsRNA5).

### Test Example 5: mRNA knock-down in HeLa cell by circular siRNA targeting hypoxanthine-guanine phosphoribosyltransferase 1 (HPRT1)

HeLa cells were suspended in RPMI1640 Medium (Life Technologies Corp., A10491-01) containing 10% fetal bovine serum. The cell suspension was inoculated at 100 µL (2000 to 3000 cells) per well to a culture plate (Nunc 96 Microwell Plate, 167008) and cultured at 37°C for 24 hours under 5% CO₂ conditions.

The test sample used was compound 6, and HPRT1_dsRNA5 was established as a comparative control. The test was conducted with four final concentrations of 1 µmol/L, 0.3 µmol/L, 0.1 µmol/L and 0.03 µmol/L at N = 3. A nucleic acid complex solution was diluted by the following procedures: the nucleic acid complex solution was diluted with Opti-MEM(Registered Trademark) I Reduced Serum Medium (manufactured by Life Technologies Corp., 31985-070) and a citrate buffer solution (20 mM citrate (pH 7) and 150 mM NaCl).

The supernatant was removed from the plate where the cells were cultured, and a serum-containing medium was added at 80 µL/well to the plate. Further, the nucleic acid complex solution was added thereto at 20 µL/well, and a nucleic acid-free diluted solution was added at 20 µL/well for a negative control group. Then, the cells were cultured at 37°C for 96 hours under 5% CO₂ conditions.

RNA recovery and cDNA preparation were carried out by the same approaches as in Test Example 1. The obtained cDNA was used as a PCR reaction template in the PCR reaction of HPRT1 gene or Actin β (hereinafter, referred to as ACTB) gene as a control by the TaqMan probe method using QuantStudio 12K Flex real-time PCR system (manufactured by Applied Biosystems, Inc.). The amplified mRNA level of each gene was measured, and the semiquantitative value of HPRT1 mRNA was calculated with the amplified ACTB mRNA level as an internal control. Also, each of the amplified HPRT1 and ACTB mRNA levels in the negative control group was similarly measured, and the semiquantitative value of HPRT1 mRNA was calculated.

The HPRT1 gene was assayed using TaqMan probe Hs02800695_m1 (manufactured by Applied Biosystems, Inc.). The ACTB gene was assayed using Hs01060665_g1 (manufactured by Applied Biosystems, Inc.). The reaction was carried out using TaqMan Gene Expression Master Mix (manufactured by Applied Biosystems, Inc., 4369542) as a reaction reagent according to the attached protocol.

The target mRNA level of the siRNA-introduced specimen was calculated as a relative ratio when the HPRT1 mRNA level of the negative control group (siRNA-unintroduced group) was defined as 1. The results about the relative ratio of the mRNA level indicated by mean ± standard deviation are shown in Figure 5.

From these results, the test sample (compound 6) was confirmed to exhibit a stronger knock-down effect than that of the comparative control (HPRT1_dsRNA5).

### Test Example 6: mRNA knock-down in human liver cancer-derived cell HepG2 by circular siRNA targeting hypoxanthine-guanine phosphoribosyltransferase 1 (HPRT1)

HepG2 cells were suspended in MEM (Gibco, 11095-080) containing 10% fetal bovine serum. The cell suspension was inoculated at 100 µL (2000 to 3000 cells) per well to a culture plate (Nunc 96 Microwell Plate, 167008) and cultured at 37°C for 24 hours under 5% CO₂ conditions.

Nucleic acid addition, RNA recovery and cDNA preparation were carried out by the same approaches as in Test Example 5. The obtained cDNA was used as a PCR reaction template in the PCR reaction of HPRT1 gene or GAPDH gene as a control by the TaqMan probe method using QuantStudio 12K Flex real-time PCR system (manufactured by Applied Biosystems, Inc.). The amplified mRNA level of each gene was measured, and the semiquantitative value of HPRT1 mRNA was calculated with the amplified GAPDH mRNA level as an internal control. Also, each of the amplified HPRT1 and GAPDH mRNA levels in the negative control group was similarly measured, and the semiquantitative value of HPRT1 mRNA was calculated.

The HPRT1 gene was assayed using TaqMan probe Hs02800695_m1 (manufactured by Applied Biosystems, Inc.). The GAPDH gene was assayed using Hs02758991_g1 (manufactured by Applied Biosystems, Inc.). The reaction was carried out using TaqMan Gene Expression Master Mix (manufactured by Applied Biosystems, Inc., 4369542) as a reaction reagent according to the attached protocol.

The target mRNA level of the siRNA-introduced specimen was calculated as a relative ratio when the HPRT1 mRNA level of the negative control group (siRNA-unintroduced group) was defined as 1. The results about the relative ratio of the mRNA level indicated by mean ± standard deviation are shown in Figure 6.

From these results, the test sample (compound 6) was confirmed to exhibit a stronger knock-down effect than that of the comparative control (HPRT1_dsRNA5).

### Test Example 7: mRNA knock-down in human liver cancer-derived cell HuH-7 by circular siRNA targeting hypoxanthine-guanine phosphoribosyltransferase 1 (HPRT1)

HuH-7 cells were suspended in DMEM (High-Glc) (Nacalai Tesque, Inc., 08458-16) containing 10% fetal bovine serum. The cell suspension was inoculated at 100 µL (2000 to 3000 cells) per well to a culture plate (Nunc 96 Microwell Plate, 167008) and cultured at 37°C for 24 hours under 5% CO₂ conditions.

Nucleic acid addition, RNA recovery, cDNA preparation and PCR reaction were carried out by the same approaches as in Test Example 6. The test results are shown in Figure 7.

From these results, the test sample (compound 6) was confirmed to exhibit a stronger knock-down effect than that of the comparative control (HPRT1_dsRNA5).

### Test Example 8: mRNA knock-down in mouse macrophage-like cell RAW264.7 by circular siRNA targeting hypoxanthine-guanine phosphoribosyltransferase 1 (HPRT1)

RAW264.7 cells were suspended in RPMI1640 Medium (Life Technologies Corp., A10491-01) containing 10% fetal bovine serum. The cell suspension was inoculated at 100 µL (2000 to 3000 cells) per well to a culture plate (Nunc 96 Microwell Plate, 167008) and cultured at 37°C for 24 hours under 5% CO₂ conditions.

Nucleic acid addition, RNA recovery and cDNA preparation were carried out by the same approaches as in Test Example 6. The obtained cDNA was used as a PCR reaction template in the PCR reaction of HPRT1 gene or B2M gene as a control by the TaqMan probe method using QuantStudio 12K Flex real-time PCR system (manufactured by Applied Biosystems, Inc.). The amplified mRNA level of each gene was measured, and the semiquantitative value of HPRT1 mRNA was calculated with the amplified B2M mRNA level as an internal control. Also, each of the amplified HPRT1 and B2M mRNA levels in the negative control group was similarly measured, and the semiquantitative value of HPRT1 mRNA was calculated.

The HPRT1 gene was assayed using TaqMan probe Mm01545399_m1 (manufactured by Applied Biosystems, Inc.). The B2M gene was assayed using Mm00437762_m1 (manufactured by Applied Biosystems, Inc.). The reaction was carried out using TaqMan Gene Expression Master Mix (manufactured by Applied Biosystems, Inc., 4369542) as a reaction reagent according to the attached protocol.

The target mRNA level of the siRNA-introduced specimen was calculated as a relative ratio when the HPRT1 mRNA level of the negative control group (siRNA-unintroduced group) was defined as 1. The results about the relative ratio of the mRNA level indicated by mean ± standard deviation are shown in Figure 8.

From these results, the test sample (compound 6) was confirmed to exhibit a stronger knock-down effect than that of the comparative control (HPRT1_dsRNA5).

### Test Example 9: Evaluation on stability of nucleic acid in serum

A nucleic acid solution adjusted to 100 µM with a citrate buffer solution (20 mM citrate (pH 7) and 150 mM NaCl) and rat serum (Cedarlane Corp., CL7000-50) were mixed at a ratio of 1:9 and left standing at 37°C for a predetermined time.

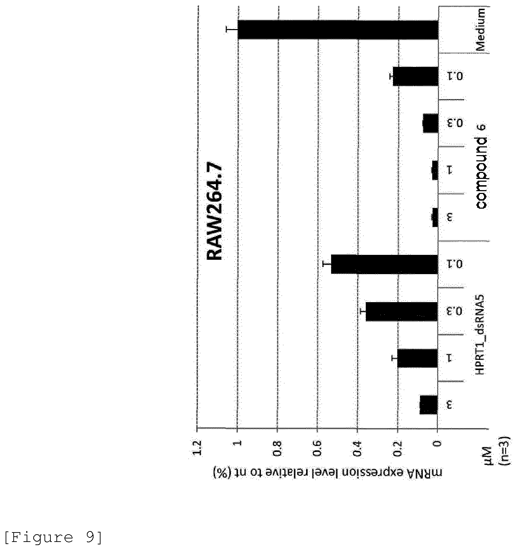

From the obtained sample, cDNA complementary to the antisense strand of siRNA was prepared (RT primer sequence: GTCGTATCCAGTGCAGGGTCCGAGGTATTCGCACTGGATACGACTCCTATGTCTG; SEQ ID NO: 71) using TaqMan MicroRNA Reverse Transcription Kit (Life Technologies Corp., 4366596) according to the attached protocol. This cDNA was used as a template in PCR reaction by the TaqMan probe method using QuantStudio 12K Flex real-time PCR system (manufactured by Applied Biosystems, Inc.) to calculate the amount of the siRNA antisense strand remaining in serum (forward primer sequence: CGCGCGCGATAAAATCTACAG; SEQ ID NO: 72, reverse primer sequence: GTGCAGGGTCCGAGGT; SEQ ID NO: 73, TaqMan probe sequence: CTGGATACGACTCCTA: SEQ ID NO: 74). The results about a relative ratio of the residual rate after still standing for the predetermined time, indicated by mean ± standard deviation are shown in Figure 9 when the concentration at the start of interaction with serum is defined as 100%.

The residual rate of the antisense strand after interaction with serum for 28 days was larger for the test sample compound 6 than for the control sample HPRT1_dsRNA5, confirming that the stability in serum was improved.

### Test Example 10: Evaluation on stability of nucleic acid in nucleolytic enzyme

Nucleolytic enzyme (Phosphodiesterase I from Crotalus adamanteus venom, Sigma-Aldrich Co. LLC, P3243-1VL) was dissolved in a buffer for reaction (50 mM Tris/HCl, pH 7.5, and 8 mM MgCl₂) and thereby adjusted to 1.2 U/mL. A nucleic acid solution adjusted to 20 µM with a citrate buffer solution (20 mM citrate (pH 7) and 150 mM NaCl) and the nucleolytic enzyme solution diluted 100-fold with the buffer for reaction were mixed at a ratio of 1:9 and left standing at 37°C for a predetermined time. Then, the reaction was terminated by heating at 95°C for 10 minutes.

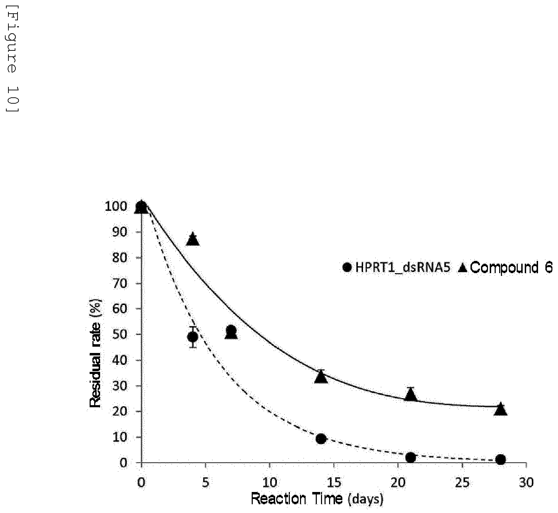

cDNA preparation and PCR reaction were carried out by the same approaches as in Test Example 9. The results about a relative ratio of the residual rate of the antisense strand after still standing for the predetermined time, indicated by mean ± standard deviation are shown in Figure 10 when the concentration at the start of interaction with nucleolytic enzyme is defined as 100%.

The residual rate of the antisense strand after interaction with nucleolytic enzyme for 24 hours was larger for the test sample compound 6 than for the control sample linear oligo HPRT1_dsRNA5, confirming that the stability in nucleolytic enzyme was high.

### Test Example 11: mRNA knock-down in mouse primary hepatocyte by circular siRNAs targeting phosphatase and tensin homolog deleted from chromosome 10 (PTEN) and Factor 9

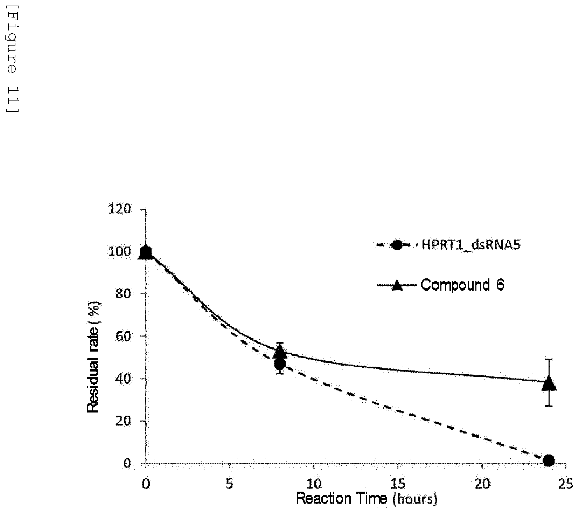

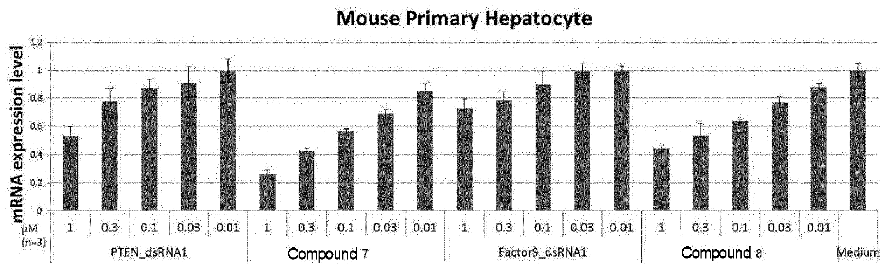

The test was carried out by the same approach as in Test Example 3 except that the test samples were changed to compound 7 and compound 8, and the negative control groups were changed to PTEN_dsRNA1 and Factor9_dsRNA1. The test results are shown in Figure 11.

### Test Example 12: mRNA knock-down in HeLa cell by circular siRNA targeting hypoxanthine-guanine phosphoribosyltransferase 1 (HPRT1)

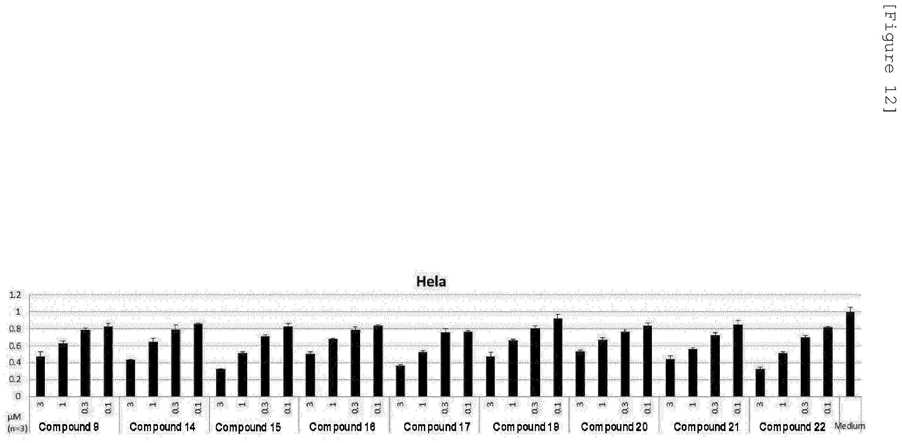

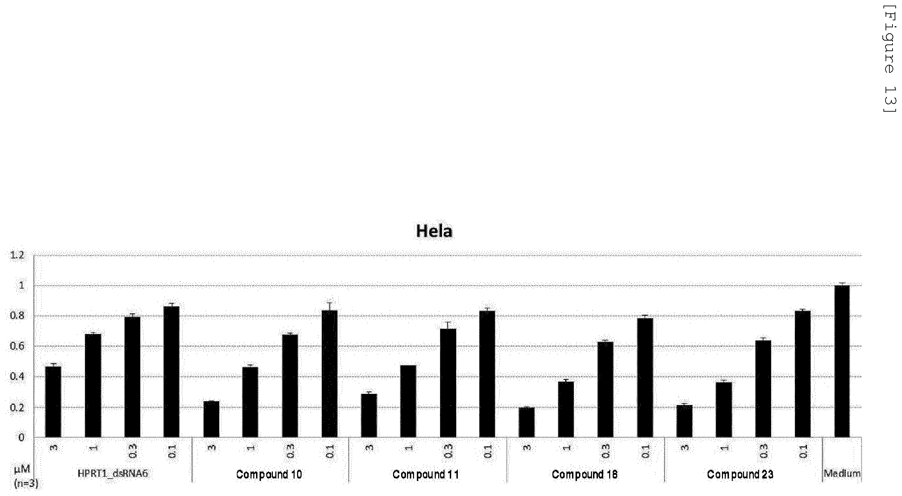

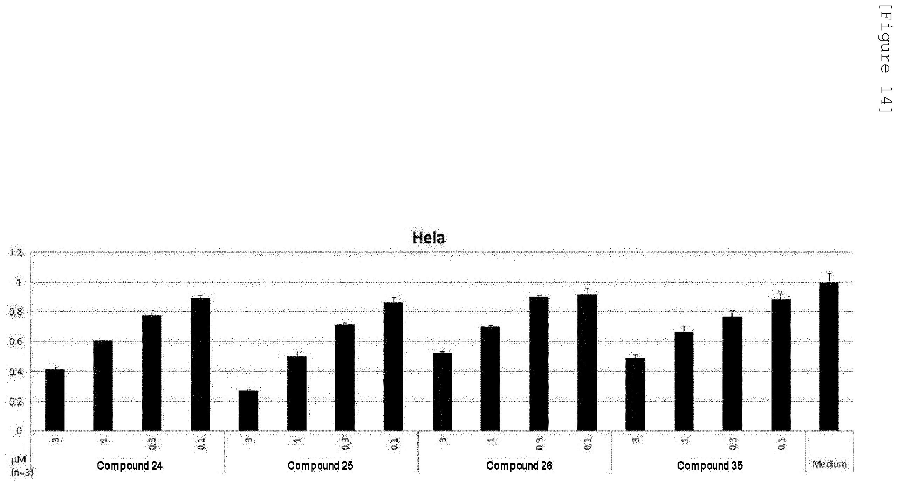

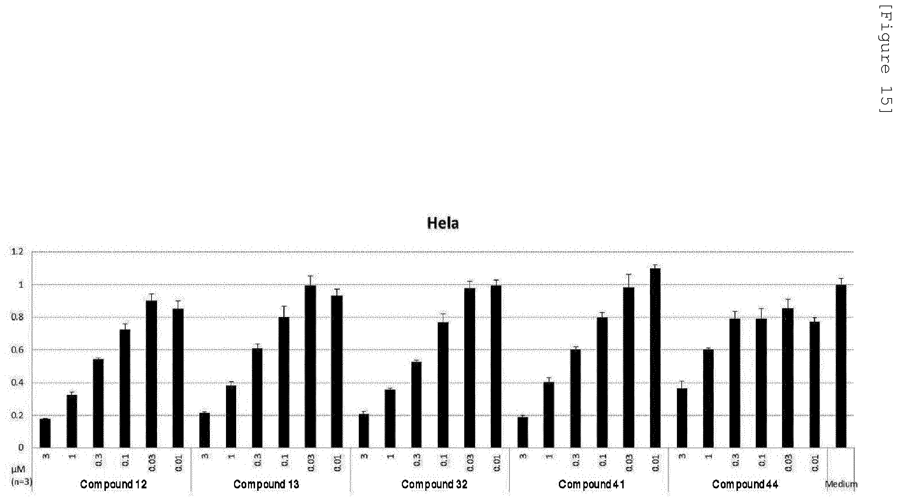

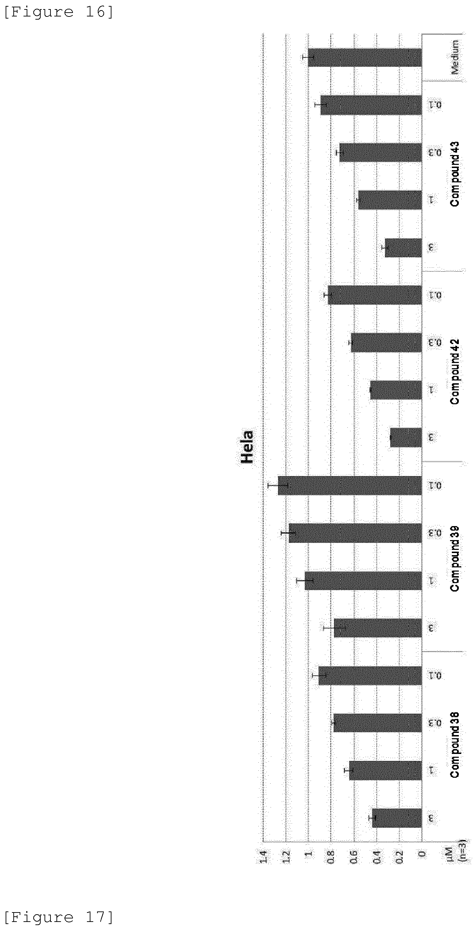

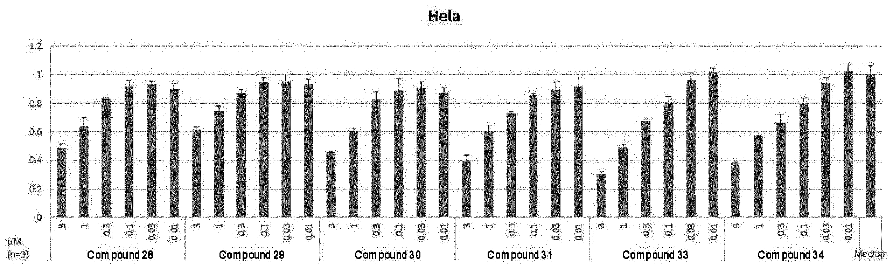

The test was carried out by the same approach as in Test Example 5 except that the test samples were changed to compounds 9 to 26, 28 to 35, 38, 39, 41 to 44, and 47. The test results are shown in Figures 12 to 18.

Test Example 13: mRNA knock-down in mouse primary hepatocyte by compounds 48 and 49 targeting hypoxanthine-guanine phosphoribosyltransferase 1 (HPRT1)

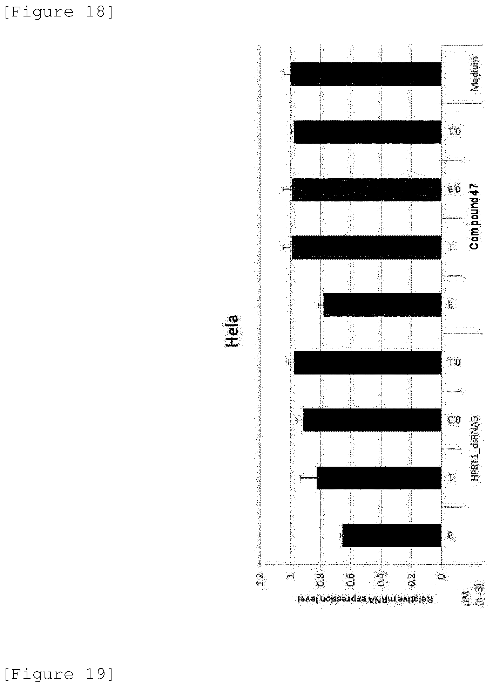

The test was carried out by the same approach as in Test Example 1 except that the test samples were changed to compounds 48 and 49. The test results are shown in Figure 19.

### Test Example 14: mRNA knock-down in mouse primary hepatocyte by compound 50 targeting beta 2-macroglobulin (B2M)

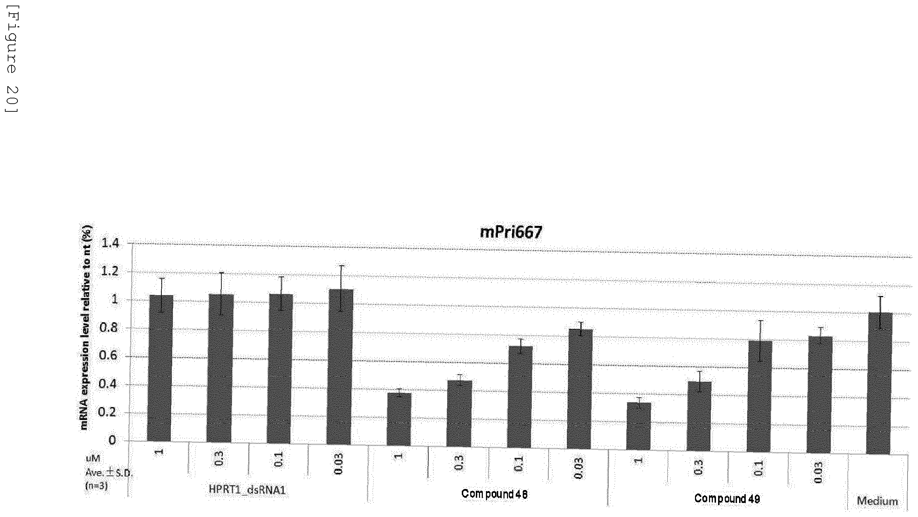

The test was carried out by the same approach as in Test Example 2 except that the test sample was changed to compound 50. The test results are shown in Figure 20.

### Test Example 15: mRNA knock-down in HeLa cell by compound 51 targeting hypoxanthine-guanine phosphoribosyltransferase 1 (HPRT1)

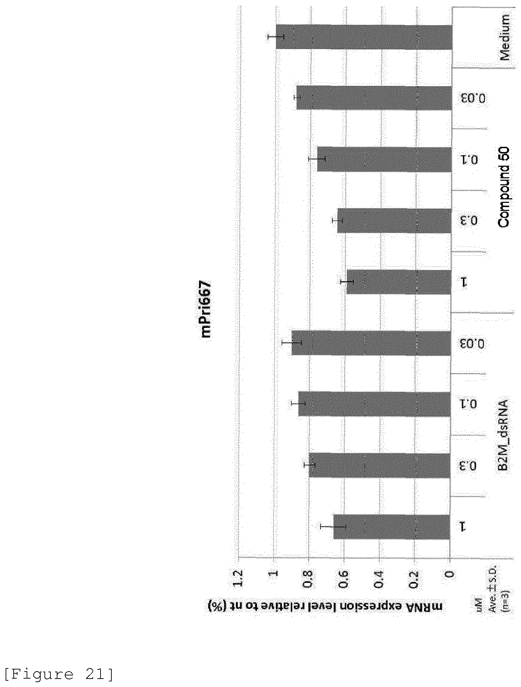

The test was carried out by the same approach as in Test Example 5 except that the test sample was changed to compound 51. The test results are shown in Figure 21.

### Test Example 16: mRNA knock-down in mouse primary hepatocyte by compound 52 targeting phosphatase and tensin homolog deleted from chromosome 10 (PTEN) and compound 53 targeting Factor 9

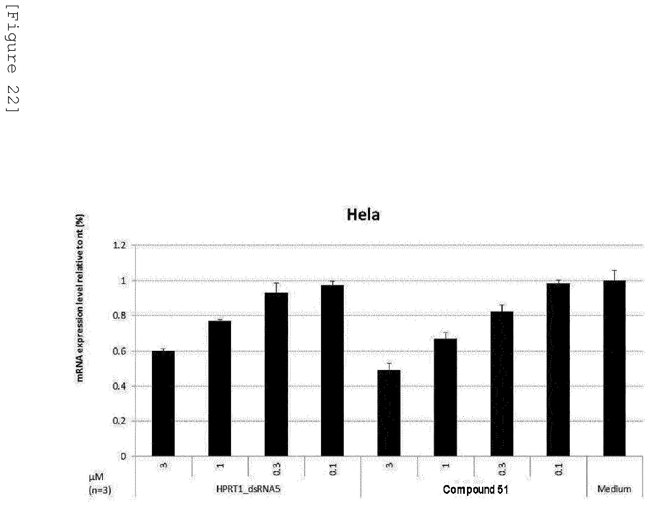

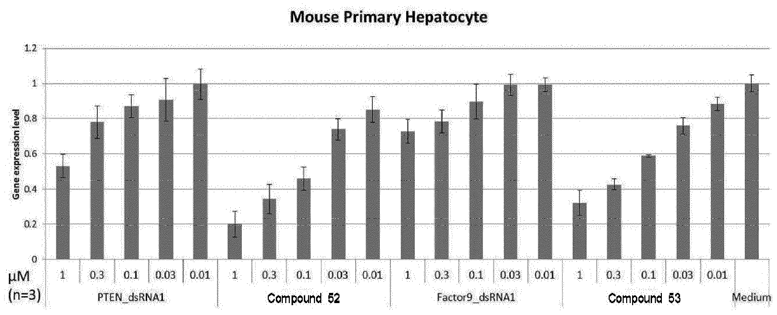

The test was carried out by the same approach as in Test Example 3 except that the test samples were changed to compound 52 and compound 53. The test results are shown in Figure 22.

### Test Example 17: In vivo mouse mRNA knock-down test of circular siRNA targeting HPRT1

Compound 6 synthesized in Example 6 and its negative control HPRT1_dsRNA5 were subjected to an *in vivo* evaluation test by a method given below. Each synthetic nucleic acid was used after being diluted with Dulbecco's Phosphate-Buffered Saline (DPBS) (manufactured by Nacalai Tesque, Inc.) according to the test. After acclimation of mice (BALB/cA, obtained from CLEA Japan, Inc.), each nucleic acid was intravenously administered at 20 mg/kg or 5 mg/kg to the mice. Only DPBS was intravenously administered to mice as a control group. Three days after the administration, each animal was euthanized, and the quadriceps femoris muscle and the liver were collected therefrom and freeze-dried in liquid nitrogen. From each frozen sample, total RNA was recovered using TRIzol (Registered Trademark) RNA isolation reagents (manufactured by Life Technologies Corp., catalog No. 15596026) and MagNA Pure 96 (manufactured by Roche Life Science) according to the methods described in the instructions attached to the products. cDNA was further prepared through reverse transcription reaction with the obtained total RNA as a template using Transcriptor First Strand cDNA synthesis kit (manufactured by F. Hoffmann-La Roche, Ltd., catalog No. 04897030001) according to the method described in the instruction attached to the product.

The obtained cDNA was used as a template in the PCR reaction of HPRT1 gene and GAPDH gene using TaqMan (Registered Trademark) Gene Expression Assay's probe (manufactured by Applied Biosystems, Inc.) as a probe and QuantStudio 12k flex real-time PCR system (manufactured by Applied Biosystems, Inc.) according to the methods described in the attached instruction manuals. The amplified mRNA level of each gene was measured, and the semiquantitative value of HPRT1 mRNA was calculated with the amplified GAPDH mRNA level as an internal control. When the similarly measured semiquantitative value of HPRT1 mRNA in the control group was defined as 1, the expression rate of HPRT mRNA in each synthetic nucleic acid administration group was determined from the semiquantitative value of HPRT1 mRNA of each synthetic nucleic acid administration group. The obtained rate of HPRT1 mRNA silencing is shown in Table 24.

**[Table 24]**

| Test material | Control | Compound 6 | | HPRTl_dsRNA5 | |
|---|---|---|---|---|---|
| Dose [mg/mL] | - | 20 | 5 | 20 | 5 |
| HPRT mRNA level in quadriceps femoris muscle [% silencing] | - | 44.11 | 16.71 | -1.16 | 4.34 |
| HPRT mRNA level in liver [% silencing] | - | 66.21 | 34.82 | 24.00 | 18.80 |

As is evident from Table 24, the compound of the present invention strongly silenced the HPRT1 gene in the quadriceps femoris muscle and the liver, as compared with HPRT1 dsRNA5.

### Free Text of Sequence Listing

SEQ ID NO: 1: HPRT1_ssRNA1 for negative control groups 1 and 2 shown in Tables 7 and 20.
SEQ ID NO: 2: HPRT1_ssRNA2 for negative control group 4 shown in Table 8.
SEQ ID NO: 3: HPRT1_ssRNA3 for negative control group 6 shown in Tables 8, 18 and 22.
SEQ ID NO: 4: HPRT1_ssRNA4 for negative control group 9 shown in Table 12.
SEQ ID NO: 5: HPRT1_ssRNA5 for Example 48 shown in Table 20.
SEQ ID NO: 6: HPRT1_ssRNA6 for Example 49 shown in Table 20.
SEQ ID NO: 7: HPRT1_ssRNA7 for Example 51 shown in Table 22.
SEQ ID NO: 8: HPRT1_asRNA1 for negative control group 1 and Examples 1, 2, 45, 48 and 49 shown in Tables 7, 17 and 20.
SEQ ID NO: 9: HPRT1_asRNA2 for negative control group 2 shown in Table 7.
SEQ ID NO: 10: HPRT1_asRNA3 for negative control group 4 and Examples 4 and 5 shown in Table 8.
SEQ ID NO: 11: HPRT1_asRNA4 for negative control group 5 shown in Table 8.
SEQ ID NO: 12: HPRT1_asRNA5 for negative control groups 6 and 9 and Examples 6, 9 to 44, 46, 47 and 51 shown in Tables 8, 11 to 19 and 22.
SEQ ID NO: 13: HPRT1_csRNA1 for Examples 1, 10, 11 and 18 to 24 shown in Tables 7 and 11 to 13.
SEQ ID NO: 14: HPRT1_csRNA2 for Examples 2, 12, 13, 25, 26, 32, 35, 38, 39, 41 and 44 shown in Tables 7 and 13 to 15.
SEQ ID NO: 15: HPRT1_csRNA3 for Examples 4, 27 to 34, 36, 42 and 43 shown in Tables 8 and 15 to 17.
SEQ ID NO: 16: HPRT1_csRNA4 for Examples 5, 37, 40 and 45 to 47 shown in Tables 8 and 17 to 19.
SEQ ID NO: 17: HPRT1_csRNA5 for Example 6 shown in Table 8.
SEQ ID NO: 18: HPRT1_csRNA6 for Example 9 shown in Table 11.
SEQ ID NO: 19: HPRT1_csRNA7 for Example 14 shown in Table 11.
SEQ ID NO: 20: HPRT1_csRNA8 for Example 15 shown in Table 11.
SEQ ID NO: 21: HPRT1_csRNA9 for Example 16 shown in Table 11.
SEQ ID NO: 22: HPRT1_csRNA10 for Example 17 shown in Table 11.
SEQ ID NO: 23: HPRT1_csRNA11 for Example 19 shown in Table 11.
SEQ ID NO: 24: HPRT1_csRNA12 for Example 20 shown in Table 11.
SEQ ID NO: 25: HPRT1_csRNA13 for Example 21 shown in Table 11.
SEQ ID NO: 26: HPRT1_csRNA14 for Example 22 shown in Table 11.
SEQ ID NO: 27: HPRT1_csRNA15 for Example 10 shown in Table 12.
SEQ ID NO: 28: HPRT1_csRNA16 for Example 11 shown in Table 12.
SEQ ID NO: 29: HPRT1_csRNA17 for Example 18 shown in Table 12.
SEQ ID NO: 30: HPRT1_csRNA18 for Example 23 shown in Table 12.
SEQ ID NO: 31: HPRT1_csRNA19 for Example 24 shown in Table 13.
SEQ ID NO: 32: HPRT1_csRNA20 for Example 25 shown in Table 13.
SEQ ID NO: 33: HPRT1_csRNA21 for Example 26 shown in Table 13.
SEQ ID NO: 34: HPRT1_csRNA22 for Example 35 shown in Table 13.
SEQ ID NO: 35: HPRT1_csRNA23 for Example 12 shown in Table 14.
SEQ ID NO: 36: HPRT1_csRNA24 for Example 13 shown in Table 14.
SEQ ID NO: 37: HPRT1_csRNA25 for Example 32 shown in Table 14.
SEQ ID NO: 38: HPRT1_csRNA26 for Example 41 shown in Table 14.
SEQ ID NO: 39: HPRT1_csRNA27 for Example 44 shown in Table 14.
SEQ ID NO: 40: HPRT1_csRNA28 for Example 38 shown in Table 15.
SEQ ID NO: 41: HPRT1_csRNA29 for Example 39 shown in Table 15.
SEQ ID NO: 42: HPRT1_csRNA30 for Example 42 shown in Table 15.
SEQ ID NO: 43: HPRT1_csRNA31 for Example 43 shown in Table 15.
SEQ ID NO: 44: HPRT1_csRNA32 for Example 28 shown in Table 16.
SEQ ID NO: 45: HPRT1_csRNA33 for Example 29 shown in Table 16.
SEQ ID NO: 46: HPRT1_csRNA34 for Example 30 shown in Table 16.
SEQ ID NO: 47: HPRT1_csRNA35 for Example 31 shown in Table 16.
SEQ ID NO: 48: HPRT1_csRNA36 for Example 33 shown in Table 16.
SEQ ID NO: 49: HPRT1_csRNA37 for Example 34 shown in Table 16.
SEQ ID NO: 50: HPRT1_csRNA38 for Example 27 shown in Table 17.
SEQ ID NO: 51: HPRT1_csRNA39 for Example 36 shown in Table 17.
SEQ ID NO: 52: HPRT1_csRNA40 for Example 37 shown in Table 17.
SEQ ID NO: 53: HPRT1_csRNA41 for Example 45 shown in Table 17.
SEQ ID NO: 54: HPRT1_csRNA42 for Example 47 shown in Table 18.
SEQ ID NO: 55: HPRT1_csRNA43 for Example 40 shown in Table 19.
SEQ ID NO: 56: HPRT1_csRNA44 for Example 46 shown in Table 19.
SEQ ID NO: 57: PTEN_ssRNA1 for negative control group 7 shown in Tables 10 and 23.
SEQ ID NO: 58: PTEN_ssRNA2 for Example 52 shown in Table 23.
SEQ ID NO: 59: PTEN_asRNA1 for negative control group 7 shown in Tables 10 and 23.
SEQ ID NO: 60: PTEN_asRNA2 for Examples 7 and 52 shown in Tables 10 and 23.
SEQ ID NO: 61: PTEN_csRNA1 for Example 7 shown in Table 10.
SEQ ID NO: 62: Factor9_ssRNA1 for negative control group 8 shown in Tables 10 and 23.
SEQ ID NO: 63: Factor9_ssRNA2 for Example 53 shown in Table 23.
SEQ ID NO: 64: Factor9_asRNA1 for negative control group 8 shown in Tables 10 and 23.
SEQ ID NO: 65: Factor9_asRNA2 for Examples 8 and 53 shown in Tables 10 and 23.
SEQ ID NO: 66: Factor9_csRNA1 for Example 8 shown in Table 10.
SEQ ID NO: 67: B2M_ssRNA1 for negative control group 3 shown in Tables 7 and 21.
SEQ ID NO: 68: B2M_ssRNA2 for Example 50 shown in Table 21.
SEQ ID NO: 69: E2M_asRNA1 for negative control group 3 and Examples 3 and 50 shown in Tables 7 and 21.
SEQ ID NO: 70: E2M_csRNA1 for Example 3 shown in Table 7.
SEQ ID NO: 71: RT primer in Test Example 9.
SEQ ID NO: 72: Forward primer in Test Example 9.
SEQ ID NO: 73: Reverse primer in Test Example 9.
SEQ ID NO: 74: TaqMan probe in Test Example 9.

## Claims

1. An oligonucleotide derivative or a salt thereof comprising a circular oligonucleotide and a linear oligonucleotide, wherein
the circular oligonucleotide and the linear oligonucleotide have base sequences complementary to each other, and form a complex via a hydrogen bond between the complementary base sequences, and
wherein the circular oligonucleotide has a base length of 10 to 40, and
wherein the circular oligonucleotide is represented by formula 1: 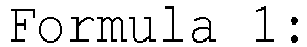 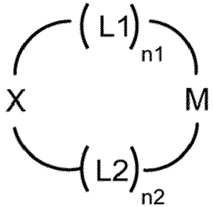 wherein
L1 and L2 each represents a linker;
n1 and n2 each independently represents an integer of 0 to 10;
M represents a moiety comprising a chemical structure that is cleaved by an intracellular environment; and
X represents an oligonucleotide.

2. The oligonucleotide derivative or a salt thereof according to claim 1, wherein the circular oligonucleotide comprises at least one phosphorothioate bond.

3. The oligonucleotide derivative or a salt thereof according to claim 1 or 2, wherein the circular oligonucleotide comprises at least one 2'-modified nucleotide.

4. The oligonucleotide derivative or a salt thereof according to any one of claims 1 to 3, wherein the base length of the circular oligonucleotide is the same as that of the linear oligonucleotide, or is longer than that of the linear oligonucleotide.

5. The oligonucleotide derivative or a salt thereof according to any one of claims 1 to 4, wherein the chemical structure that is cleaved by an intracellular environment is -S-S-, -S-C(O)- or -C(O)-S-.

6. The oligonucleotide derivative or a salt thereof according to any one of claims 1 to 5, wherein M is selected from the group consisting of formula 3-1 to formula 3-6: 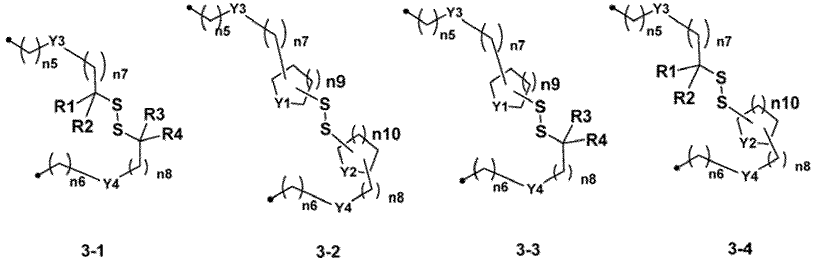 wherein
R1 and R2 each independently represents a hydrogen atom or C1-C3 alkyl, or R1 and R2, together with the carbon atom to which they are bonded, form a ring having 3 to 6 carbon atoms;
R3 and R4 each independently represents a hydrogen atom or C1-C3 alkyl, or R3 and R4, together with the carbon atom to which they are bonded, form a ring having 3 to 6 carbon atoms;
n5 to n8 each independently represents an integer of 0 to 10;
n9 and n10 each independently represents an integer of 1 to 4;
Y1 to Y4 each independently represents a bond, -NR5-, -O-or -S-; and
R5 represents a hydrogen atom, C1-C3 alkyl or C2-C4 alkanoyl, and 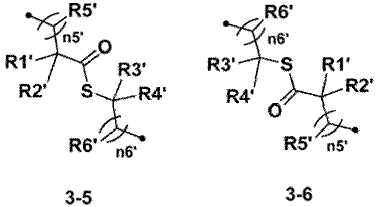 wherein
R1' and R2' each independently represents a hydrogen atom or C1-C3 alkyl, or R1' and R2', together with the carbon atom to which they are bonded, form a ring having 3 to 6 carbon atoms;
R3' and R4' each independently represents a hydrogen atom or C1-C3 alkyl, or R3' and R4', together with the carbon atom to which they are bonded, form a ring having 3 to 6 carbon atoms;
R5' and R6' represent a hydrogen atom or C1-C3 alkyl independently on the basis of each carbon atom to which they are bonded; and
n5' and n6' each independently represents an integer of 1 to 10.

7. The oligonucleotide derivative or a salt thereof according to any one of claims 1 to 6, wherein the oligonucleotide derivative or a salt thereof has at least one targeting compound.

8. A pharmaceutical composition comprising an oligonucleotide derivative or a salt thereof according to any one of claims 1 to 7.

9. An agent for suppressing an expression of a target gene utilizing RNA interference (RNAi), comprising an oligonucleotide derivative or a salt thereof according to any one of claims 1 to 7.

10. A circular oligonucleotide comprising at least one phosphorothioate bond, the circular oligonucleotide being represented by formula 4: 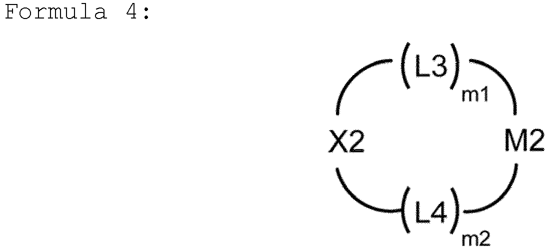 wherein
L3 and L4 each represents a linker;
m1 and m2 each independently represents an integer of 0 to 10;
M2 represents a moiety comprising a chemical structure that is cleaved by an intracellular environment,
wherein the chemical structure that is cleaved by an intracellular environment is -S-S-, -S-C(O)- or -C(O)-S-,
wherein M2 is selected from the group consisting of formula 6-1 to formula 6-6: 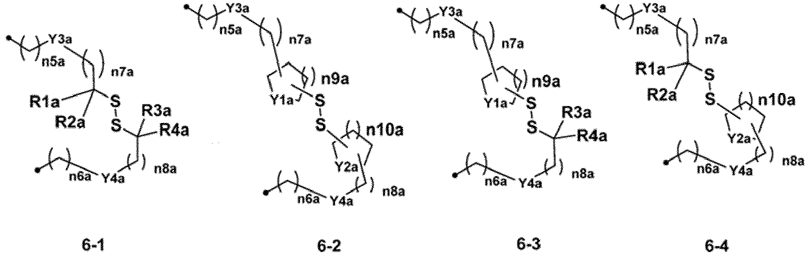 wherein
R1a and R2a each independently represents a hydrogen atom or C1-C3 alkyl, or R1a and R2a, together with the carbon atom to which they are bonded, form a ring having 3 to 6 carbon atoms;
R3a and R4a each independently represents a hydrogen atom or C1-C3 alkyl, or R3a and R4a, together with the carbon atom to which they are bonded, form a ring having 3 to 6 carbon atoms;
n5a to n8a each independently represents an integer of 0 to 10;
n9a and n10a each independently represents an integer of 1 to 4;
Y1a to Y4a each independently represents a bond, - NR5a-, -O- or -S-; and
R5a represents a hydrogen atom, C1-C3 alkyl or C2-C4 alkanoyl, and 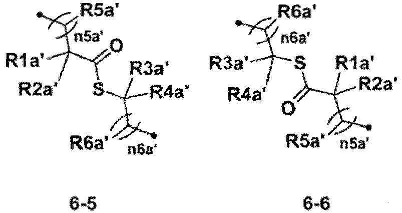 wherein
R1a' and R2a' each independently represents a hydrogen atom or C1-C3 alkyl, or R1a' and R2a', together with the carbon atom to which they are bonded, form a ring having 3 to 6 carbon atoms;
R3a' and R4a' each independently represents a hydrogen atom or C1-C3 alkyl, or R3a' and R4a', together with the carbon atom to which they are bonded, form a ring having 3 to 6 carbon atoms;
R5a' and R6a' represent a hydrogen atom or C1-C3 alkyl independently on the basis of each carbon atom to which they are bonded; and
n5a' and n6a' each independently represents an integer of 1 to 10; and
X2 represents an oligonucleotide.

11. A linear oligonucleotide comprising at least one phosphorothioate bond, the linear oligonucleotide being represented by formula 7: 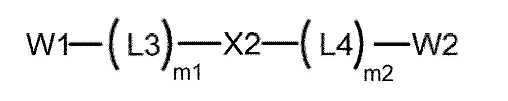 wherein
X2, L3, L4, m1 and m2 are as defined in claim 11; and
W1 and W2 are moieties comprising or forming functional groups reacting with each other to form a chemical structure that is cleaved by an intracellular environment,
wherein the chemical structure that is cleaved by an intracellular environment is -S-S-, -S-C(O)- or -C(O)-S-, and
wherein W1 and W2 are each independently -A1-S-S-A2 or -B1-COO-B2 (wherein the case is excluded where W1 and W2 are -B1-COO-B2 at the same time);
A1 and B1 are each independently C2-C10 alkylene optionally having a substituent;
A2 is C1-C10 alkyl optionally having a substituent; and
B2 is a hydrogen atom or C1-C6 alkyl optionally having a substituent.

12. A method for producing a circular oligonucleotide according to claim 10, comprising circularizing a linear oligonucleotide according to claim 11.

13. A method for producing an oligonucleotide derivative or a salt thereof according to any one of claims 1 to 7, comprising complexing a circular oligonucleotide according to claim 10 with a linear oligonucleotide having a base sequence complementary to the circular oligonucleotide via a hydrogen bond.

## Patentansprüche

1. Oligonukleotidderivat oder ein Salz davon, umfassend ein zirkuläres Oligonukleotid und ein lineares Oligonukleotid,
wobei das zirkuläre Oligonukleotid und das lineare Oligonukleotid Basensequenzen aufweisen, die komplementär zueinander sind, und über eine Wasserstoffbrückenbindung zwischen den komplementären Basensequenzen einen Komplex bilden, und
wobei das zirkuläre Oligonukleotid eine Basenlänge von 10 bis 40 aufweist, und
wobei das zirkuläre Oligonukleotid durch die Formel 1 dargestellt wird: 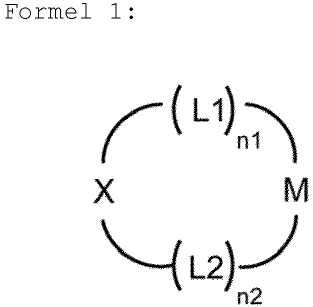 wobei
L1 und L2 jeweils einen Linker darstellen;
n1 und n2 jeweils unabhängig voneinander eine ganze Zahl von 0 bis 10 darstellen;
M eine Einheit darstellt, umfassend eine chemische Struktur, die durch eine intrazelluläre Umgebung gespalten wird; und
X ein Oligonukleotid darstellt.

2. Oligonukleotidderivat oder ein Salz davon nach Anspruch 1, wobei das zirkuläre Oligonukleotid zumindest eine Phosphorothioat-Bindung umfasst.

3. Oligonukleotidderivat oder ein Salz davon nach Anspruch 1 oder 2, wobei das zirkuläre Oligonukleotid zumindest ein 2'-modifiziertes Nukleotid umfasst.

4. Oligonukleotidderivat oder ein Salz davon nach einem der Ansprüche 1 bis 3, wobei die Basenlänge des zirkulären Oligonukleotids die gleiche ist wie die des linearen Oligonukleotids oder länger ist als die des linearen Oligonukleotids.

5. Oligonukleotidderivat oder ein Salz davon nach einem der Ansprüche 1 bis 4, wobei es sich bei der chemischen Struktur, die durch eine intrazelluläre Umgebung gespalten wird, um -S-S-, -S-C(O)- oder -C(O)-S- handelt.

6. Oligonukleotidderivat oder ein Salz davon nach einem der Ansprüche 1 bis 5, wobei M ausgewählt ist aus der Gruppe bestehend aus Formel 3-1 bis Formel 3-6: 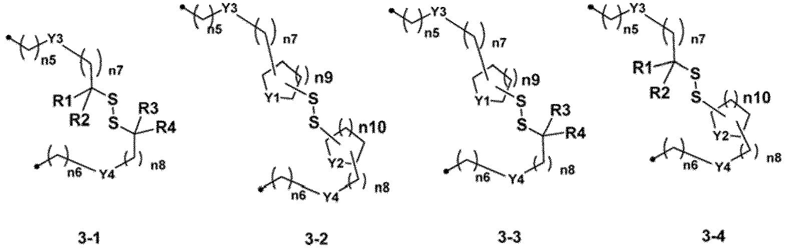 wobei
R1 und R2 jeweils unabhängig voneinander ein Wasserstoffatom oder C1-C3-Alkyl darstellen, oder R1 und R2, zusammen mit dem Kohlenstoffatom, an das sie gebunden sind, einen Ring bilden, der 3 bis 6 Kohlenstoffatome aufweist;
R3 und R4 jeweils unabhängig voneinander ein Wasserstoffatom oder C1-C3-Alkyl darstellen, oder R3 und R4, zusammen mit dem Kohlenstoffatom, an das sie gebunden sind, einen Ring bilden, der 3 bis 6 Kohlenstoffatome aufweist;
n5 bis n8 jeweils unabhängig voneinander eine ganze Zahl von 0 bis 10 darstellen;
n9 und n10 jeweils unabhängig voneinander eine ganze Zahl von 1 bis 4 darstellen;
Y1 bis Y4 jeweils unabhängig voneinander eine Bindung, -NR5-, -O- oder -S- darstellen; und R5 ein Wasserstoffatom, C1-C3-Alkyl oder C2-C4-Alkanoyl darstellt, und 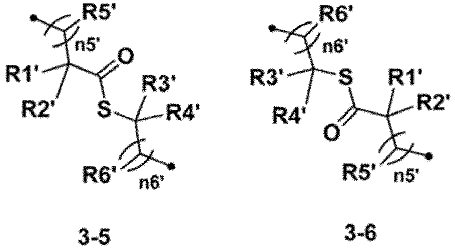 wobei
R1' und R2' jeweils unabhängig voneinander ein Wasserstoffatom oder C1-C3-Alkyl darstellen, oder R1' und R2', zusammen mit dem Kohlenstoffatom, an das sie gebunden sind, einen Ring bilden, der 3 bis 6 Kohlenstoffatome aufweist;
R3' und R4' jeweils unabhängig voneinander ein Wasserstoffatom oder C1-C3-Alkyl darstellen, oder R3' und R4', zusammen mit dem Kohlenstoffatom, an das sie gebunden sind, einen Ring bilden, der 3 bis 6 Kohlenstoffatome aufweist;
R5' und R6' ein Wasserstoffatom oder C1-C3-Alkyl darstellen, unabhängig basierend auf jedem Kohlenstoffatom, an das sie gebunden sind; und
n5' und n6' jeweils unabhängig voneinander eine ganze Zahl von 1 bis 10 darstellen.

7. Oligonukleotidderivat oder ein Salz davon nach einem der Ansprüche 1 bis 6, wobei das Oligonukleotidderivat oder ein Salz davon zumindest eine Targeting-Verbindung aufweist.

8. Pharmazeutische Zusammensetzung, umfassend ein Oligonukleotidderivat oder ein Salz davon nach einem der Ansprüche 1 bis 7.

9. Mittel zum Unterdrücken einer Expression eines Zielgens unter Verwendung von RNA-Interferenz (RNAi), umfassend ein Oligonukleotidderivat oder ein Salz davon nach einem der Ansprüche 1 bis 7.

10. Zirkuläres Oligonukleotid, umfassend zumindest eine Phosphorothioat-Bindung, wobei das zirkuläre Oligonukleotid durch Formel 4 dargestellt wird: 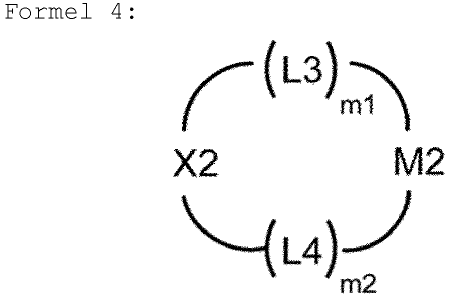 wobei
L3 und L4 jeweils einen Linker darstellen;
m1 und m2 jeweils unabhängig voneinander eine ganze Zahl von 0 bis 10 darstellen;
M2 eine Einheit darstellt, umfassend eine chemische Struktur, die durch eine intrazelluläre Umgebung gespalten wird,
wobei es sich bei der chemischen Struktur, die durch eine intrazelluläre Umgebung gespalten wird, um -S-S-, - S-C(O)- oder -C(O)-S- handelt,
wobei M2 ausgewählt ist aus der Gruppe bestehend aus Formel 6-1 bis Formel 6-6: 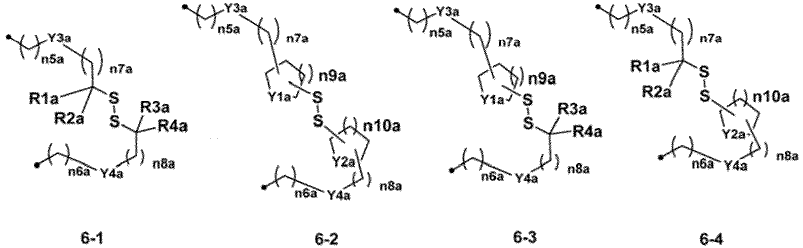 wobei
R1a und R2a jeweils unabhängig voneinander ein Wasserstoffatom oder C1-C3-Alkyl darstellen, oder R1a und R2a, zusammen mit dem Kohlenstoffatom, an das sie gebunden sind, einen Ring bilden, der 3 bis 6 Kohlenstoffatome aufweist;
R3a und R4a jeweils unabhängig voneinander ein Wasserstoffatom oder C1-C3-Alkyl darstellen, oder R3a und R4a, zusammen mit dem Kohlenstoffatom, an das sie gebunden sind, einen Ring bilden, der 3 bis 6 Kohlenstoffatome aufweist;
n5a bis n8a jeweils unabhängig voneinander eine ganze Zahl von 0 bis 10 darstellen,
n9a und n10a jeweils unabhängig voneinander eine ganze Zahl von 1 bis 4 darstellen;
Y1a bis Y4a jeweils unabhängig voneinander eine Bindung, -NR5a-, -O- oder -S- darstellen; und
R5a ein Wasserstoffatom, C1-C3-Alkyl oder C2-C4-Alkanoyl darstellt, und 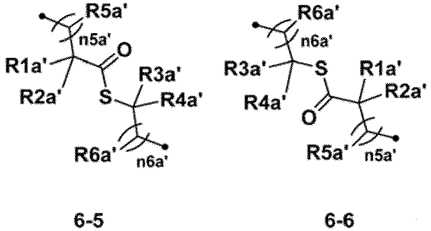 wobei
R1a' und R2a' jeweils unabhängig voneinander ein Wasserstoffatom oder C1-C3-Alkyl darstellen, oder R1a' und R2a', zusammen mit dem Kohlenstoffatom, an das sie gebunden sind, einen Ring bilden, der 3 bis 6 Kohlenstoffatome aufweist;
R3a' und R4a' jeweils unabhängig voneinander ein Wasserstoffatom oder C1-C3-Alkyl darstellen, oder R3a' und R4a', zusammen mit dem Kohlenstoffatom, an das sie gebunden sind, einen Ring bilden, der 3 bis 6 Kohlenstoffatome aufweist;
R5a' und R6a' ein Wasserstoffatom oder C1-C3-Alkyl darstellen, unabhängig basierend auf jedem Kohlenstoffatom, an das sie gebunden sind; und
n5a' und n6a' jeweils unabhängig voneinander eine ganze Zahl von 1 bis 10 darstellen; und
X2 ein Oligonukleotid darstellt.

11. Zirkuläres Oligonukleotid, umfassend zumindest eine Phosphorothioat-Bindung, wobei das zirkuläre Oligonukleotid durch Formel 7 dargestellt wird: 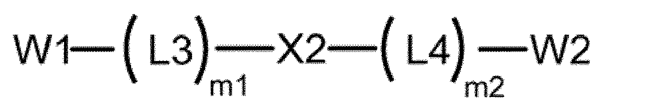
wobei X2, L3, L4, m1 und m2 wie in Anspruch 11 definiert sind; und
W1 und W2 Einheiten sind, die funktionelle Gruppen umfassen oder bilden, die miteinander reagieren, um eine chemische Struktur zu bilden, die durch eine intrazelluläre Umgebung gespalten wird,
wobei es sich bei der chemischen Struktur, die durch eine intrazelluläre Umgebung gespalten wird, um -S-S-, - S-C(O)- oder -C(O)-S- handelt, und
wobei W1 und W2 jeweils unabhängig voneinander -A1-S-S-A2 oder -B1-COO-B2 (wobei der Fall ausgeschlossen ist, dass W1 und W2 zur gleichen Zeit -B1-COO-B2 sind) sind;
A1 und B1 jeweils unabhängig voneinander C2-C10-Alkylen sind, gegebenenfalls aufweisend einen Substituenten;
A2 C1-C10-Alkyl ist, gegebenenfalls aufweisend einen Substituenten; und
B2 ein Wasserstoffatom oder C1-C6-Alkyl ist, gegebenenfalls aufweisend einen Substituenten.

12. Verfahren zum Herstellen eines zirkulären Oligonukleotids nach Anspruch 10, umfassend die Zirkularisierung eines linearen Oligonukleotids nach Anspruch 11.

13. Verfahren zum Herstellen eines Oligonukleotidderivats oder eines Salzes davon nach einem der Ansprüche 1 bis 7, umfassend die Komplexbildung eines zirkulären Oligonukleotids nach Anspruch 10 mit einem linearen Oligonukleotid, das eine Basensequenz aufweist, die komplementär zum zirkulären Oligonukleotid ist über eine Wasserstoffbrückenbindung.

## Revendications

1. Dérivé d'oligonucléotide ou sel correspondant comprenant un oligonucléotide circulaire et un oligonucléotide linéaire,
l'oligonucléotide circulaire et l'oligonucléotide linéaire possédant des séquences de bases complémentaires les unes aux autres, et formant un complexe via une liaison hydrogène entre les séquences de bases complémentaires, et
l'oligonucléotide circulaire possédant une longueur de bases de 10 à 40, et
l'oligonucléotide circulaire étant représenté par la formule 1 : 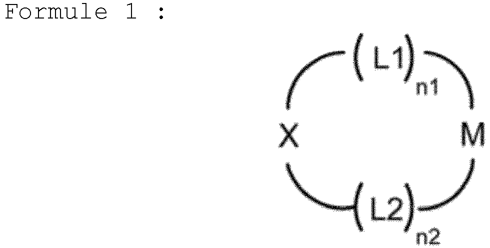
L1 et L2 représentant chacun un lieur ;
n1 et n2 représentant chacun indépendamment un entier de 0 à 10 ;
M représentant un groupement comprenant une structure chimique qui est clivée par un environnement intracellulaire ; et
X représentant un oligonucléotide.

2. Dérivé d'oligonucléotide ou sel correspondant selon la revendication 1, l'oligonucléotide circulaire comprenant au moins une liaison phosphorothioate.

3. Dérivé d'oligonucléotide ou sel correspondant selon la revendication 1 ou 2, l'oligonucléotide circulaire comprenant au moins un nucléotide modifié en 2' .

4. Dérivé d'oligonucléotide ou sel correspondant selon l'une quelconque des revendications 1 à 3, la longueur de bases de l'oligonucléotide circulaire étant la même que celle de l'oligonucléotide linéaire, ou étant plus longue que celle de l'oligonucléotide linéaire.

5. Dérivé d'oligonucléotide ou sel correspondant selon l'une quelconque des revendications 1 à 4, la structure chimique qui est clivée par un environnement intracellulaire étant -S-S-, -S-C(O)-ou -C(O)-S-.

6. Dérivé d'oligonucléotide ou sel correspondant selon l'une quelconque des revendications 1 à 5, M étant choisi dans le groupe constitué par la formule 3-1 à la formule 3-6 : 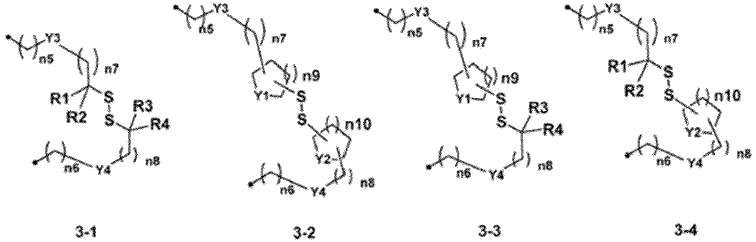
R1 et R2 représentant chacun indépendamment un atome d'hydrogène ou alkyle en C1-C3, ou R1 et R2, conjointement avec l'atome de carbone auquel ils sont liés, formant un cycle possédant 3 à 6 atomes de carbone ;
R3 et R4 représentant chacun indépendamment un atome d'hydrogène ou alkyle en C1-C3, ou R3 et R4, conjointement avec l'atome de carbone auquel ils sont liés, formant un cycle possédant 3 à 6 atomes de carbone ;
n5 à n8 représentant chacun indépendamment un entier de 0 à 10 ;
n9 et n10 représentant chacun indépendamment un entier de 1 à 4 ;
Y1 à Y4 représentant chacun indépendamment une liaison, -NR5-, -O- ou -S- ; et
R5 représentant un atome d'hydrogène, alkyle en C1-C3, ou alcanoyle en C2-C4, et 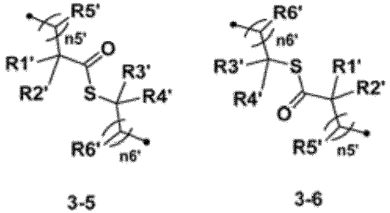
R1' et R2' représentant chacun indépendamment un atome d'hydrogène ou alkyle en C1-C3, ou R1' et R2', conjointement avec l'atome de carbone auquel ils sont liés, formant un cycle possédant 3 à 6 atomes de carbone ;
R3' et R4' représentant chacun indépendamment un atome d'hydrogène ou alkyle en C1-C3, ou R3' et R4', conjointement avec l'atome de carbone auquel ils sont liés, formant un cycle possédant 3 à 6 atomes de carbone ;
R5' et R6' représentant un atome d'hydrogène ou alkyle en C1-C3 indépendamment sur la base de chaque atome de carbone auxquels ils sont liés ; et
n5' et n6' représentant chacun indépendamment un entier de 1 à 10.

7. Dérivé d'oligonucléotide ou sel correspondant selon l'une quelconque des revendications 1 à 6, le dérivé d'oligonucléotide ou un sel correspondant possédant au moins un composé de ciblage.

8. Composition pharmaceutique comprenant un dérivé d'oligonucléotide ou un sel correspondant selon l'une quelconque des revendications 1 à 7.

9. Agent pour la suppression d'une expression d'un gène cible utilisant l'interférence par ARN (ARNi), comprenant un dérivé d'oligonucléotide ou un sel correspondant selon l'une quelconque des revendications 1 à 7.

10. Oligonucléotide circulaire comprenant au moins une liaison phosphorothioate, l'oligonucléotide circulaire étant représenté par la formule 4 : 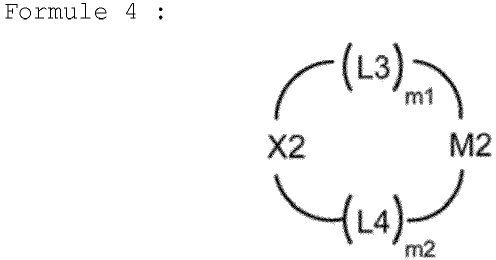
L3 et L4 représentant chacun un lieur ;
m1 et m2 représentant chacun indépendamment un entier de 0 à 10 ;
M2 représentant un groupement comprenant une structure chimique qui est clivée par un environnement intracellulaire,
la structure chimique qui est clivée par un environnement intracellulaire étant -S-S-, -S-C(O)-ou -C(O)-S-,
M2 étant choisi dans le groupe constitué par la formule 6-1 à la formule 6-6 : 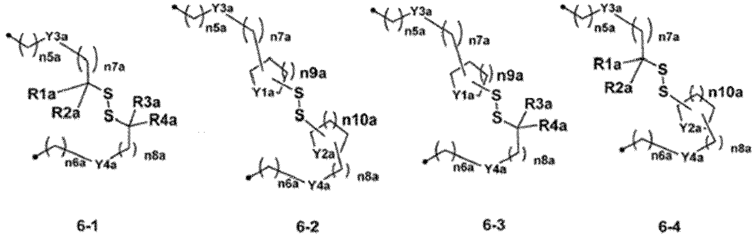
R1a et R2a représentant chacun indépendamment un atome d'hydrogène ou alkyle en C1-C3, ou R1a et R2a, conjointement avec l'atome de carbone auquel ils sont liés, formant un cycle possédant 3 à 6 atomes de carbone ;
R3a et R4a représentant chacun indépendamment un atome d'hydrogène ou alkyle en C1-C3, ou R3a et R4a, conjointement avec l'atome de carbone auquel ils sont liés, formant un cycle possédant 3 à 6 atomes de carbone ;
n5a à n8a représentant chacun indépendamment un entier de 0 à 10 ;
n9a et n10a représentant chacun indépendamment un entier de 1 à 4 ;
Y1a à Y4a représentant chacun indépendamment une liaison, -NR5a-, -O- ou -S- ; et
R5a représentant un atome d'hydrogène, alkyle en C1-C3, ou alcanoyle en C2-C4, et 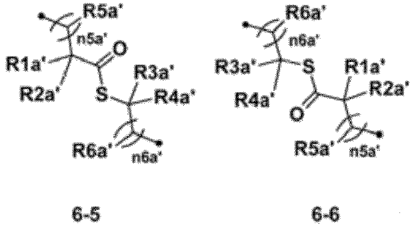
R1a' et R2a' représentant chacun indépendamment un atome d'hydrogène ou alkyle en C1-C3, ou R1a' et R2a', conjointement avec l'atome de carbone auquel ils sont liés, formant un cycle possédant 3 à 6 atomes de carbone ;
R3a' et R4a' représentant chacun indépendamment un atome d'hydrogène ou alkyle en C1-C3, ou R3a' et R4a', conjointement avec l'atome de carbone auquel ils sont liés, formant un cycle possédant 3 à 6 atomes de carbone ;
R5a' et R6a' représentant un atome d'hydrogène ou alkyle en C1-C3 indépendamment sur la base de chaque atome de carbone auxquels ils sont liés ; et
n5a' et n6a' représentant chacun indépendamment un entier de 1 à 10 ; et
X2 représentant un oligonucléotide.

11. Oligonucléotide linéaire comprenant au moins une liaison phosphorothioate, l'oligonucléotide linéaire étant représenté par la formule 7 : 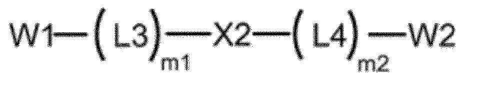
X2, L3, L4, m1 et m2 étant tels que définis dans la revendication 11 ; et
W1 et W2 étant des groupements comprenant ou formant des groupes fonctionnels réagissant les uns avec les autres pour former une structure chimique qui est clivée par un environnement intracellulaire,
la structure chimique qui est clivée par un environnement intracellulaire étant -S-S-, -S-C(O)-ou -C(O)-S-, et
W1 et W2 étant chacun indépendamment -A1-S-S-A2 ou -B1-COO-B2 (le cas où W1 et W2 sont -B1-COO-B2 en même temps étant exclu) ;
A1 et B1 étant chacun indépendamment alkylène en C2-C10 possédant éventuellement un substituant ;
A2 étant alkyle en C1-C10 possédant éventuellement un substituant ; et
B2 étant un atome d'hydrogène ou alkyle en C1-C6 possédant éventuellement un substituant.

12. Procédé pour la production d'un oligonucléotide circulaire selon la revendication 10, comprenant la circularisation d'un oligonucléotide linéaire selon la revendication 11.

13. Procédé pour la production d'un dérivé d'oligonucléotide ou d'un sel correspondant selon l'une quelconque des revendications 1 à 7, comprenant la complexation d'un oligonucléotide circulaire selon la revendication 10 avec un oligonucléotide linéaire possédant une séquence de bases complémentaire à l'oligonucléotide circulaire via une liaison hydrogène.
